(19) Europäisches Patentamt
European Patent Office
Office européen des brevets

(11) EP 1 399 441 B1

(12) EUROPEAN PATENT SPECIFICATION

(45) Date of publication and mention of the grant of the patent:
**05.07.2006 Bulletin 2006/27**

(21) Application number: **02754750.4**

(22) Date of filing: **25.06.2002**

(51) Int Cl.:
*C07D 417/14* (2006.01) *C07D 411/14* (2006.01)
*C07D 407/14* (2006.01) *C07D 413/14* (2006.01)
*A61K 31/445* (2006.01) *A61P 25/00* (2006.01)
*A61P 3/10* (2006.01) *A61P 15/00* (2006.01)
*C07D 407/06* (2006.01) *C07D 491/04* (2006.01)
*C07D 401/06* (2006.01) *C07D 409/14* (2006.01)
*C07D 471/04* (2006.01)

(86) International application number:
**PCT/EP2002/007009**

(87) International publication number:
**WO 2003/002561 (09.01.2003 Gazette 2003/02)**

# (54) N-AROYL CYCLIC AMINE DERIVATIVES AS OREXIN RECEPTOR ANTAGONISTS

N-AROYL-(CYCLISCHES AMIN)-DERIVATE ALS OREXINREZEPTOR ANTAGONISTEN

DERIVES D'AMINE CYCLIQUES N-AROYLE UTILISES COMME ANTAGONISTES DU RECEPTEUR DE L'OREXINE

(84) Designated Contracting States:
**AT BE CH CY DE DK ES FI FR GB GR IE IT LI LU MC NL PT SE TR**
Designated Extension States:
**SI**

(30) Priority: **28.06.2001 GB 0115863**
**19.12.2001 GB 0130342**

(43) Date of publication of application:
**24.03.2004 Bulletin 2004/13**

(73) Proprietor: **SMITHKLINE BEECHAM PLC**
**Brentford,**
**Middlesex TW8 9GS (GB)**

(72) Inventors:
- **BRANCH, Clive, Leslie**
  **Third Avenue,**
  **Harlow,**
  **Essex CM19 5AW (GB)**
- **CHAN, Wai, Ngor**
  **Third Avenue,**
  **Harlow,**
  **Essex CM19 5AW (GB)**
- **JOHNS, Amanda**
  **Third Avenue,**
  **Harlow,**
  **Essex CM19 5AW (GB)**
- **JOHNSON, Christopher, Norbert**
  **Third Avenue,**
  **Harlow,**
  **Essex CM19 5AW (GB)**
- **NASH, David, John**
  **Third Avenue,**
  **Harlow,**
  **Essex CM19 5AW (GB)**
- **NOVELLI, Riccardo**
  **Third Avenue,**
  **Harlow,**
  **Essex CM19 5AW (GB)**
- **PILLEUX, Jean-Pierre**
  **Third Avenue,**
  **Harlow,**
  **Essex CM19 5AW (GB)**
- **PORTER, Roderick, Alan**
  **Third Avenue,**
  **Harlow,**
  **Essex CM19 5AW (GB)**
- **STEAD, Rachel, Elizabeth, Anne**
  **Third Avenue,**
  **Harlow,**
  **Essex CM19 5AW (GB)**
- **STEMP, Geoffrey**
  **Third Avenue,**
  **Harlow,**
  **Essex CM19 5AW (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

(74) Representative: **Lawrence, Geoffrey Mark Prouse**
**GlaxoSmithKline,**
**Corporate Intellectual Property,**
**980 Great West Road**
**Brentford,**
**Middlesex TW8 9GS (GB)**

(56) References cited:
**WO-A-00/47576**
**WO-A-00/47580**
**WO-A-01/96302**
**WO-A-99/09024**

## Description

This invention relates to *N*-aroyl cyclic amine derivatives and their use as pharmaceuticals.

Many medically significant biological processes are mediated by proteins participating in signal transduction pathways that involve G-proteins and/or second messengers.

Polypeptides and polynucleotides encoding the human 7-transmembrane G-protein coupled neuropeptide receptor, orexin-1 (HFGAN72), have been identified and are disclosed in EP-A-875565, EP-A-875566 and WO 96/34877. Polypeptides and polynucleotides encoding a second human orexin receptor, orexin-2 (HFGANP), have been identified and are disclosed in EP-A-893498.

Polypeptides and polynucleotides encoding polypeptides which are ligands for the orexin-1 receptor, e.g. orexin-A (Lig72A) are disclosed in EP-A-849361.

Orexin receptors are found in the mammalian host and may be responsible for many biological functions, including pathologies including, but not limited to, depression; anxiety; addictions; obsessive compulsive disorder, affective neurosis/disorder; depressive neurosis/disorder; anxiety neurosis; dysthymic disorder, behaviour disorder; mood disorder, sexual dysfunction; psychosexual dysfunction; sex disorder; sexual disorder, schizophrenia; manic depression; delerium; dementia; severe mental retardation and dyskinesias such as Huntington's disease and Gilles de la Tourett's syndrome; disturbed biological and circadian rhythms; feeding disorders, such as anorexia, bulimia, cachexia, and obesity; diabetes; appetite/taste disorders; vomiting/nausea; asthma; cancer, Parkinson's disease; Cushing's syndrome / disease; basophil adenoma; prolactinoma; hyperprolactinemia; hypopituitarism; hypophysis tumor / adenoma; hypothalamic diseases; Froehlich's syndrome; adrenohypophysis disease; hypophysis disease; hypophysis tumor / adenoma; pituitary growth hormone; adrenohypophysis hypofunction; adrenohypophysis hyperfunction; hypothalamic hypogonadism; Kallman's syndrome (anosmia, hyposmia); functional or psychogenic amenorrhea; hypopituitarism; hypothalamic hypothyroidism; hypothalamic-adrenal dysfunction; idiopathic hyperprolactinemia; hypothalamic disorders of growth hormone deficiency; idiopathic growth hormone deficiency; dwarfism; gigantism; acromegaly; and sleep disturbances associated with such diseases as neurological disorders, neuropathic pain and restless leg syndrome, heart and lung diseases; acute and congestive heart failure; hypotension; hypertension; urinary retention; osteoporosis; angina pectoris; myocardial infarction; ischaemic or haemorrhagic stroke; subarachnoid haemorrhage; head injury such as sub-arachnoid haemorrhage associated with traumatic head injury; ulcers; allergies; benign prostatic hypertrophy; chronic renal failure; renal disease; impaired glucose tolerance; migraine; hyperalgesia; pain; enhanced or exaggerated sensitivity to pain, such as hyperalgesia, causalgia and allodynia; acute pain; burn pain; atypical facial pain; neuropathic pain; back pain; complex regional pain syndromes I and II; arthritic pain; sports injury pain; pain related to infection, e.g. HIV, post-polio syndrome, and post-herpetic neuralgia; phantom limb pain; labour pain; cancer pain; post-chemotherapy pain; post-stroke pain; post-operative pain; neuralgia; nausea, vomiting; conditions associated with visceral pain including irritable bowel syndrome, migraine and angina; urinary bladder incontinence e.g. urge incontinence; tolerance to narcotics or withdrawal from narcotics; sleep disorders; sleep apnea; narcolepsy; insomnia; parasomnia; jet-lag syndrome; and neurodegenerative disorders, which includes nosological entities such as disinhibition-dementia-parkinsonism-amyotrophy complex; pallido-ponto-nigral degeneration, epilepsy, and seizure disorders.

Experiments have shown that central administration of the ligand orexin-A (described in more detail below) stimulated food intake in freely-feeding rats during a 4 hour time period. This increase was approximately four-fold over control rats receiving vehicle. These data suggest that orexin-A may be an endogenous regulator of appetite. Therefore, antagonists of its receptor may be useful in the treatment of obesity and diabetes, see *Cell,* 1998, 92, 573-585.

There is a significant incidence of obesity in westemised societies. According to WHO definitions a mean of 35% of subjects in 39 studies were overweight and a further 22% clinically obese. It has been estimated that 5.7% of all healthcare costs in the USA are a consequence of obesity. About 85% of Type 2 diabetics are obese, and diet and exercise are of value in all diabetics. The incidence of diagnosed diabetes in westemised countries is typically 5% and there are estimated to be an equal number undiagnosed. The incidence of both diseases is rising, demonstrating the inadequacy of current treatments which may be either ineffective or have toxicity risks including cardiovascular effects. Treatment of diabetes with sulfonylureas or insulin can cause hypoglycaemia, whilst metformin causes GI side-effects. No drug treatment for Type 2 diabetes has been shown to reduce the long-term complications of the disease. Insulin sensitisers will be useful for many diabetics, however they do not have an anti-obesity effect.

Rat sleep/EEG studies have also shown that central administration of orexin-A, an agonist of the orexin receptors, causes a dose-related increase in arousal, largely at the expense of a reduction in paradoxical sleep and slow wave sleep 2, when administered at the onset of the normal sleep period. Therefore antagonists of its receptor may be useful in the treatment of sleep disorders including insomnia.

The present invention provides *N*-aroyl cyclic amine derivatives which are non-peptide antagonists of human orexin receptors, in particular orexin-1 receptors. In particular, these compounds are of potential use in the treatment of obesity, including obesity observed in Type 2 (non-insulin-dependent) diabetes patients, and/or sleep disorders Additionally these compounds are useful in stroke, particularly ischemic or haemorrhagic stroke, and/or blocking the emetic

response i.e. the compounds are useful in the treatment of nausea and vomiting.

International Patent Applications WO99/09024, WO99/58533, WO00/47577 and WO00/47580 disclose phenyl urea derivatives and WO00/47576 discloses quinolinyl cinnamide derivatives as orexin receptor antagonists.

According to the invention there is provided a compound of formula (I):

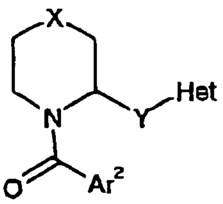

(I)

wherein:

X represents ($CH_2$);
Y represents $CH_2$, CO, CH(OH), or $CH_2CH(OH)$;
Het is an optionally substituted bicyclic heteroaryl group selected from the group consisting of quinoxalinyl, quinazolinyl, pyridopyrazinyl, benzoxazolyl benzothienyl, benzofuranyl, benzimidazolyl, naphthyridinyl, benzothiazolyl, indolyl, triazolopyridinyl, furopyridinyl, pyridopyrimidinyl, isoquinolinyl, quinolinyl, oxazolylpyridinyl, tetrabydrobenzimidazolyl tetrahydrobenzofuranyl or tetrahydrotriazolopyridinyl:
$Ar^2$ represents an optionally substituted phenyl or a 5- or 6-membered heterocyclyl group selected from the group consisting of furanyl, thienyl, pyrrolyl, oxazolyl, thiazolyl, imidazolyl, oxadiazolyl, thiadiazolyl, triazolyl, triazinyl, pyridazinyl, pyridinyl pyrimidinyl, isothiazolyl, isoxazolyl, pyrazinyl or pyrazolyl, wherein the phenyl or heterocyclyl group is substituted by $R^1$; or $Ar^2$ represents an optionally substituted bicyclic aromatic or bicyclic heteroaromatic group containing up to 3 heteroatoms selected from N, O and S;
$R^1$ represents hydrogen, optionally substituted($C_{1-4}$)alkoxy, halo, cyano, optionally substituted($C_{1-6}$)alkyl, optionally substituted phenyl, or an optionally substituted 5- or 6-membered heterocyclyl group containing up to 4 heteroatoms selected from N, O and S;

wherein the optional substituents for the groups Het, $Ar^2$ and $R^1$ are halogen, hydroxy, oxo, cyano, nitro, ($C_{1-4}$)alkyl, ($C_{1-4}$)alkoxy, hydroxy($C_{1-4}$)alkyl, hydroxy($C_{1-4}$)alkoxy, halo($C_{1-4}$)alkyl, halo($C_{1-4}$)alkoxy, aryl($C_{1-4}$)alkoxy, ($C_{1-4}$)alkylthio, hydroxy($C_{1-4}$)alkyl, ($C_{1-4}$)alkoxy($C_{1-4}$)alkyl, ($C_{3-6}$)cycloalkyl($C_{1-4}$)alkoxy, ($C_{1-4}$)alkanoyl, ($C_{1-4}$)alkoxycarbonyl, ($C_{1-4}$) alkylsulfonyl, ($C_{1-4}$)alkylsulfonyloxy, ($C_{1-4}$)alkylsulfonyl($C_{1-4}$)alkyl, arylsulfonyl, arylsulfonyloxy, arylsulfonyl($C_{1-4}$)alkyl, ($C_{1-4}$)alkylsulfonamido, ($C_{1-4}$)alkylamido, ($C_{1-4}$)alkylsulfonamido($C_{1-4}$)alkyl, ($C_{1-4}$)alkylamido($C_{1-4}$)alkyl, arylsulfonamido, arylcarboxamido, arylsulfonamido($C_{1-4}$)alkyl, arylcarboxamido($C_{1-4}$)alkyl, aroyl, aroyl($C_{1-4}$)alkyl, or aryl($C_{1-4}$)alkanoyl group; a group $R^aR^bN$-, $R^aCO(CH_2)_r$, $R^aCON(R^a)(CH_2)_r$, $R^aR^bNCO(CH_2)_r$, $R^aR^bNSO_2(CH_2)_r$ or $R^aSO_2NR^b(CH_2)_r$ where each of $R^a$ and $R^b$ independently represents a hydrogen atom or a ($C_{1-4}$)alkyl group or where appropriate $R^aR^b$ forms part of a ($C_{3-6}$) azacyloalkane or ($C_{3-6}$)(2-oxo)azacycloalkane ring and r represents zero or an integer from 1 to 4. Additional substituents are ($C_{1-4}$)acyl, aryl, aryl($C_{1-4}$)alkyl, ($C_{1-4}$)alkylamino($C_{1-4}$)alkyl $R^aR^bN(CH_2)n$-, $R^aR^bN(CH_2)_nO$-, wherein n represents an interger from 1 to 4. Additionally when the substituent is $R^aR^b(CH_2)n$-or $R^aR^bN(CH_2)nO$, $R^a$ with at least one $CH_2$ of the $(CH_2)n$ portion of the group form a ($C_{3-6}$)azacycloalkane and $R^b$ represents hydrogen, a ($C_{1-4}$)alkyl group or with the nitrogen to which it is attached forms a second ($C_{3-6}$)azacycloalkane fused to the first ($C_{3-6}$)azacycloalkane;
or a pharmaceutically acceptable salt thereof.

Preferably where $Ar^2$ represents phenyl or a 5- or 6-membered heterocyclyl group the $R^1$ group is situated adjacent to the point of attachment to the amide carbonyl.

Y is preferably $CH_2$.

Het may have up to 5, preferably 1, 2 or 3 optional substituents.

Preferably Het is benzofuranyl, benzoxazolyl, benzimidazolyl, furopyridinyl, benzothiazolyl, indolyl, benzothienyl, triazolopyridinyl, quinolinyl and tetrahydrotriazolopyryidinyl, more preferably benzimidazolyl, benzofuranyl, benzoxazolyl, even more preferably benzofuranyl or benzimidazolyl.

More specifically, examples of $Ar^2$ are thiazolyl, pyrazolyl, triazolyl, pyridazinyl, oxazolyl, pyridinyl, pyrimidinyl, isoxazolyl and thienyl.

When $R^1$ is a 5- or 6-membered heterocyclyl group containing up to 4 heteroatoms selected from N, O and S, it may be furanyl, thienyl, pyrrolyl, oxazolyl, thiazolyl, imidazolyl, oxadiazolyl, thiadiazolyl, pyridinyl, triazolyl, triazinyl,

pyridazinyl, pyrimidinyl, isothiazolyl, isoxazolyl, pyrazinyl or pyrazolyl. Additional heterocyclyl groups can be morpholinyl, piperazinyl and thiomorpholinyl. Furthermore it can be tetrazolyl, piperidinyl or pyrrolidinyl.

Preferably when $R^1$ is a 5- or 6-membered heterocyclyl group containing up to 4 heteroatoms selected from N, O and S, it may be oxadiazolyl, pyridinyl, pyrimidinyl, morpholinyl pyrazolyl or pyrrolyl.

Examples of where $Ar^2$ represents an optionally substituted bicyclic aromatic or bicyclic heteroaromatic include naphthyl, quinolinyl, napththyridinyl, benzofuranyl, benzimidazolyl, quinoxalinyl or quinazolinyl. Additionally $Ar^2$ may be isoquinolinyl or benzoxazolyl. Furthermore it can benzotriazolyl, benzothienyl, indolyl, benzothiazolyl, or benzothiadiazolyl.

Preferably $Ar^2$ represents optionally substituted phenyl, pyridinyl, thiazolyl, pyrazolyl, pyridazinyl, thienyl, naphthyl, triazolyl, isoxazolyl, quinolinyl, or isoquinolinyl.

More preferably $Ar^2$ represents optionally substituted phenyl, pyridinyl, thiazolyl, pyrazolyl, thienyl, triazolyl, quinolinyl, or isoquinolinyl.

Even more preferably $Ar^2$ represents optionally substituted phenyl, pyridinyl, thiazolyl, pyrazolyl, thienyl, or 1,2,3-triazolyl.

Alternatively $R^1$ represents hydrogen, optionally substituted$(C_{1-4})$alkoxy, halo, optionally substituted$(C_{1-6})$alkyl, optionally substituted phenyl, or an optionally substituted 5- or 6-membered heterocyclyl group containing up to 3 heteroatoms selected from N, O and S.

Preferably $R^1$ represents optionally substituted$(C_{1-4})$alkoxy, halo, optionally substituted$(C_{1-6})$alkyl, optionally substituted phenyl, or an optionally substituted 5- or 6-membered heterocyclyl group containing up to 3 heteroatoms selected from N, O and S.

Even more preferably $R^1$ represents an optionally substituted phenyl, pyridinyl, pyrazolyl pyrimidinyl or oxadiazolyl group.

Preferred optional substituents for $Ar^2$ are halogen, cyano, $(C_{1-4})$alkyl, hydroxy$(C_{1-4})$alkyl, $(C_{1-4})$alkoxy$(C_{1-4})$ alkyl, $R^aR^bN(CH_2)_n$ or $R^aR^bN$, more preferably halogen, cyano and $(C_{1-4})$alkyl. Additional substituents are $(C_{1-4})$acyl, $R^aR^bN(CH_2)$nO, $(C_{1-4})$alkoxy, phenyl, and $(C_{1-4})$alkylamido.

More preferred substituents for $Ar^2$ are $(C_{1-4})$alkyl, hydroxy$(C_{1-4})$alkyl, $R^aR^bN$, $(C_{1-4})$alkoxy, $R^aR^bN(CH_2)$n, $(C_{1-4})$acyl, and $(C_{1-4})$alkylamido.

Preferred optional substituents for Het are halogen, cyano, $(C_{1-4})$alkyl, hydroxy$(C_{1-4})$alkyl, $(C_{1-4})$acyl, $(C_{1-4})$ alkoxy$(C_{1-4})$alkyl, $R^aR^bNCO(CH_2)_r$, $R^aR^bN(CH_2)$n, $R^aR^bN(CH_2)$nO or $R^aR^bN$. Additional optional substituents are $(C_{1-4})$ alkoxy or $CF_3$.

More preferred substituents for Het are halogen, $R^aR^bNCO(CH_2)_r$, cyano, $(C_{1-4})$alkoxy. $(C_{1-4})$alkyl, $R^aR^bN$ $(CH_2)$n, hydroxy$(C_{1-4})$alkyl, $(C_{1-4})$acyl or $(C_{1-4})$alkoxy$(C_{1-4})$aJkyl.

Preferred optional substituents for $R^1$ are halogen, $(C_{1-4})$alkoxy$(C_{1-4})$alkyl, $R^aR^bN$, $R^aR^bN(CH_2)$nO or $R^aR^bN$ $(CH_2)$n. Additional optional sustituents are $(C_{1-4})$alkyl, $(C_{1-4})$alkoxy and $(C_{1-4})$acyl.

More preferred substituents for $R^1$ are halogen, $R^aR^bN(CH_2)$nO, $(C_{1-4})$alkyl, and $(C_{1-4})$alkoxy.

In the groups Het and $Ar^2$, substituents positioned *ortho* to one another may be linked to form a ring.

Illustrative compounds of formula (I) can be selected from:

| | |
|---|---|
| 1 | (RS)-2-(2-Benzofuranylmethyl)-1-((5-(4- fluorophenyl)-2-methyl-thiazol-4-yl)-carbonyl)-piperidine |
| 2 | (RS)-1-(2-Benzooxazol-2-ylmethyl-piperidin-1-yl)-1-[5-(4-fluoro-phenyl)-2-methyl-thiazol-4-yl]-methanone |
| 3 | (RS)-1 -(2-Benzooxazol-2-ylmethyl-piperidin-1-yl)- 1-[2-(3-methyl-[1,2,4]oxadiazol-5-yl)-phenyl]-methanone |

and pharmaceutical acceptable salts thereof.

Additional compounds of formula (I) can be selected from:

| | |
|---|---|
| 4 | (RS)-1-(2-Benzofiuan-2-ylmethyl-piperidin-1-yl)-1-[4-(4-fluoro-phenyl)-1-methyl-1-*H*-pyrazol-3-yl]-methane |
| 5 | (RS)-1-[2-(5-Fluoro-benzofuran-2-ylmethyl)-piperidin-1-yl]-1-[5-(4-fluoro-phenyl)-2-methyl-thiazol-4-yl]-methanone |
| 6 | (RS)-1-[2-(5-Fluoro-benzofuran-2-ylmethyl)-piperidin-1-yl]-1-(5-pyridin-2-yl-thiazol-4-yl)-methanone |
| 7 | (RS)-1-[2-(5-Fluoro-benzofuran-2-ylmethyl)-piperidin-1-yl]-1-quinolin-4-yl-methanone |
| 8 | (RS)-1-[2-(5-Fluoro-benzofuran-2-ylmethyl)-piperidin-1-yl]-1-quinolin-5-yl-methanone |
| 9 | (RS)-1-[2-(5-Fluoro-benzofuran-2-ylmethyl)-piperidin-1-yl]-1-(2-methoy-pyridin-3-yl)-methanone |

Table continued

| | |
|---|---|
| 10 | (RS)-1-[2-Dimethylamino-5-(4-fluoro-phenyl)-thiazol-4-yl]-1-[2-(5-fluoro-benzofuran-2-ylmethyl)-piperidin-1-yl]-methanone |
| 11 | (RS)-1-[2-(5-Fluoro-benzofuran-2-ylmethyl)-piperidin-1-yl]-1-(2-morpholin-4-yl-phenyl)-methanone |
| 12 | (RS)-1-[2-(5-Fluoro-benzofuran-2-ylmethyl)-piperidin-1-yl]-1-(2-trifluoromethoxy-phenyl)-methanone |
| 13 | (RS)-1-(2-Benzofuran-2-ylmethyl-piperidin-1-yl)-1-[2-(2-dimethylamino-ethoxy)-phenyl]-methanone |
| 14 | (RS)-1-(2-Benzofuran-2-ylmethyl-piperidin-1-yl)-1-quinolin-8-yl-methanone |
| 15 | (RS)-1-(2-Benzofuran-2-ylmethyl-piperidin-1-yl)-1-(2-pyrimidin-2-yl-phenyl)-methanone |
| 16 | (RS)-1-(2-Benzofuran-2-ylmethyl-piperidin-1-yl)-1-[5-(4-fluoro-phenyl)-2H-[1,2,3]triazol-4-yl]-methanone |
| 17 | (RS)-1-(2-Benzofuran-2-ylmethyl-piperidin-1-yl)-1-[5-(4-fluoro-phenyl)-1H-pyrazol-3-yl]-methanone |
| 18 | (RS)-1-(2-Benzofuran-2-ylmethyl-piperidin-1-yl)-1-[5-(4-fluoro-phenyl)-2-hydroxymethyl-thiazol-4-yl]-methanone |
| 19 | (RS)-1-(2-Benzofuran-2-ylmethyl-piperidin-1-yl)-1-isoquinolin-8-yl-methanonc |
| 20 | (RS)-1-(2-Benzofuran-2-ylmethyl-piperidin-1-yl)-1-isoquinolin-5-yl-methanone |
| 21 | (RS)-1-(2-Benzofuran-2-ylmethyl-piperidin-1-yl)-1-{5-[3-(3-dimethylamino-propoxy)-phenyl]-2-methyl-thiazol-4-yl}-methanone |
| 22 | (RS)-1-(2-Benzofuran-2-ylmethyl-piperidin-1-yl)-1-{5-[3-(4-dimethylamino-butoxy)phenyl]-2-methyl-thiazol-4-yl}-methanone |
| 23 | (RS)-1-[5-(4-Fluoro-phenyl)-2-methyl-thiazol-4-yl]-1-(2-furo[2,3-b]pyridin-2-ylmethyl-piperidin-1-yl)-methanone |
| 24 | (RS)-1-[4-(4-Fluoro-phenyl)-1-methyl-1-methyl-1*H*-pyrazole-3-yl]-(2-furo[2,3-*b*]pyridin-2-ylmethyl-piperidin-1-yl)methanone |
| 25 | (RS)-1-[5-(4-Fluoro-phenyl)-2-methyl-thiazol-4-yl]-1-(2-quinolin-2-ylmethyl-piperidin-1-yl)-methanone |
| 26 | (RS)-1-Benzofuran-2-yl-1-(1-{1-[5-(4-fluoro-phenyl)-2-methyl-thiazol-4-yl]-methanoyl}-piperidin-2-yl)-methanone |
| 27 | (RS)-1-[2-(1*H*-benzoimidazol-2-ylmethyl)-piperidin-1-yl]-1-[:5-(4-fluoro-phenyl)-2-methyl-thiazol-4-yl]-methanone |
| 28 | (RS)-1-[2-(S-Chloro-1H-benzoimidazol-2-ylmethyl)-piperidin-1-yl]-1-[5-(4-fluoro-phenyl)-2-methyl-thiazol-4-yl]-methanone |
| 29 | (RS)-1-[2-(5-Fluoro-1H-benzoimidazol-2-ylmethyl)-pipendin-1-yl]-1-[5-(4-fluoro-phenyl)-2-methyl-thiazol-4-yl]-methanone |
| 30 | (RS)-1-[2-(5-Chloro-6-fluoro-1H-benzoimidazol-2-ylmethyl)-piperidin-1-yl]-1-[5-(4-ftuoro-phenyl)-2-methyl-thiazol-4-yl]-methanone |
| 31 | (RS)-1-[5-(4-Fluoro-phenyl)-2-methyl-thiazol-4-yl]-1-[2-(4-methyl-1H-benzoimidazol-2-ylmethyl)-piperidin-1-yl]-methanone |
| 32 | (RS)-1-[2-(4,6-Difluoro-1H-benzoimidazol-2-ylmethyl)-piperidin-1-yl]-1-[5-(4- |
| | fluoro-phenyl)-2-methyl-thiazol-4-yl]-methanone |
| 33 | (RS)-1-[2-(4-Dimethylaminomethyl-1*H*-benzoimidazol-2-ylmethyl)-piperidin-1-yl]-1-[5-(4-fluoro-phenyl)-2-methyl-thiazol-4-yl]-methanone |
| 34 | (RS)-1-[2-(5-Bromo-1H-benzoimidazol-2-ylmethyl)-piperidin-1-yl]-1-[5-(4-fluoro-phenyl)-2-methyl-thiazol-4-yl]-methanone |
| 35 | (RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone |

Table continued

| | |
|---|---|
| 36 | (RS)-1-[2-(4,5-Difluoro-1*H*-benzoimidazol-2-ylmethyl)-piperidin-1-yl]-1-[5-(4-fluoro-phenyl)-2-methyl-thiazol-4-yl]-methanone |
| 37 | (RS)-1-[2-(5,6-Difluoro-1*H*-benzoimidazol-2-ylmethyl)-piperidin-1-yl]-1-[5-(4-fluoro-phenyl)-2-hydroxymethyl-thiazol-4-yl]-methanone |
| 38 | (RS)-1-[2-(4,5-Difluoro-1*H*-benzoimidazol-2-ylmethyl)-piperidin-1-yl]-1-[5-(4-fluoro-phenyl)-2-hydroxymethyl-thiazol-4-yl]-methanone |
| 39 | (RS)-1-[2-(5,6-Difluoro-1*H*-benzoimidazol-2-ylmethyl)-piperidin-1-yl]-1-[4-(4-fluoro-phenyl)-1-methyl-1H-pyrazol-3-yl]-methanone |
| 40 | (RS)-1-[2-(5-Methoxy-1*H*-benzoimidazol-2-ylmethyl)-piperidin-1-yl]-1-[5-(4-fluoro-phenyl)-2-methyl-thiazol-4-yl]-methanone |
| 41 | (RS)-1-[2-(6,7-Difluoro-1H-benzoimidazol-2-ylmethyl)-piperidin-1-yl]-1-(2-methyl-5-phenyl-thiazol-4-yl)-methanone |
| 42 | (RS)-1-[2-(6,7-Difluoro-1H-benzoimidazol-2-ylmethyl)-piperidin-1-yl]-1-[4-(4-fluoro-phenyl)-1-methyl-1H-pyrazol-3-yl]-methanone |
| 43 | (RS)-1-[2-(6,7-Difluoro-1H-benzoimidazol-2-ylmethyl)-piperidin-1-yl]-1-[4-(4-fluoro-phenyl)-1 H-pyrazol-3-yl]-methanone |
| 44 | (RS)-1-[2-(4,5-Difluoro-1H-benzoimidazol-2-ylmethyl)-piperidin-1-yl]-1-{5-[3-(2-dimethylamino-ethoxy)-phenyl]-2-methyl-thiazol-4-yl}-methanone |
| 45 | (RS)-1-[2-(4,5-Difluoro-1H-benzoimidazol-2-ylmethyl)-piperidin-1-yl]-1-[5-(2-fluoro-phenyl)-2-methyl-thiazol-4-yl]-methanone |
| 46 | (RS)-1-[2-(4,5-Difluoro-1H-benzoimidazol-2-ylmethyl)-piperidin-1-yl]-1-[5-(3-fluoro-phenyl)-2-methyl-thiazol-4-yl]-methanone |
| 47 | (RS)-1-[2-(4,5-Difluoro-1H-benzoimidazol-2-ylmethyl)-piperidin-1-yl]-(2-trifluoromethoxy-phenyl)-methanone |
| 48 | (RS)-1-[2-(4,5-Difluoro-1H-benzoimidazol-2-ylmethyl)-piperidin-1-yl]-1-(2-ethoxy-phenyl)-methanone |
| 49 | (RS)-1-[2-(4,5-Difluoro-1H-benzomidazol-2-ylmethyl)-piperidin-1-yl]-1-(2,6-dimethoxy-phenyl)-methanone |
| 50 | (RS)-1-[2-(6,7-Difluoro-1H-benzoimidazol-2-ylmethyl)-piperidin-1-yl]-1-(2-pyrazot-1-yl-phenyl)-methanone |
| 51 | (RS)-1-[2-(5,6-Difluoro-1H-benzoimidazol-2-ylmethyl)-piperidin-1-yl]-1-[4-(4-fluoro-phenyt)-1H-pyrazol-3-yl]-methanone |
| 52 | (RS)-1-[2-(5,6-Difluoro-1H-benzoimidazol-2-ylmethyl)-piperidin-1-yl]-1-[2-(3-methyl-[1,2,4]oxadiazol-5-yl)-phenyl]-methanone |
| 53 | (RS)-1-[2-(Difluoro-1H-benzoimidazol-2-ylmethyl)-piperidin-1-yl]-1-[1-(2-dimethytamino-ethyl)-4-(4-fluoro-phenyl)-1H-pyrazol-3-yl]-methanone |
| 54 | (RS)-1-[2-(5.6-Difluoro-1H-benzoimidazol-2-ylmethyl)-piperidin-1-yl]-1-[5-(4-fluoro-phenyl)-2-methyl-2H-[1,2,3]triazol-4-yl]-methanone |
| 55 | (RS)-1-[2-(5.6-Difluoro-1H-benzoimidazol-2-ylmethyl)-piperidin-1-yl]-1- {2-[3-(3-dimethylamino-propoxy)-phenyl]-thuophen-3-yl}-methanone |
| 56 | (RS)-1-[2-(5,6-Difluoro-1-methyl-1*H*-benzoimidazol-2-ylmethyl)-piperidin-1-yl]-1-[S-(4-fluoro-phenyl)-2-methyl-thiazol-4-yl]-methanone |
| 57 | (RS)-1-(2-Benzothiazol-2-ylmethylpiperidin-1-yl)-1-[5-(4-fluorophenyl)-2-methylthiazol-4-yl]-methanone |
| 58 | (RS)-1-(2-Benzothiazol-2-ylmethylpiperidin-1-yl)-1-[4-(4-fluorophenyl)-1-methyl- |
| | 1-*H*-pyrazol-3-yl]-methanone |
| 59 | (RS)-1-(2-Benzothiazol-2-ylmethylpiperidin-1-yl)-1-[2-(3-methyl-[1,2,4]oxadiazol-5-yl)phenyl]-methanone |
| 60 | (RS)-1-[5-(4-Fluorophenyl)-2-methylthiazol-4-yl]-1-[2-(5,6,7,8-tetrahydro-[1,2,4]-triazolo[1,5-a]pyridin-2-ylmethyl)-piperidin-1-yl]-methanone |

Table continued

| | |
|---|---|
| 61 | (RS)-1-[5-(4-Fluorophenyl)-2-methylthiazol-4-yl]-1-(2-[1,2,4]triazolo[1,5-a]pyridin-2-ylmethylpiperidin-1-yl)-methanone |
| 62 | 1-[(RS)-2-((RS)-2-Benzofuran-2-yl-2-hydroxy-ethyl)-piperidin-1-yl]-1-[5-(4-fluoro-phenyl)-2-methyl-thiazol-4-yl]-methanone (as separate diastereoisomers) |
| 63 | (RS)-1-[2-(4-Bromo-1*H*-benzoimidazol-2-ylmethyl)-piperidin-1-yl]-1-[5-(4-fluorophenyl)-2-methylthiazol-4-yl] methanone |
| 64 | (RS)-1-[2-(4-Cyano-1*H*-benzoimidazol-2-ylmethyl)-piperidin-1-yl]-1-[5-(4-fluorophenyl)-2-methylthiazol-4-yl] methanone |
| 65 | (RS)-1-[2-(4-Acetyl-1*H*-benzoimidazol-2-ylmethyl)-piperidin-1-yl]-1-[5-(4-fluorophenyl)-2-methylthiazol-4-yl] methanone |
| 66 | (RS)-2-(1- {1-[5-(4-Fluoro-phenyl)-2-methyl-thiazol-4-yl]-methanoyl}-piperidin-2-ylmethyt)-1H-benzoimidazole-5-carbonitrile |
| 67 | (RS)-1-[2-(5,6-Difluoro-1-propyl-1*H*-benzoimidazol-2-ylmethyl)-piperidin-1-yl]-1-[5-(4-fluoro-phenyl)-2-methyl-thiazol-4-yl]-methanone |
| 68 | (RS)-1-{2-[5,6-Difluoro-1-(2-methoxy-ethyl)-1*H*-benzoimidazol-2-ylmethyl]-piperidin-1-yl}-1-[5-(4-fluoro-phenyl)-2-methyl-thiazol4-yl]-methanone |
| 69 | (RS)-1-{2-[1-(2-Dimethytanuno-ethyl)-5,6-dtftuoro-1*H*-benzoinudazol-2-ylmethyl]-piperidin-1-yl}-1-[5-(4-fluoro-phenyl)-2-methyl-thiazol-4-yl]-methanone |
| 70 | (RS)-1-{2-[5,6-difluoro-1-(2-hydroxy-ethyl)-1*H*-benzoimidazol-2-ylmethyl]-piperidin-1-yl}-1-[5-(4-fluoro-phenyl)-2-methyt-thiazot-4-yl]-methanone |
| 71 | (RS)-1-[2-(6,7-Difluoro-1-methyl-1*H*-benzomudazol-2-ylmethyl)-piperidin-1-yl]-1-[5-(4-fluorophenyl)-2-methyl-thiazol-4-yl]-methanone |
| 72 | (RS)-1-[2-(4,5-Difluoro-1-methyl-1H-benzoimidazol-2-ylmethyl)-piperidin-1-yl]-1-[5-(4-fluorophenyl)-2-methyl-thiazol-4-yl]-methanone |
| 73 | (RS)-2-(1-{1-[5-(4-Fluorophenyl)-2-methyl-thiazol-4-yl]-methanoyl}-piperidin-2-ylmethyl)-benzofuran-3-carboxylic acid amide |
| 74 | (RS)-2-(1-{1-[4-(4-Fluorophenyl)-1-methyl-1*H*-pyrazol-3-yl]-methanoyl}-piperidin-2-ylmethyl)-benzofuran-3-carboxylic acid amide |
| 75 | (RS)-1-[5-(4-Fluoro-phenyl)-2-methyl-thiazol-4-yl]-1-(2-furo[3,2-b]-pyridin-2-ylmethyl-piperidin-1-yl) methanone |
| 76 | (RS)-1-[4-(4-Fluoro-phenyl)-1-methyl-1H-pyrazole-3-yl)-(2-furo[3,2-b]pyridin-2-ylmethyl-piperidin-1-yl) methanone |
| 77 | (RS)-1-[2-(3-Brorno-benzofuran-2-ylmethyl)-piperidin-1-yl]-1-[5-(4-fluoro-phenyl)-2-methyl-thiazol-4-yl]-methanone |
| 78 | (RS)-2-(1-{1-[5-(4-Fluoro-phenyl)-2-methyl-thiazol-4-yl]-methanoyl}-piperidin-2-ylmethyl)-benzofuran-3-carbonitrile |
| 79 | (RS)-1-[2-(1-{1-[5-(4-fluoro-phenyl)-2-methyl-thiazol-4-yl]-methanoyl}-piperidin-2-ylmethyl)-benzofuran-3-yl]-ethanone |
| 80 | (R)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanoneand(S)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone |

and pharmaceutically acceptable salts thereof.

Further compounds of formula (I) can be selected from

| | |
|---|---|
| 81 | (RS)-1-(2-Benzofuran-2-ylmethyl-piperidin-1-yl)-1-[5-(4-methoxy-phenyl)-2-methyl-thiazol-4-yl]-methanone |

Table continued

| | |
|---|---|
| 82 | (RS)-1-(2-Benzofuran-2-ylmethyl-piperidin-1-yl)-1-{5-[3-(2-dimethylamino-ethoxy)-phenyl]-2-methyl-thiazol-4-yl}-methanone |
| 83 | (RS)-1-[2-(4,5-Difluoro-1H-benzoimidazol-2-ylmethyl)-piperidin-1-yl]-1-(5-methyl-3-phenyl-isoxazol-4-yl)-methanone |
| 84 | 1-[3-(2,6-Dichloro-phenyl)-5-methyl-isoxazol-4-yl]-1-[2-(4,5-difluoro-1H-benzoimidazol-2-ylmethyl)-piperidin-1-yl]-methanone |
| 85 | (RS)-1-[2-(4,5-Difluoro-1H-benzoimidazol-2-ylmethyl)-piperidin-1-yl]-1-[4-(4-fluoro-phenyl)-2-methyl-2H-pyrazol-3-yl]-methanone |
| 86 | (RS)-1-[3-(2-Chloro-6-fluoro-phenyl)-5-methyl-isoxazol-4-yl]-1-[2-(4,5-difluoro-1H-benzoimidazol-2-ylmethyl)-piperidin-1-yl]-methanone |
| 87 | (RS)-1-[3-(2-Chloro-phenyl)-5-methyl-isoxazol-4-yl]-1-[2-(4,5-difluoro-1H-benzoimidazol-2-ylmethyl)-piperidin-1-yl]-methanone |
| 88 | (RS)-1-[2-(4,5-Difluoro-1H-benzoimidazol-2-ylmethyl)-piperidin-1-yl]-1-(2-propoxy-phenyl)-methanone |
| 89 | (RS)-1-[2-(4,5-Difluoro-1H-benzoimidazol-2-ylmethyl)-piperidin-1-yl]-1-(2-isopropoxy-phenyl)-methanone |
| 90 | (RS)-1-(2-Benzyloxy-phenyl)-1-[2-(4,5-difluoro-1H-benzoimidazol-2-ylmethyl)-piperidin-1-yl]-methanone |
| 91 | (RS)-1-[2-(4,5-Difluoro-1H-benzoimidazol-2-ylmethyl)-piperidin-1-yl]-1-(2-ethoxy-6-methoxy-phenyl)-methanone |
| 92 | (RS)-1-[2-(4,5-Difluoro-1H-benzoimidazol-2-ylmethyl)-piperidin-1-yl]-1-(2-ethoxy-6-methyl-phenyl)-methanone |
| 93 | (RS)-1-(2,6-Diethoxy-phenyl)-1-[2-(4,5-difluoro-1H-benzoimidazol-2-ylmethyl)-piperidin-1-yl]-methanone |
| 94 | 1-(3-{ 1-[2-(4,5-Difluoro-1H-benzoimidazol-2-ylmethyl)-piperidin-1-yl]-methanoyl}-4-ethoxy-phenyl)-ethanone |
| 95 | (RS)-1-[2-(4,5-Difluoro-1H-benzoimidazol-2-ylmethyl)-pipendin-1-yl]-1-(2-ethoxy-naphthalen-1-yl)-methanone |
| 96 | (RS)-1-[2-(4,5-Difluoro-1H-benzoimidazol-2-ylmethyl)-piperidin-1-yl]-1-(2-pyridin-2-yl-phenyl)-methanone |
| 97 | 1-[2-(6,7-Difluoro-1H-benzoimidazol-2-ylmethyl)-piperidin-1-yl]-1-(2-pyrrol-1-yl-phenyl)-methanone |
| 98 | (RS)-1-[2-(4,5-Difluoro-1H-benzoimidazol-2-ylmethyl)-piperidin-1-yl]-1-{5-[3-(3-dimethylamino-propoxy)-phenyl]-2-methyl-thiazol-4-yl}-methanone |
| 99 | (RS)-1-[2-(4,5-Difluoro-1H-benzoimidazol-2-ylmethyl)-piperidin-1-yl]-1-{5-[3-(4-dimethylamino-butoxy)-phenyl]-2-methyl-thiazol-4-yl}-methanone |
| 100 | (RS)-1-[2-(5,6-Difluoro-1H-benzoimidazol-2-ylmethyl)-piperidin-1-yl]-1-[5-(4-fluoro-phenyl)-2-methoxy-thiazol-4-yl]-methanone |
| 101 | (RS)-1-[2-(5,6-Difluoro-1H-benzoimidazol-2-ylmethyl)-piperidin-1-yl]-1-[2-ethyl-5-(4-fluoro-phenyl)-thiazol-4-yl]-methanone |
| 102 | (RS)-1-[2-(5,6-Difluoro-1H-benzoimidazol-2-ylmethyl)-piperidin-1-yl]-1-[5-(2-fluoro-phenyl)-2-methyl-thiazol-4-yl]-methanone |
| 103 | (RS)-1-[2-(5,6-Difluoro-1H-benzoimidazol-2-ylmethyl)-piperidin-1-yl]-1-[5-(3-fluoro-phenyl)-2-methyl-thiazol-4-yl]-methanone |
| 104 | (RS)-1-[2-(4-Fluoro-1H-benzoimidazol-2-ylmethyl)-piperidin-1-yl]-1-[5-(4-fluoro-phenyl)-2-methyl-thiazol-4-yl]-methanone |
| 105 | (RS)-1-[2-(4-Fluoro-1H-benzoimidazol-2-ylmethyl)-piperidin-1-yl]-1-[4-(4-fluoro-phenyl)-1-methyl-1H-pyrazol-3-yl]-methanone |
| 106 | (RS)-1-[2-(4-Fluoro-1H-benzoimidazol-2-ylmethyl)-piperidin-1-yl]-1-[4-(4-fluoro-phenyl)-1 H-pyrazol-3-yl]-methanone |

Table continued

| | |
|---|---|
| 107 | (RS)-1-[2-(4-Fluoro-1H-benzoimidazol-2-ylmethyl)-piperidin-1-yl]-1-[4-(4-fluoro-phenyl)-2-methyl-2H-pyrazol-3-yl]-methanone |
| 108 | (RS)-1-(2-Ethoxy-phenyl)-1-[2-(4-fluoro-1 H-benzoimidazol-2-ylmethyl)-piperidin-1-yl]-methanone |
| 109 | (RS)-1-[2-(4-Fluoro-1H-benzoimidazol-2-ylmethyl)-piperidin-1-yl]-1-[5-(4-fluoro-phenyl)-2-hydroxymethyl-thiazol-4-yl]-methanone |
| 110 | (RS)-1-[5-(4-Fluoro-phenyl)-2-methyl-thiazol-4-yl]-1-{2-[4-(1-hydroxy-ethyl)-1*H*-benzoimidazol-2-ylmethyl]-piperidin-1-yl}-methanone |
| 111 | (RS)- 1-(2-Benzofuran-2-ylmethyl-piperidin-1-yl)-1-[5-(4-chloro-phenyl)-2-methyl-thiazol-4-yl]-methanone |
| 112 | (RS)-1-[2-(3-Chloro-furo[3,2-b]pyridin-2-ylmethyl)-piperidin-1-yl]-1-[5-(4-fluoro-phenyl)-2-methyl-thiazol-4-yl]-methanone |
| 113 | (RS)-1-[2-(5,6-Difluoro-1-methyl-1H-benzoimidazol-2-ylmethyl)-piperidin-1-yl]-1-[5-(4-fluoro-phenyl)-2-hydroxymethyl-thiazol-4-yl]-methanone |
| 114 | (RS)-1-[5-(4-Fluoro-phenyl)-2-methyl-thiazol-4-yl]-1-[2-(1H-indol-2-ylmethyl)-piperidin-1-yl]-methanone |
| 115 | (RS)-5-[1-(2-Benzofuran-2-ylmethyl-piperidin-1-yl)-methanoyl]-4*H*-benzo[1,4]oxazin-3-one |
| 116 | (RS)-1-[2-(5-Bromo-benzofuran-2-ylmethyl)-pipendin-1-yl]-1-[5-(4-fluoro-phenyl)-2-methyl-thiazol-4-yl]-methanone |
| 117 | (RS)-1-[2-(5-Cyano-benzofuran-2-ylmethyl)-pipendin-1-yl]-1-[5-(4-fluoro-phenyl)-2-methyl-thiazol-4-yl]-methanone |
| 118 | (RS)-1-[2-(4-Bromo-benzofuran-2-ylmethyl)-piperidin-1-yl]-1-[5-(4-fluoro-phenyl)-2-methyl-thiazol-4-yl]-methanone |
| 119 | (RS)-1-[2-(4-Cyano-benzofuran-2-ylmethyl)-piperidin-1-yl]-1-[5-(4-fluoro-phenyl)-2-methyl-thiazol-4-yl]-methanone |
| 120 | (RS)-1-[5-(4-Fluoro-phenyl)-2-methyl-thiazol-4-yl]-1-[2-(3-methyl-benzofuran-2-ylmethyl)-piperidin-1-yl]-methanone |
| 121 | (RS)-1-[2-(4-Fluoro-benzofuran-2-ylmethyl)-piperidin-1-yl]-1-[5-(4-fluoro-phenyl)-2-methyl-thiazol-4-yl]-methanone |
| 122 | (RS)-1-[2-(4-Fluoro-benzofuran-2-ylmethyl)-piperidin-1-yl]-1-[4-(4-fluoro-phenyl)-1-methyl-1H-pyrazol-3 -yl]-methanone |
| 123 | (RS)- 1-(2-Benzo[b] thiophen-2-ylmethyl-piperidin-1-yl)-1-[5-(4-fluoro-phenyl)-2-methyl-thiazol-4-yl]-methanone |
| 124 | (RS)-1-(2-Benzo[b]thiophen-2-ylmethyl-piperidin-1-yl)-1-[4-(4-fluoro-phenyl)-1-methyl-1H-pyrazol-3-yl]-methanone |
| 125 | (RS)-1-(2-Benzo[b]thiophen-2-ylmethyl-piperidin-1-yl)-1-quinolin-8-yl-methanone |
| 126 | (RS)-1-(2-Benzofuran-2-ylmethyl-piperidin-1-yl)-1-(2-methyl-5-phenyl-thiazol-4-yl)-methanone |
| 127 | (RS)-1-(2-Benzofuran-2-ylmethyl-piperidin-1-yl)-1-[2-(3-methyl-[1,2,4]oxadiazol-5-yl)-phenyl]-methanone |
| 128 | (R)-1-[2-(4,6-Difluoro-1*H*-benzoimidazol-2-ylmethyl)-piperidin-1-yl]-1-[5-(4-fluoro-phenyl)-2-methyl-thiazol-4-yl]-methanone and (S)-1-[2-(4,6-Difluoro-1*H*-benzoimidazol-2-ylmethyl)-piperidin-1-yl]-1-[5-(4-fluoro-phenyl)-2-methyl-thiazol-4-yl]-methanone |

and pharmaceutically acceptable salts thereof.

When a halogen atom is present in the compound of formula (I) it may be fluorine, chlorine, bromine or iodine.

When the compound of formula (1) contains an alkyl group, whether alone or forming part of a larger group, e.g. alkoxy or alkylthio, the alkyl group may be straight chain, branched or cyclic, or combinations thereof, it is preferably methyl or ethyl.

When used herein the term aryl means a 5- to 6- membered ring for example phenyl, or a 7- to 12 membered bicyclic ring system where at least one of the rings is aromatic for example naphthyl.

When used herein the term bicyclic hetero aryl means a 7- to 12 membered bicyclic ring system where at least one of the rings is aromatic for example benzimidazoyl, or tetrahydrobenzimidazolyl.

It will be appreciated that compounds of formula (I) may exist as *R* or *S* enantiomers. The present invention includes within its scope all such isomers, including mixtures. Where additional chiral centres are present in compounds of formula (I), the present invention includes within its scope all possible diastereoismers, including mixtures thereof. The different isomeric forms may be separated or resolved one from the other by conventional methods, or any given isomer may be obtained by conventional synthetic methods or by stereospecific or asymmetric syntheses.

It will be understood that the invention includes pharmaceutically acceptable derivatives of compounds of formula (I) and that these are included within the scope of the invention.

Particular compounds according to the invention include those mentioned in the examples and their pharmaceutically acceptable derivatives.

As used herein "pharmaceutically acceptable derivative" includes any pharmaceutically acceptable salt, ester or salt of such ester of a compound of formula (I) which, upon administration to the recipient is capable of providing (directly or indirectly) a compound of formula (I) or an active metabolite or residue thereof.

It will be appreciated that for use in medicine the salts of the compounds of formula (I) should be pharmaceutically acceptable. Suitable pharmaceutically acceptable salts will be apparent to those skilled in the art and include acid addition salts formed with inorganic acids e.g. hydrochloric, hydrobromic, sulphuric, nitric or phosphoric acid; and organic acids e.g. succinic, maleic, acetic, fumaric, citric, tartaric, benzoic, p-toluenesulfonic, methanesulfonic or naphthalenesulfonic acid. Other salts e.g. oxalates, may be used, for example in the isolation of compounds of formula (I) and are included within the scope of this invention. Also included within the scope of the invention are solvates and hydrates of compounds of formula (I).

Certain of the compounds of formula (I) may form acid addition salts with one or more equivalents of the acid. The present invention includes within its scope all possible stoichiometric and non-stoichiometric forms.

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

According to a further feature of the invention there is provided a process for the preparation of compounds of formula (I) and salts thereof. The following schemes detail some synthetic routes to compounds of the invention.

**Scheme 1**

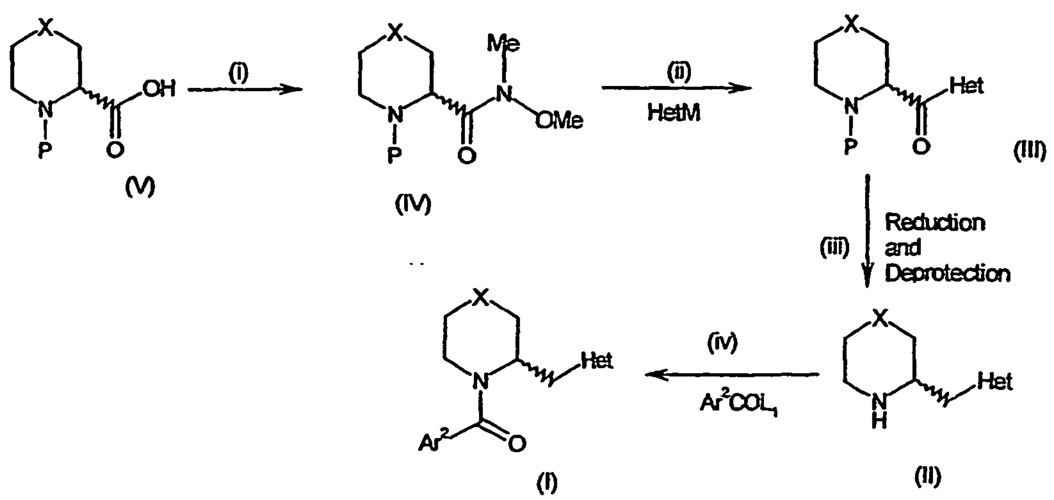


wherein, X, Het, and $Ar^2$ are as defined for compounds of formula (I), P is a protecting group and M is a metal for example lithium.

Examples of suitable leaving groups $L_1$ include halogen, OC(=O)alkyl and OC(=O)O-alkyl. The transformation (II) to (I) may be carried out in an inert solvent such as dichloromethane, in the presence of a base such as triethylamine. Alternatively this step may be carried out when $L^1$ represents hydroxy, in which case reaction with (II) takes place in an inert solvent such as dichloromethane in the presence of a diimide reagent such as 1-ethyl-3-(3-dimethylaminopropyl) carbodiimide hydrochloride, and an activator such as 1-hydroxybenzotriazole.

Examples of protecting groups P include *t*-butyloxycarbonyl, trifluoroacetyl and benzyloxycarbonyl. Deprotection conditions are respectively, acid (e.g. trifluoroacetic acid in dichloromethane), base (e.g. sodium hydroxide in a solvent such as aqueous methanol) and catalytic hydrogenolysis in an inert solvent (e.g using palladium on charcoal in a lower alcohol or ethyl acetate).

Compounds of formula (V) are known in the literature or can be prepared by known methods.

**Scheme 2**

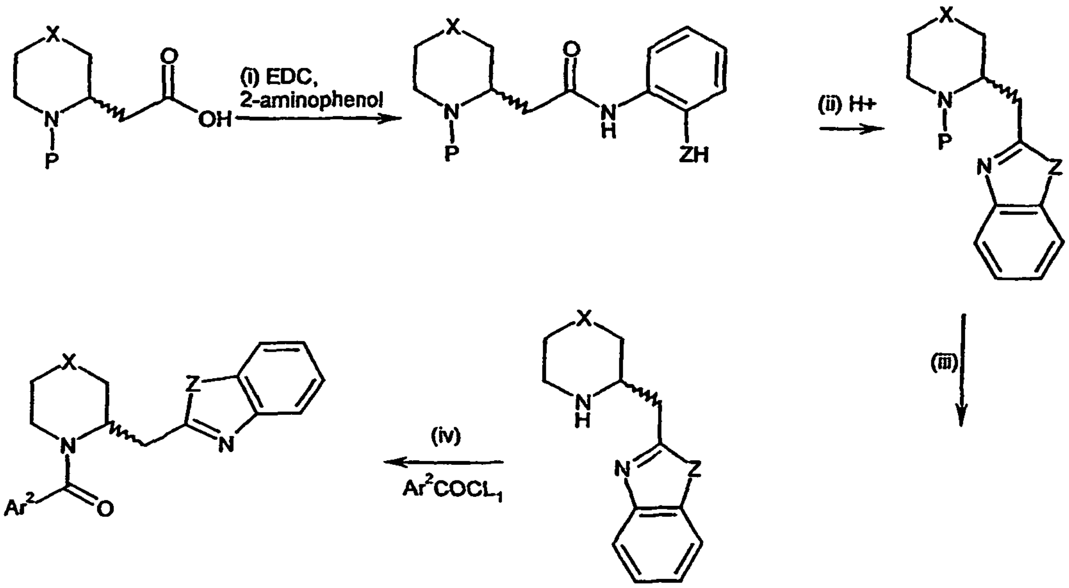


wherein, X, and Ar$^2$ are as defined for compounds of formula (I), Z is S, or O, and P is a protecting group.

**Scheme 3**

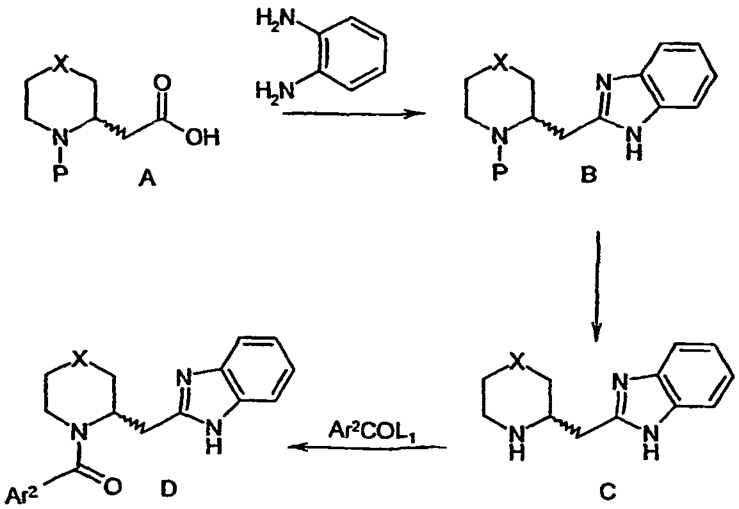


wherein, X, and Ar$^2$ are as defined for compounds of formula (I) and P is a protecting group.

Examples of protecting groups P include *t*-butyloxycarbonyl, trifluoroacetyl and benzyloxycarbonyl. Deprotection conditions are respectively, acid (e.g. trifluoroacetic acid in dichloromethane), base (e.g. sodium hydroxide in a solvent such as aqueous methanol) and catalytic hydrogenolysis in an inert solvent (e.g using palladium on charcoal in a lower alcohol or ethyl acetate).

The transformation of A to B can be carried out at elevated temperature in the absence of solvent or in the

presence of an acid such as sulfuric acid or polyphosphoric acid, usually at elevated temperature. Deprotection can occur in situ under acidic conditions, if for example P is t-butoxycarbonyl to afford C directly.

Examples of suitable leaving groups $L_1$ include halogen, OC(=O)alkyl and OC(=O)O-alkyl. The transformation C to D may be carried out in an inert solvent such as dichloromethane, in the presence of a base such as triethylamine. Alternatively this step may be carried out when $L_1$ represents hydroxy, in which case reaction with C takes place in an inert solvent such as dichloromethane in the presence of a diimide reagent such as 1-ethyl-3-(3-dimethylaminopropyl) carbodiimide hydrochloride, and an activator such as 1-hydroxybenzotriazole. Also when $L_1$ represents hydroxy the reaction can be effected using O-(7-azabenzotrazol-1-yl)-N,N,N',N'-tetramethyluronium hexafluorophosphate (HATU) with a base such as triethylamine or N,N-diisopropylethylamine.

**Scheme 4**

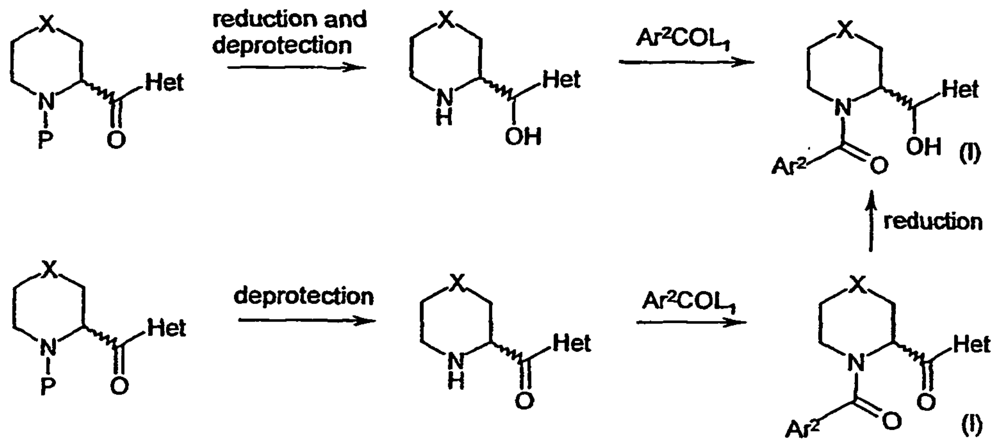


Wherein Het, X and $Ar^2$ are as defined for formula I and P is a protecting group. Suitable reducing agents include sodium borohydride which can be used at ambient temperature in methanol.

**Scheme 5**

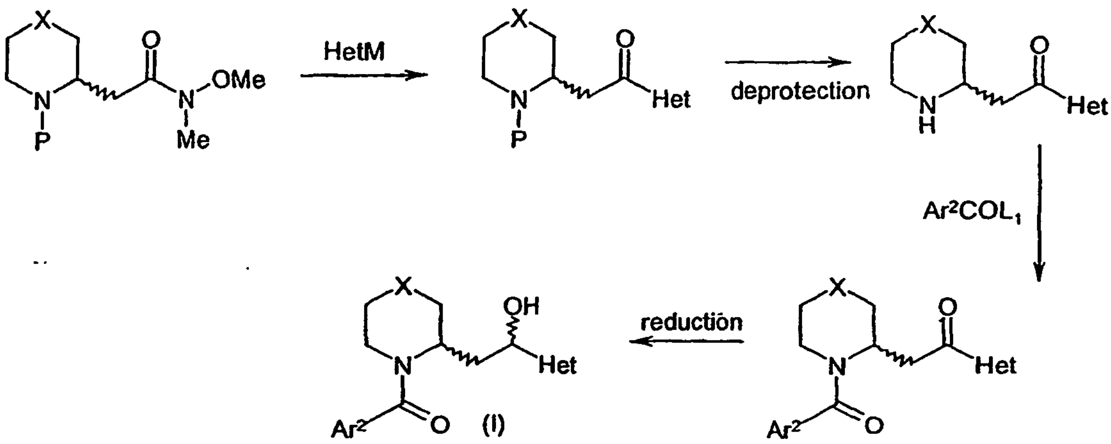


Wherein Het, X and $Ar^2$ are as defined for formula I, P is a protecting group and M is a metal for example lithium.

Within the schemes above there is scope for functional group interconversion; conversion of one compound of formula (I) to another of formula (I) by interconversion of substituents.

The compounds of formula (I) may be prepared singly or as compound libraries comprising at least 2, e.g. 5 to 1000, preferably 10 to 100 compounds of formula (I). Compound libraries may be prepared by a combinatorial 'split and mix' approach or by multiple parallel synthesis using either solution phase or solid phase chemistry, by procedures

known to those skilled in the art.

Thus according to a further aspect of the invention there is provided a compound library comprising at least 2 compounds of formula (I), or pharmaceutically acceptable derivatives thereof.

Pharmaceutically acceptable salts may be prepared conventionally by reaction with the appropriate acid or acid derivative.

The compounds of formula (I) and their pharmaceutically acceptable derivatives are useful for the manufacture of a medicament for the treatment of diseases or disorders where an antagonist of a human orexin receptor is required such as obesity and diabetes; prolactinoma; hypoprolactinemia; hypothalamic disorders of growth hormone deficiency, idiopathic growth hormone deficiency; Cushings syndrome/disease; hypothalamic-adrenal dysfunction; dwarfism; sleep disorders; sleep apnea; narcolepsy; insomnia; parasomnia; jet-lag syndrome; sleep disturbances associated with diseases such as neurological disorders, neuropathic pain and restless leg syndrome; heart and lung diseases; depression; anxiety; addictions; obsessive compulsive disorder; affective neurosis/disorder; depressive neurosis/disorder, anxiety neurosis; dysthymic disorder; behaviour disorder; mood disorder, sexual dysfunction; psychosexual dysfunction; sex disorder, sexual disorder, schizophrenia; manic depression; delerium; dementia; bulimia and hypopituitarism. The compounds of formula (I) or pharmaceutically acceptable derivatives thereof are also useful in the treatment of stroke, particular ischaemic or haemorrhagic stroke. Furthermore the compounds of formula (I) or pharmaceutically acceptable derivatives useful in the blocking an emetic response.

The compounds of formula (I) and their pharmaceutically acceptable derivatives are particularly useful for the manufacture of a medicament for the treatment of obesity, including obesity associated with Type 2 diabetes, and sleep disorders. Additionally the compounds are useful in stroke and/or blocking the emetic response i.e. nausea and vomiting.

Other diseases or disorders for which the compounds a formula I may be used for the manufacture of a medicament include disturbed biological and circadian rhythms; adrenohypophysis disease; hypophysis disease; hypophysis tumor / adenoma; adrenohypophysis hypofunction; functional or psychogenic amenorrhea, adrenohypophysis hyperfunction; migraine; hyperalgesia; pain; enhanced or exaggerated sensitivity to pain such as hyperalgesia, causalgia and allodynia; acute pain; burn pain; atypical facial pain; neuropathic pain; back pain; complex regional pain syndromes I and II; arthritic pain; sports injury pain; pain related to infection e.g. HIV, post-polio syndrome and post-herpetic neuralgia, phantom/limb pain; labour pain; cancer pain; post-chemotherapy pain; post-stroke pain; post-operative pain, neuralgia; and tolerance to narcotics or withdrawal from narcotics.

The invention also provides the use of a compound of formula (I), or a pharmaceutically acceptable derivative thereof, in the manufacture of a medicament for the treatment or prevention of obesity, diabetes, sleep disorders, insommia, parasommia or stroke.

For use in therapy the compounds of the invention are usually administered as a pharmaceutical composition. The invention also provides a pharmaceutical composition comprising a compound of formula (I), or a pharmaceutically acceptable derivative thereof, and a pharmaceutically acceptable carrier.

The compounds of formula (I) and their pharmaceutically acceptable derivatives may be administered by any convenient method, e.g. by oral, parenteral, buccal, sublingual, nasal, rectal or transdermal administration, and the pharmaceutical compositions adapted accordingly.

The compounds of formula (I) and their pharmaceutically acceptable derivatives which are active when given orally can be formulated as liquids or solids, e.g. as syrups, suspensions, emulsions, tablets, capsules or lozenges.

A liquid formulation will generally consist of a suspension or solution of the active ingredient in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluorochlorohydrocarbon or hydrofluorocarbon.

Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

Preferably the composition is in unit dose form such as a tablet, capsule or ampoule.

The dose of the compound of formula (I), or a pharmaceutically acceptable derivative thereof, used in the treatment or prophylaxis of the abovementioned disorders or diseases will vary in the usual way with the particular disorder or disease being treated, the weight of the subject and other similar factors. However, as a general rule, suitable unit doses may be 0.05 to 1000 mg, more suitably 0.05 to 500 mg. Unit doses may be administered more than once a day for example two or three times a day, so that the total daily dosage is in the range of about 0.01 to 100 mg/kg, and such therapy may extend for a number of weeks or months. In the case of pharmaceutically acceptable derivatives the above figures are calculated as the parent compound of formula (I)

No toxicological effects are indicated/expected when a compound of formula (I) is administered in the above mentioned dosage range.

Human orexin-A has the amino acid sequence:

pyroGlu Pro Leu Pro Asp Cys Cys Arg Gln Lys Thr Cys Ser Cys Arg Leu
1 5 10 15
Tyr Glu Leu Leu His Gly Ala Gly Asn His Ala Ala Gly Ile Leu Thr
20 25 30
Leu-$NH_2$

Orexin-A can be employed in screening procedures for compounds which inhibit the ligand's activation of the orexin-1 receptor.

In general, such screening procedures involve providing appropriate cells which express the orexin-1 receptor on their surface. Such cells include cells from mammals, yeast, Drosophila or *E. coli.* In particular, a polynucleotide encoding the orexin-1 receptor is used to transfect cells to express the receptor. The expressed receptor is then contacted with a test compound and an orexin-1 receptor ligand to observe inhibition of a functional response. One such screening procedure involves the use of melanophores which are transfected to express the orexin-1 receptor, as described in WO 92/01810.

Another screening procedure involves introducing RNA encoding the orexin-1 receptor into *Xenopus* oocytes to transiently express the receptor. The receptor oocytes are then contacted with a receptor ligand and a test compound, followed by detection of inhibition of a signal in the case of screening for compounds which are thought to inhibit activation of the receptor by the ligand.

Another method involves screening for compounds which inhibit activation of the receptor by determining inhibition of binding of a labelled orexin-1 receptor ligand to cells which have the receptor on their surface. This method involves transfecting a eukaryotic cell with DNA encoding the orexin-1 receptor such that the cell expresses the receptor on its surface and contacting the cell or cell membrane preparation with a compound in the presence of a labelled form of an orexin-1 receptor ligand. The ligand may contain a radioactive label. The amount of labelled ligand bound to the receptors is measured, e.g. by measuring radioactivity.

Yet another screening technique involves the use of FLIPR equipment for high throughput screening of test compounds that inhibit mobilisation of intracellular calcium ions, or other ions, by affecting the interaction of an orexin-1 receptor ligand with the orexin-1 receptor.

The following Examples illustrate the preparation of pharmacologically active compounds of the invention. The Descriptions D1-D 59 illustrate the preparation of intermediates to compounds of the invention.

In the Examples $^1H$ NMR's were measured at 250MHz in $CDCl_3$ unless otherwise stated.

The following abbreviations are used herein;

PyBop means benzotriazol-1-yloxytris(pyrrolidino)phosphonium hexafluorophosphate THF means tetrahyrdofuran EDC.HCL means 1-(3-dimethylaminopropyl)-3-ethylcarbodiimide hydrochloride

HATU means O-(7-azabenzotriazol-1-yl)-N,N,N',N'-tetramethyluronium hexafluorophosphate TFA means trifluoroacetatic acid

DMF means N,N-dimethylformamide

DME means 1,2-dimethoxyethane

**Description 1: (RS)-1-(*tert*-butyloxycarbonyl)-2-(N-metholy-N-methylcarbamoyl)-piperidine**

To (RS)-1-(*tert*-butyloxycarbonyl) 2-piperidine carboxylic acid (2.64 g, 11.5 mmol) in dichloromethane (10 ml) was added sequentially N,O-dimethyl hydroxylamine (1.34 g, 13.7 mmol), triethylamine (6 ml, 43.0 mmol) and Py.Bop (6.60 g, 12.7 mmol). The resultant mixture was stirred at ambient temperature for 6 h, then diluted with dichloromethane (170 ml) and poured into 1M HCl (22 ml). The organic phase was separated and washed with saturated aqueous sodium hydrogen carbonate (3 x 25 ml) and brine (25 ml) then evaporated *in vacuo.* The resultant colourless oil was chromatographed on silica gel eluting with 20 % ethyl acetate in hexane to give the title compound (2.43 g, 77 %) as a colourless oil.

Mass spectrum (API$^+$): Found 273 (MH$^+$). $C_{13}H_{24}N_2O_4$ requires 272.

**Description 2: (RS)-2-(2-Benzofuranylcarbonyl)-1-(*tert*-butyloxycarbonyl)piperidine**

To a solution of benzofuran (0.37 ml, 336 mmol) in THF (40 ml) at -35°C was added *n*butyllithium (1M in THF) (3.34 ml, 3.34 mmol) over 3 min. The resultant mixture was stirred for 10 min. at -35°C then (RS)-1-(*tert*-butyloxycarbonyl)-2-(N-methoxy-N-methylcarbamoyl)-piperidine (1.0 g, 3.68 mmol) in THF (5 ml) was added over 1 min. and the resultant solution stirred for 15 min. at -35°C. The mixture was poured into saturated ammonium chloride (20 ml) and extracted with ethyl acetate (3 x 20 ml). The combined organics were washed with saturated aqueous sodium hydrogen carbonate (15 ml), then evaporated *in vacuo.* The resultant residue was chromatographed on silica gel eluting with 10 % ethyl acetate in hexane to give the title compound (0.32 g, 26 %) as a yellow solid.

Mass spectrum (API$^+$): Found 330 (MH$^+$) $C_{19}H_{23}NO_4$ requires 329.

**Description 3: (RS)-2-(2-Benzofuranylmethyl)piperidine**

To a solution of (RS)-2-(2-benzofuranylcarbonyl)-1-(*tert*-butyloxycarbonyl)piperidine (0.30 g, 0.91 mmol) in diethylene glycol (20 g), was added hydrazine hydrate (0.16 ml, 2.79 mmol) and the resultant mixture heated at 170°C for 30 min. then cooled to room temperature. Potassium hydroxide (0.44 g, 7.86 mmol) was added and the mixture heated at 200°C for 18 h. The resultant was then poured into water (100 ml) and extracted with diethyl ether (3 x 20 ml). The combined organic extracts were dried ($Na_2SO_4$) and evaporated *in vacuo* to give the title compound (10 mg, 36 %) as a golden oil.

Mass spectrum (API$^+$). Found 216 (MH$^+$) $C_{14}H_{17}NO$ requires 215.

**Description 4: (RS)-2-[(2-Hydroxy-phenylcarbamoyl)-methyl]-piperidino-1-carboxylic acid *tert* butyl ester**

A mixture of 2-carboxymethyl-piperidine-1-carboxylic acid tert butyl ester (0.97g), (*Peschke, Bernd; Ankersen, Michael; Hansen, Birgit Sehested; Hansen, Thomas Kruse; Johansen, Nils Langeland; Lau, Jesper; Madsen, Kjeld; Petersen, Hans; Thogersen, Henning; Watson, Brett.*

*Eur. J. Med. Chem. (1999), 34(5), 363-380)* in dimethylformamide (8.0ml) was treated sequentially with EDC.HCl (0.76g), 2-aminophenol (0.43g) and 1 hydroxybenzotriazole (0.05g). The mixture was stirred for 2h, diluted with ethyl acetate and washed with water, dried ($MgSO_4$) and solvent removed at reduced pressure to give the title compound (1.29g) as a gum.

Mass Spectrum (API$^+$): Found 335 (MH$^+$). $C_{18}H_{26}N_2O_4$ requires 334

**Description 5 (RS)-2-Benzooxazol-2-ylmethyl-piperidine-1-carboxylic acid tert butyl ester**

(RS)-2-[(2-Hydroxy-phenylcarbamoyl)-methyl]-piperidine-1-carboxylic acid *tert* butyl ester (1.25g), 4-toluenesulphonic acid (0.07g) and 4-N,N-dimethylaminopyridine (0.023g) were combined in 1, 3-dichlorobenzene (30ml) and the mixture boiled for 48h. The mixture was diluted with dichloromethane and washed with water. The organic phase was dried ($MgSO_4$) and solvent removed at reduced pressure. The residue was column chromatographed (silica gel, 0 $\rightarrow$ 1% methanol in dichloromethane) to give the title compound (0.50g)

Mass Spectrum (API$^+$): Found 317 (MH$^+$). $C_{18}H_{24}N_2O_3$ requires 316.

**Description 6: (RS)-2-Piperidin-2-ylmethyl-benzooxazole**

(RS)-2-Benzooxazol-2-ylmethyl-piperidine-1-carboxylic acid tert butyl ester (0.50g) in dichloromethane (8ml) was cooled (ice-bath) and trifluoroacetic acid (2ml) added. The mixture was stirred with ice-cooling for 4h, poured onto saturated potassium carbonate (20ml) and extracted with dichloromethane. The combined extracts were dried ($MgSO_4$) and solvent removed at reduced pressure to give the title compound (0.10g)

Mass Spectrum (API$^+$): Found 217 (MH$^+$). $C_{13}H_{16}N_2O$ requires 216.

**Description 7: (RS)-2-[1-(5-Fluoro-benzofuran-2-yl)methanoyl)-piperidine-1-carboxylic acid *tert*-butyl ester**

The title compound was prepared using the method of Description 2, from 5-fluorobenzofuran (1.36g, 10mmol, for preparation see WO99/51575), as a pale yellow solid (2.81g, 84%)

$^1$H NMR δ: 120-1.60 (11H, m), 1.60 - 1.85 (2H, m), 1.90 (1H, broad m), 2.15 - 2.30 (1H, broad m), 3.20 - 3.40 (1H, broad m), 3.85 - 4.05 (1H, broad m), 5.20 - 5.70 (1H, broad m), 7.10 - 7.25 (1H, m), 7.25 - 7.4 (1H, d, J = 7.4 Hz), 7.45 - 7.75 (2H, m).

**Description 8: (RS)-2-(5-Fluoro-benzofuran-2-ylmethyl)-pipe$_l$idine**

The title compound was prepared using the method of Description 3, from (RS)-2-[1-(5-Fluoro-benzofuran-2-yl)methanoyl)-piperidine-1-carboxylic acid *tert*-butyl ester, D7 (1.21g, 3.49 mmol), as a pale yellow gum (0.72g, 89%).

Mass spectrum (API$^+$): Found 234 (MH$^+$) $C_{14}H_{16}FNO$ requires 233.

**Description 9: (RS)-1-Benzofuran-2-yl-1-piperidin-2-yl-methanone**

A solution of (RS)-2-(2-benzofuranylcarbonyl)-1-(*tert*-butyloxycarbonyl)piperidine, D2 (0.86g, 2.61 mmol) in trifluoroacetic acid (5ml) and dichloromethane (20ml) was warmed at 35°C for 0.5h. The reaction mixture was evaporated *in vacuo* and the residue partitioned between dichloromethane (30ml) and 1N sodium hydroxide (30ml). The aqueous layer was extracted with dichloromethane (30ml) and the combined organic layers dried ($Na_2SO_4$) and evaporated *in vacuo* to afford the title compound as a pale yellow gum (0.59g, 99%).

Mass spectrum (API$^+$): Found 230 (MH$^+$). $C_{14}H_{15}NO_2$ requires 229.

**Description 10: (RS)-2-(1-Furo[2,3-*b*]pyridio-2-yl)methanoyl-piperidine-1-carboxylic acid *tert*-butyl ester**

2.5N n-Butylithium in hexanes (2.3ml, 5.75mmol) was added dropwise over 10 min to a stirred solution of furo [2,3-*b*]pyridine (0.57g, 4.79mmol) (H. Sliwa, Bull.Soc.Chim. France, 646, 1970) in anhydrous THF (20ml) at -75°C under argon. The resultant mixture was stirred for 5 min at - 70°C then a solution of (RS)-1-(*tert*-butyloxycarbonyl)-2-(N-methoxy-N-methylcarbamoyl)-piperidine, D1 (1.41g, 5.19mmol) in anhydrous THF (20ml) was added over 2 min. and the resultant solution stirred at -70°C for 15 min. The mixture was poured into saturated ammonium chloride (100ml) and extracted with ethyl acetate (2X50ml). The combined organics were dried ($Na_2SO_4$) and evaporated *in vacuo.* The residue was chromatographed on silica gel eluting with 0-1% ethyl acetate in dichloromethane to give the title compound as a colourless solid (0.51g, 32%).

$^1$H NMR δ: 1.25-1.55 (11H, m), 1.60 - 1.80 (2H, m), 1.85 - 2.05 (1H, broad m), 2.15 - 2.40 (1H, broad m), 3.15 - 3.40 (1H, broad m), 3.80 - 4.15 (1H, broad m), 5.35 - 5.65 (1H, broad m), 7.30 (1H, broad m), 7.56 (1H, broad s), 8.08 (1H, broad m), 8.53 (1H, broad m).

**Description 11: (RS)-2-Piperidin-2-ylmethyl-furo[2,3-*b*]pyridine**

The title compound was prepared using the method of Description 3, from (RS)2-(1-furo[2,3-*b*]pyridin-2-yl) methanoyl)piperidine-1-carboxylic acid *tert*-butyl ester, D10 (0.51g, 1.55mmol), as a light brown gum (0.26g, 74%).

Mass spectrum (API$^+$): Found 217 (MH$^+$) $C_{13}H_{16}N_2O$ requires 216.

**Description 12: (RS)-2-(1-Quinolin-2-yl-methanoyl)-piperidine-1-carboxylic acid t*ert*-butyl ester**

30% Potassium hydride dispersed in mineral oil (0.8g, 6mmol) was added to a stirred solution of 2-bromoquinoline (1.12g, 5.29mmol) (O. Sugimoto, **Tet. Lett**. 4, (1999), 7477-8) in anhydrous THF (20ml) at -70°C under argon. To this was added 2N n-butyllithium in hexanes (2.7ml, 6.75 mmol) dropwise over 5 min. The resultant mixture was stirred for 10 min at -70°C then a solution of (RS)-1-(*tert*-butyloxycarbonyl)-2-(N-methoxy-N-methylcarbamoyl)-piperidine, D1 (1.57g, 5.77mmol) in anhydrous THF (10ml) was added over 2 min. The cooling bath was removed and the reaction mixture allowed to warm to -20°C and held at -20°C for 10 min. The mixture was poured into saturated ammonium chloride (300ml) and extracted with ethyl actetate (2 x 100ml). The combined organics were dried ($Na_2SO_4$) and evaporated *in vacuo.* The residue was chromatographed on silica gel eluting with 0-10% ethyl acetate in pentane to yield the title compound as a pale yellow solid (1.2g, 66%).

$^1$H NMR δ: 1.2 -1.65 (12H, m), 1.65-1.85 (1H, m), 1.90-2.10 (1H, m), 2.20-2.50 (1H, m), 330-3.60 (1H, m), 3.85-4.20 (1H, m), 6.20-6.40 (1H, m), 7.50-7.90 (3H, m), 8.07 (1H, d, J = 8.4Hz), 8.18 (1H, d, J = 8.8Hz), 8.20-8.35 (1H, m).

**Description 13: (RS)-2-Piperidin-2-ylmethyl-quinoline**

The title compound was prepared using the method of Description 3, from (RS)-2-(1-quinolin-2-yl-methanoyl)-piperidine-1-carboxylic acid *tert*-butyl ester, D12 (1.2g, 3.66mmol), as a pale brown gum (0.053g, 6%)

Mass spectrum (Electrospray LC/MS): Found 227 ($MH^+$). $C_{15}H_{18}N_2$ requires 226.

**Description 14: 2-Amino-*N,N*-dimethyl-3-nitro-benzamide**

EDC.HCl (2.47 g, 12.88mmol) was added to a stirred mixture of 2-amino-3-nitrobenzoic acid (2.18g, 11.98mmol) (Mickelson, **J. Med. Chem.** 39(23) 4662 (1996)), 2M dimethylamine in THF (6.45ml, 12.9mmol) and 1-hydroxybenzotriazole (0.1g, 0.74 mmol) in dichloromethane (100 ml). The reaction mixture was stirred at room temperature for 18h, washed with saturated aqueous sodium bicarbonate and the organic layer dried ($Na_2SO_4$) and evaporated *in vacuo* to afford the title compound as a pale orange gum (2.5g, 100%).

$^1$HNMR δ: 3.08 (6H, s), 6.70 (1H, m), 6.85 (2H, broad s), 7.33 (1H, dd, J = 2 and 8Hz), 8.2 (1H, dd, J = 2 and 8Hz).

**Description 15: 3-Dimethylaminomethyl-benzene-1,2-diamine**

1M Lithium aluminium hydride in THF (18.1ml, 18.1 mmol) was added dropwise over 5 min. to a stirred, ice cooled solution of 2-Amino-*N*,*N*-dimethyl-3-nitro-benzamide, D14 (1.26g, 6.03mmol) in anhydrous THF (40ml) under argon. The reaction mixture was stirred at 0°C for 0.5h then at room temperature for a further 2h before being recooled in ice, as water (3.2ml), 2N sodium hydroxide (3.6ml) and water (3.2ml) were added dropwise sequentially. The mixture was stirred for 10 min, solid $Na_2SO_4$ added and stirring continued for 10 min. Solids were filtered, washed with ethyl acetate and the combined organics evaporated *in vacuo* to give the title compound as an orange solid (0.9g, 91%).

$^1$H NMR δ: 2.19 (6H, s), 3.29 (2H, broad s), 3.42 (2H, s), 4.50 (2H, broad s), 650-6.80 (3H, m).

**Description 16: (RS)-(1-{1-[5-(4-Fluoro-phenyl)-2-methyl-thiazol-4-yl]-methanoyl}-piperidin-2yl)-acetic acid methyl ester**

A stirring solution of 5-(4-fluoro-phenyl)-2-methyl-thiazole-4-carboxylic acid (3.00g, 12.5mmol) in DMF (50ml), under argon was treated sequentially with *N,N*-diisopropylethylamine (6.8ml), HATU (4.77g, 12.5mmol) then after stirring for 0.25h, piperidin-2-yl-acetic acid methyl ester (Beckett et al, J.Med.Chem., 563,1969) (1.83g, 12.5mmol) was added. The mixture was stirred for 16h at room temperature and was then diluted with water.

The product was extracted into diethyl ether. The organic phase was washed with water (3X) and brine, was dried ($MgSO_4$) and the solvent removed *in vacuo* to afford the title compound as a yellow gum (4.00g, 85%).

Mass spectrum (API$^+$). Found 377 ($MH^+$). $C_{19}H_{21}FN_2O_3S$ requires 376.

**Description 17: (RS)-(1-{1-[5-(4-Fluoro-phenyl)-2-methyl-thiazol-4-yl]-methanoyl}-piperidin-2-yl)-acetic acid**

A stirring solution of (1-{1-[5-(4-Fluoro-phenyl)-2-methyl-thiazol-4-yl]-methanoyl}-piperidin-2yl)-acetic acid methyl ester, D16 (4.00g, 10.6mmol) in methanol/water (75ml/25ml) was treated with sodium hydroxide (0.85g, 21.3mmol). The mixture was stirred for 16h at room temperature. The methanol was removed *in vacuo* and the residue was diluted with water and extracted with diethyl ether (2X). The aqueous phase was acidified with 5N HCl and the product was extracted with ethyl acetate. The organic phase was dried ($MgSO_4$) and the solvent removed *in vacuo.* The residue was triturated with pentane to afford the title compound as a white solid (3.10g, 82%).

$^1$H NMR ($D_6$-DMSO) δ: 0.98 (0.6H, m), 1.15-1.70 (5.4H, bm), 2.18 (0.4H, dd), 2.38 (0.6H, dd, J = 6 and 15Hz), 2.70 (4.4H, m), 2.93 (0.6H, m), 3.17 (0.6H, d, J = 12Hz), 3.94 (0.4H, m), 4.40 (0.4H, d, J = 12Hz), 5.05 (0.6H, m), 7.27 (2H, m), 7.47 (2H, m), 12.20 (1H, bs).

**Description 18: (RS)-5,6-Difluoro-2-piperidin-2-ylmethyl-1*H*-benzoimidazole**

A mixture of 2-carboxymethyl-piperidine-1-carboxylic acid *tert*-butyl ester (Beckett et al, J.Med.Chem., 563,1969) (10.0g, 41 mmol) and 4,5-difluoro-benzene-1,2-diamine (5.95g, 41mmol) in polyphosphoric acid (140g) was heated at 140°C for 7h. The cooled reaction mixture was poured onto excess solid potassium carbonate and crushed ice. The resulting basic, aqueous solution was extracted with ethyl acetate (2X) and the combined organics were washed with brine, dried ($Na_2SO_4$) and the solvent removed *in vacuo.* The residue was triturated with diethyl ether, ethyl acetate and pentane to afford the title compound as a light brown solid (7.14g, 69%). $^1$H NMR ($D_6$-DMSO) δ: 1.08 (1H, m), 1.27 (2H, m), 1.50 (2H, m), 1.70 (1H, m), 2.48 (1H, m, obscured by DMSO), 2.78 (2H, d, J = 7Hz), 2.88 (2H, m), 7.51 (2H, app.t, J = 6Hz).

**Description 19: (RS)-2-(5,6-Difluoro-1*H*-benzoimidazol-2-ylmethyl)-piperidine-1-carboxylic acid *tert*-butyl ester**

4,5-Difluoro-benzene-1,2-diamine (1.00g, 6.9mmol) and 2-carboxymethyl-piperidine-1-carboxylic acid *tert*-butyl ester (Beckett et al, J.Med.Chem., 563,1969) (1.69g, 6.9mmol) were heated at 120°C with stirring, under argon for 4h. A further portion of 2-carboxymethyl-piperidine-1-carboxylic acid *tert*-butyl ester (0.85g) was added and heating was continued for a further 2h. The cooled reaction mixture was diluted with ethyl acetate, washed with saturated aqueous sodium bicarbonate (2X) and brine, dried ($MgSO_4$) and the solvent removed *in vacuo.* The residue was chromatographed (silica gel, 0-30% ethyl acetate-pentane) to give a yellow solid which was triturated with diethyl ether to afford the title compound as a pale brown solid (0.38g, 15%).

Mass spectrum ($API^+$). Found 352 ($MH^+$) $C_{18}H_{23}F_2N_3O_2$ requires 351.

**Description 20: (RS)-5,6-Difluoro-2-piperidin-2-ylmethyl-1*H*-benzoimidazole. 2HCl**

A stirring solution of 2-(5,6-Difluoro-1*H*-benzoimidazol-2-ylmethyl)-piperidine-1-carboxylic acid *tert*-butyl ester, D19 (375mg, 1.1mmol) in MeOH (20ml) was treated with 1M HCl in 1,4-dioxane (5ml). After stirring under argon at room temperature for 5h the volatiles were removed *in vacuo* to afford the title compound as a gum (100%).

Mass spectrum ($API^+$). Found 252 ($MH^+$) $C_{13}H_{15}F_2N_3$ requires 251.

**Description 21: (RS)-4,5-Difluoro-2-piperidin-2-ylmethyl-1*H*-benzoimidazole**

A mixture of 2-carboxymethyl-piperidine-1-carboxylic acid *tert*-butyl ester (Beckett et al, J.Med.Chem., 563,1969) (6.72g, 28mmol) and 3,4-difluoro-benzene-1,2-diamine (4.00g, 28mmol) in polyphosphoric acid (130g) was heated at 140°C for 7.5h. The cooled reaction mixture was poured onto excess solid potassium carbonate and crushed ice. The resulting basic, aqueous solution was extracted with ethyl acetate (2X) and the combined organics were washed with brine, dried ($Na_2SO_4$) and the solvent removed *in vacuo.* The residue was triturated with diethyl ether, ethyl acetate and pentane to afford the title compound as a white solid (4.29g, 62%).

$^1H$ NMR ($D_6$-DMSO) δ: 1.08 (1H, m), 1.28 (2H, m), 1.50 (2H, m), 1.72 (1H, m), 2.46 (1H, m, obscured by DMSO), 2.81 (2H, d, J = 7Hz), 2.90 (2H, m), 7.14 (1H,m), 7.24 (1H, m).

**Description 22: 1-(2-Dimethylamino-ethyl)-4-(4-fluoro-phenyl)-1*H*-pyrazole-3-carboxylic acid ethyl ester**

4-(4 Fluoro-phenyl)-1*H*-pyrazole-3-carboxylic acid ethyl ester (2.4g, 10mmol) was added to an ice-cooled slurry of sodium hydride (0.82g, 60% dispersion in mineral oil, 20mmol) in DMF (20ml), under argon. *N,N*-diisopropylethylamine (1.8ml, 10mmol) was added followed by 2-chloro-ethyl dimethylamine.HCl (1.48g, 10mmol) and potassium iodide (5mg). The reaction mixture was stirred at room temperature for 16h then at 80°C for 48h. 2N HCl and water were added and the product extracted with ethyl acetate (3X). The combined organics were washed with brine, dried ($Na_2SO_4$) and the solvent removed *in vacuo.* Chromatography (silica gel, 3-10% methanol-dichloromethane) afforded 2-(2-dimethylamino-ethyl)-4-(4-fluoro-phenyl)-1*H*-pyrazole-3-carboxylic acid ethyl ester (900mg) and the title compound (630mg).

$^1H$ NMR δ: 1.31 (3H, t. J = 7Hz), 2.29 (6H, s), 2.80 (2H, t, J = 6Hz), 4.30 (4H, m), 7.05 (2H, m), 7.44 (2H, m), 7.56 (1H, s).

**Description 23:1-(2-Dimethylamino-ethyl)-4-(4-fluoro-phenyl)-1*H*-pyrazole-3-carboxylic acid**

A solution of 1-(2-Dimethylamino-ethyl)-4-(4-fluoro-phenyl)-1*H*-pyrazole-3-carboxylic acid ethyl ester, D22 (610mg, 2mmol) in methanol/water (15ml/10ml) was treated with 2N sodium hydroxide (4ml). After stirring at room temperature for 16h the methanol was removed *in vacuo* and the residue treated with 2N HCl (4ml). The water was removed *in vacuo.* Dichloromethane was added to the residue and the inorganic solid was removed by filtration. Removal of the solvent form the filtrate *in vacuo* afforded the title compound as a white solid (350mg, 63%). Mass spectrum ($API^+$). Found 278 ($MH^+$) $C_{14}H_{16}FN_3O_2$ requires 277.

**Description 24: 5-(4-Fluoro-phenyl)-2-methyl-2*H*-[1,2,3]triazole-4-carboxylic acid methyl ester**

5-(4-Fluoro-phenyl)-2*H*-[1,2,3]triazole-4-carboxylic acid methyl ester (2.21g, 10mmol) in THF (10ml) was added drop-wise to an ice-cooled slurry of sodium hydride (0.42g, 60% dispersion in mineral oil, 10mmol) in THF (45ml), under argon. After stirring at room temperature for 1h, the reaction mixture was heated at 80°C for 16h. Water was added to the cooled mixture and the product was extracted with diethyl ether (2X). The combined organics were washed with brine, dried ($Na_2SO_4$) and the solvent removed *in vacuo.* Chromatography (silica gel, 10-50% ethyl acetate-pentane) afforded 5-(4-Fluoro-phenyl)-1-methyl-2*H*-[1,2,3]triazole-4-carboxylic acid methyl ester (763mg, 33%) and the title com-

pound (943mg, 40%).

$^1H$ NMR δ: 3.94 (3H, s), 4.29 (3H, s), 7.13 (2H, m), 7.87 (2H, m).

**Description 25: 5-(4-Fluoro-phenyl)-2-methyl-2*H*-[1,2,3]triazole-4-carboxylic acid**

5-(4-Fluoro-phenyl)-2-methyl-2*H*-[1,2,3]triazole-4-carboxylic acid methyl ester D24 (940mg, 4mmol) in water (65ml) was treated with 2N sodium hydroxide (4ml) and the mixture was heated at 120°C for 2h. The volume of water was reduced to 20ml *in vacuo* and the ice-cooled residue was treated with 2N HCl (4ml). Filtration, washing with water and drying *in vacuo* afforded the title compound as a white solid (810mg, 91%).

Mass spectrum (API$^+$). Found 222 (MH$^+$) $C_{10}H_8FN_3O_2$ requires 221.

**Description 26: (RS)-[1-(2,2,2-Trifluoroethanoyl)-piperidin-2-yl] acetic acid ethyl ester**

(RS)-Piperidin-2-yl acetic acid ethyl ester (Rhodes et al, J.Am.Pharm.Assoc.,1956,45,746) (6.0g) was dissolved in dry dichloromethane (60 ml) and cooled to -10 °C under an atmophere of argon. Triethylamine (5.0ml), followed by trifluoroacetic anhydride (5.1ml) was added to the stirred solution and stirring was continued at room temperature for 16h. The reaction solution was then partitioned between dichloromethane and saturated sodium bicarbonate solution. The organic solution was dried ($MgSO_4$) and the solvent removed *in vacuo* to yield the title compound as an oil (4.2 g).

Mass Spectrum (API$^+$): Found 268 (MH$^+$). $C_{11}H_{16}F_3NO_3$ requires 267.

**Description 27:(R.S)-1-(2-Benzothiazol-2-ylmethylpiperidin-1-yl)-2,2,2-triflaoro-ethanone**

[1-(2,2,2-Trifluoroethanoyl)piperidin-2-yl] acetic acid ethyl ester, D26 (1.34g) and 2-aminothiophenol (1.50g) were suspended in polyphosphoric acid (30ml) and heated to 90°C for 2h with vigorous stirring. After cooling, the reaction mixture was poured into iced water and stirred vigorously for 1h. The aqueous solution was then extracted several times with ethyl acetate. After drying ($MgSO_4$), the solvent was removed *in vacuo* and the residue chromatographed (silica gel) to afford the title compound as an oil (1.2g).

Mass Spectrum (API$^+$): Found 329 (MH$^+$). $C_{15}H_{15}F_3N_2SO$ requires 328.

**Description 28:(RS)-2-Piperidin-2-ylmethylbenzothiazole**

1-(2-Benzothiazol-2-ylmethylpiperidin-1-yl)-2,2,2-trifluoroethanone, D29 (1.2g) was dissolved in methanol (40ml) and 2N sodium hydroxide solution (20ml) and heated to 50 °C for 0.75h. After cooling, the reaction solution was partitioned between dichloromethane and water. The organic solution was dried ($MgSO_4$) and the solvent removed *in vacuo.* The residue was chromatographed (silica gel, 0-10% [9:1 MeOH/conc. ammonia solution] in dichloromethane) to afford the title compound (0.68 g).

Mass Spectrum (API$^+$): Found 233 (MH$^+$). $C_{13}H_{16}N_2S$ requires 232.

**Description 29: (RS)- 1-Benzyl-2-bromomethylpiperidine**

(RS)-(1-Benzyl-piperidin-2-yl)-methanol (R.Sreekumar et al, Tetrahedron Lett., 1998, 39 5151) (10.0g) was converted to the title compound (5.2g) by treatment with triphenylphosphine (13.0g) and N-bromosuccinimide (9.4g) in dichloromethane using literature procedures, (Tetrahedron Lett" 1999, 40, 7477-8).

Mass Spectrum (API$^+$): Found 270 (MH$^+$). $C_{13}H_{18}{}^{81}BrN$ requires 269.

**Description 30: (RS)-1-Benzyl-2-cyanomethylpiperidine**

(RS)-1-Benzyl-2-bromomethylpiperidine, D29 (4.2g) was dissolved in dry dimethylsulphoxide (15ml) and treated with sodium cyanide (5.0g). The solution was then stirred at 85°C under argon for 18h. The reaction solution was then poured into water (300ml) and extracted with dichloromethane (2 x 200ml). The organic solution was dried ($MgSO_4$) and the solvent removed *in vacuo.* Chromatography (silica gel, diethyl ether/petroleum ether mixtures) afforded the title compound (3.3g).

Mass Spectrum (API$^+$): Found 215 (MH$^+$). $C_{14}H_{18}N_2$ requires 214.

**Description 31: (RS)- 2-(1-Benzylpiperidin-2-yimethyl)-[1,2,4]-triazolo[1,5-a]pyridine**

(RS)-1-Benzyl-2-cyanomethylpiperidine, D30 (1.0g) was dissolved in dry tetrahydrofuran (10ml) containing ethanol (0.28ml). The reaction solution was then cooled to ice bath temperature and hydrogen chloride gas was bubbled

through the solution for 1h. The solution was then stirred at room temperature for 16h. The resulting solution was diluted with tetrahydrofuran (30ml) and dichloromethane (30ml) and 1-aminopyridinium iodide (1.0g), followed by excess potassium carbonate and ethanol (30ml) was added. The reaction mixture was then stirred at room temperature under argon for 24h. The mixture was then filtered and the solvent removed *in vacuo.* The residue was chromatographed (silica gel, 80-20 diethyl ether-petroleum ether then 9-1 dichloromethane-methanol) to afford the title compound.

Mass Spectrum (API$^+$): Found 307 (MH$^+$). $C_{14}H_{18}N_2$ requires 306.

**Description 32: (RS)-2-Piperidin-2-ylmethyl-5,6,7,8-tetrahydro-[1,2,4]-triazolo[1,5-alpyridine and 2-Piperidin-2-ylmethyl-[1,2,4]-triazolol [1,5-a]pyridine**

(RS)-2-(1-Benzylpiperidin-2-ylmethyl)-[1,2,4]-triazolo[1,5-a]pyridine, D31 (0.200g) was dissolved in ethanol (25ml). Palladium hydroxide (0.100g), followed by platinum IV sulphide (0.020g) were added and the reaction mixture hydrogenated at 50 psi and 50 °C for 16 h. The suspension was evaporated to yield a mixture of the title compounds (0.180g).

**Description 33: (RS)-2-[(Methoxy-methyl-carbamoyl)-methyl]-piperidine-1-carboxylic acid *tert*-butyl ester**

A solution of 2-carboxymethyl-piperidine-1-carboxylic acid *tert*-butyl ester (Beckett et al, J,Med.Chem., 563,1969) (2.29g, 10mmol) in DMF (20ml) was treated sequentially with *N,N*-diisopropylethylamine (4.0ml), HATU (3.8g, 10mmol) and *O,N*-dimethyl-hydroxylamine.HCl (0.98g, 10mmol). The reaction mixture was stirred under argon at room temperature for 16h. The volatiles were removed *in vacuo* and the residue was chromatographed (silica gel, diethyl ether) to afford the title compound as a white solid (2.60g, 90%).

Mass Spectrum (API$^+$):Found 187 (MH$^+$- 'BOC). $C_{14}H_{26}N_2O_4$ requires 286.

**Description 34: (RS)-2(2-Benzofuran-2-yl-2-oxo-ethyl)-piperidine-1-carboxylic acid *tert*-butyl ester**

To a solution of benzofuran (0.95g, 8.Ommol) in THF (40 ml), under argon at -40°C was added n-butyllithium (2.5M in hexanes) (4.00ml, 10.0mmol) over 5 min. The resultant mixture was stirred for 15 min. at -40°C then (RS)-2-[(methoxy-methyl-carbamoyl)-methyl]-piperidine- 1-carboxylic acid *tert*-butyl ester, D33 (2.30g, 8.0mmol) in THF (10ml) was added over 1 min. and the resultant solution stirred for 20 min. at -40°C. The mixture was poured into saturated ammonium chloride (20 ml) and extracted with ethyl acetate (3X). The combined organics were dried ($MgSO_4$) and the solvent removed *in vacuo.* The resultant residue was chromatographed (silica gel, dichloromethane) to afford the title compound (2.2g, 84%).

$^1H$ NMR $\delta$: 1.35 (9H, s), 1.44 (1H, m), 1.65 (5H, m), 2.94 (1H, dt, J = 3 and 13Hz), 3.17 (2H, m), 4.05 (1H, broad d), 4.89 (1H, m), 7.31 (1H, t, J = 8Hz), 7.48 (1H, m), 7.57 (2H, m), 7.72 (1H, d, J = 8Hz).

**Description 35: (RS)-1-Benzofuran-2-yl-2-piperidin-2-yl-ethanone**

A stirring solution of (RS)-2(2-benzofuran-2-yl-2-xo-ethyl)-piperidine-1-carboxylic acid *tert*-butyl ester, D34 (1.68g, 4.9mmol) in dichloromethane (20ml) was treated with trifluoroacetic acid (5ml). The mixture was stirred at 50°C for 1h, cooled and the volatiles removed *in vacuo.* The residue was dissolved in dichloromethane and washed with saturated sodium bicarbonate , dried ($MgSO_4$) and the solvent removed *in vacuo* to afford the title compound (1.20g, 99%).

Mass Spectrum (API$^+$):Found 244 (MH$^+$). $C_{15}H_{17}NO_2$ requires 243.

**Description 36: (RS)-1-Benzofuran-2-yl-2-(1-{1-[5-(4-fluoro-phenyl)-1-methyl-thiazol-4-yl]-methanoyl}-piperidin-2-yl)-ethanone**

The title compound was prepared from 5-(4-fluoro-phenyl)-2-methyl-thiazole-4-carboxylic acid and (RS)-1-benzofuran-2-yl-2-piperidin-2-yl-ethanone, D35 according to a procedure similar to that for Description 33.

Mass Spectrum (API$^+$): Found 463 (MH$^+$). $C_{26}H_{23}FN_2O_3S$ requires 462.

**Description 37: (RS)-4-Bromo-2-piperidin-2-ylmethyl-1H-benzimidazole**

The title compound was prepared using the method of Description 18 from 3-bromobenzene-1,2-diamine (0.50g, 2.7mmol) and 2-carboxymethyl-piperidine-1-carboxylic acid *tert*-butyl ester (0.65g, 2.7mmol), as a pale brown amorphous solid (0.70g, 88%)

Mass spectrum (Electrospray LC/MS): Found M-H 292. $C_{13}H_{16}{}^{79}BrN_3$ requires 293.

**Description 38: (RS)-1-(2-Benzofuran-2-ylmethyl-piperidin-1-yl)-2,2,2-trifluoro-ethanone**

To (RS)-2-(2-Benzofuranylmethyl)piperidine, D3 (500mg, 2.3mmol) in dichloromethane containing triethylamine (0.35ml, 2.5mmol) at 0°C under argon was added trifluoroacetic anhydride (0.36ml, 2.5mmol) in dichloromethane (3ml) over 5-10 min. The reaction was stirred at room temperature for 64h, diluted with dichloromethane and washed sequentially with water and saturated sodium hydrogen carbonate, dried ($Na_2SO_4$) and the solvent removed *in vacuo.* Chromatography (silica gel) afforded the title compound as a solid (740mg, 99%).

$^1H$ NMR δ: 1.50-1.90 (6H, m), 2.90-3.20 (2H, m), 3.27-3.35 (1H, m), 3.85-3.89 (0.66H, m), 4.51-4.54 (0.66H, m), 5.09-5-14 (0.66H, m), 6.48 and 6.50 (1H, s), 7.15-7.25 (2H, m), 7.39-7.45 (1H, m), 7.45-7.50 (1H, m).

**Description 39: (RS)-1-(2-(3-Bromo-benzofuran-2-ylmethyl-piperidin-1-yl)-2,2,2-trifluoroethanone**

To a solution of (RS)-1-(2-benzofuran-2-ylmethyl-piperidin-1-yl)-2,2,2-trifluoro-ethanone, D38 (700mg, 2.2mmol) in diethyl ether (10ml) at -12°C under argon was added a solution of bromine (360mg, 2.2mmol) in dichloromethane (4ml) drop-wise over 0.3h. The reaction mixture was allowed to reach room temperature and after 1h the volatiles were removed *in vacuo.* Re-evaporation from dichloromethane (3X) afforded the title compound as a solid (840mg, 96%). Mass spectrum (Electrospray LC/MS): Found 310 ($MH^+$-Br). $C_{16}H_{15}{}^{79}BrF_3NO_2$ requires 389.

**Description 40: (RS)-2-(1-(2,2,2-Trifluoro-ethanoyl)-piperidin-2-yl-methyl)-benzofuran-3-carbonitrile**

To a solution of (RS)-1-(2-(3-bromo-benzofuran-2-ylmethyl-piperidin-1-yl)-2,2,2-trifluoroethanone, D39 (840mg, 2.1mmol) in N-methylpyrrolidinone (15ml) was added copper(I)cyanide (387mg, 4.2mmol) and the mixture refluxed for 4.5h. The reaction mixture was cooled and diluted with water (100ml), 0.880 ammonia (3ml) and ethyl acetate (3X). The combined extracts were dried ($Na_2SO_4$) and the solvent removed *in vacuo.* The residual oil was redissolved in ethyl acetate (75ml)and washed with water (4X), brine, dried ($Na_2SO_4$) and the solvent removed *in vacuo.* Chromatography (silica gel) afforded the title compound as a solid.

Mass spectrum (Electrospray LC/MS): Found 337 ($MH^+$). $C_{17}H_{15}F_3N_2O_2$ requires 336.

**Description 41: (RS)-2-(1-Furo[3,2-b]pyridin-2-yl)methanoyl)piperidine-1-carboxylic acid *tert*-butyl ester**

The title compound was prepared using the method of Description 10 from furo[3,2-b]pyridine (1.14g, 9.6mmol) (Shiotani and Morita, *J.Het.Chem.*,23,665,1986), as a pale yellow solid (1.97g, 62%).

Mass Spectrum ($API^+$):Found 331($MH^+$). $C_{18}H_{12}N_2O_4$ requires 330.

**Description 42:(RS)-2-Piperidin-2-ylmethyl-furo[3,2-b]pyridine**

The title compound was prepared using the method of Description 3 from (RS)-2-(1-furo[3,2-b]pyridin-2-yl) methanoyl)piperidine-1-carboxylic acid *tert*-butyl ester, D41 (1.95g, 5.9mmol), as a pale green gum (0.75g, 59%).

Mass Spectrum ($API^+$):Found 217($MH^+$). $C_{13}H_{16}N_2O$ requires 216.

**Description 43: (RS)-4-Fluoro-2-piperidin-2-ylmethyl-1*H*-benzoimidazole**

(RS)-2-Carboxymethyl-piperidine-1-carboxylic acid *tert*-butyl ester (1.93g) was heated with 1,2-diamino-3-fluorobenzene (1.00g) (Kirk,K.L, J.Org. Chem.,1969, 34, 384) as described in Description 18 to afford the title compound (1.14g).

Mass Spectrum ($API^+$): Found 234($MH^+$). $C_{13}H_{16}FN_3$ requires 333.

**Description 44: (RS)-2-(5,6-Difluoro-1*H*-benzoimidazol-2-ylmethyl)-piperidine-1-earboxylic acid *tert*-butyl ester**

A solution of (RS)-5,6-difluoro-2-piperidin-2-ylmethyl-1*H*-benzoimidazole, D18 (1.5g) in dichloromethane was treated with triethylamine (0.98ml), then di-*tert*-butyl dicarbonate (1.3g).

After stirring for 18h at room temperature under argon the mixture was diluted with dichloromethane then washed with HCl (2M). The aqueous phase was basified with solid $K_2CO_3$ and extracted with dichloromethane. The organic phase was washed with aqueous $NaHCO_3$, brine and dried ($MgSO_4$). The solvent was removed *in vacuo* to afford the title compound (2g).

Mass Spectrum ($API^+$): Found 352 ($MH^+$). $C_{18}H_{23}F_2N_3O_2$ requires 351.

**Description 45: (RS)-2-(5,6-Difluoro-1-methyt-1*H*-benxoimidazol-2-ylmethyl)-piperidine-1-carboxylic acid *tert*-butyl ester**

To a stirred suspension of sodium hydride (0.46g, 60% dispersion in mineral oil) in dimethylformamide (30ml) at 0°C, under argon, was added (RS)-2-(5,6-difluoro-1*H*-benzoimidazol-2-ylmethyl)-piperidine-1-carboxylic acid *tert*-butyl ester, D44 (2g) and the reaction mixture stirred for a further 30min. before the addition of iodomethane (0.78ml). The reaction mixture was then stirred at room temperature for 18h, a further equivalent of iodomethane (0.35ml) was added and stirring continued for 24h. Further portions of sodium hydride (0.23g, 60% dispersion in mineral oil) and iodomethane (0.35ml) were added and the reaction stirred for an additional 24h. After diluting with water, the aqueous layer was extracted with diethyl ether (3X). The combined organics were dried ($MgSO_4$) and solvent removed *in vacuo* to afford the title compound (0.5g).

Mass Spectrum (Electrospray LC/MS): Found 266 ($MH^+$-$^t$BOC). $C_{19}H_{25}F_2N_3O_2$ requires 365.

**Description 46:(RS)-5,6-Difluoro-1-methyl-2-piperidin-2-ylmethyl-1*H*-benzoimidazole**

(RS)-2-(5,6-Difluoro-1-methyl-1*H*-benzoimidazol-2-ylmethyl)-piperidine-1-carboxylic acid *tert*-butyl ester, D45 (0.5g) was treated with trifluoroacetic acid (5ml) as described for Description 6 to afford the title compound (0.363g)

Mass spectrum (Electrospray): Found 266 ($MH^+$). $C_{14}H_{17}F_2N_3$ requires 265.

**Description 47: 1-(2-Trimethylsilanyl-ethoxymethyl)-1*H*-indole**

A stirring, ice-cooled solution of indole (5.86g, 50mmol) in DMF (130ml) under argon was treated with sodium hydride (3.3g, 60% dispersion on mineral oil, 80mmol) and the reaction mixture then stirred at room temperature for 45min. After cooling to 0°C 2,2-(trimethylsilyl)ethoxymethyl chloride **(**12.5g, 75mmol) was added dropwise. The reaction mixture was then stirred at room temperature for 4h. The DMF was removed *in vacuo,* the residue was poured into water and the product was extracted with diethyl ether. The combined organics were washed with brine, dried ($Na_2SO_4$) and the solvent removed *in vacuo.* Chromatography (silica gel, 0-20% diethyl ether-40-60 pet.ether) afforded the title compound (12.2g, 100%).

$^1$HNMR δ: 0.00 (9H, s), 0.96 (2H, t, J = 8Hz), 3.53 (2H, t, J = 8Hz), 5.55 (2H, s), 6.59 (1H, m), 7.20 (2H, m), 7.30 (1H, m with $CHCl_3$), 7.55 (1H, dd, J = 1 and 8Hz), 7.70 (1H, dd, J = 1 and 8Hz).

**Description 48: (RS)-2-{1-[1-(2-Trimethylsilanyl-ethoxymethyl)-1*H*-indol-2-yl]-methanoyl}-piperidine-1-carboxylic acid *tert*-butyl ester**

A stirring, ice-cooled solution of 1-(2-trimethylsilanyl-ethoxymethyl)-1*H*-indole, D47 (742mg, 3mmol) in DME (5ml) was treated drop-wise with n-butyl lithium (2.2ml, 1.6M in hexanes, 3.5mmol). The resulting green solution was stirred at 0-5°C for 15min. then was treated with (RS)-1-(*tert*-butyloxycarbonyl)-2-(N-methoxy-N-methylcarbamoyl)-piperidine, D 1 (952mg, 3.5mmol) in DME (2.5ml). After stirring at this temperature for a further 45 min. saturated aqueous $NH_4Cl$ was added (10ml) and the product was extracted with diethyl ether. The organics were combined, the solvent removed *in vacuo* and the residue chromatographed (silica gel, 10% diethyl ether-pentane) to afford the title compound as a colourless oil (5 10mg, 37%).

Mass Spectrum (API$^+$): Found 359 ($MH^+$-$^t$BOC). $C_{25}H_{38}N_2O_4Si$ requires 458.

**Description 49: (RS)- 2-Piperidin-2-ylmethyl-1-(1-trimethylsilanyl-ethoxymethyl)-1*H*-indole**

The title compound (1.1g) was prepared from (RS)-2-{1-[1-(2-trimethylsilanyl-ethoxymethyl)-1*H*-indol-2-yl]-methanoyl}-piperidine-1-carboxylic acid *tert*-butyl ester, D48 (1.9g) according to a procedure similar to that for Description 3 and used without purification.

**Description 50: (RS)-2-[1-(5-Bromo-benzofuran-2-yl)-methanoyl]-piperidine-1-carboxylic acid *tert*-butyl ester**

The title compound (0.96g) was prepared from 5-bromo-benzofuran (3.6g, prepared according to procedures similar to those described in Synth.Commun.,

257, (1989)) and (RS)-1-(tert-butyloxycarbonyl)-2-(N-methoxy-N-methylcarbamoyl)-piperidine, D1 (4.9g) according to a procedure similar to that for Description 2.

Mass Spectrum (API$^+$): Found 308 ($MH^+$-$^t$BOC). $C_{19}H_{22}{}^{79}BrNO_4$ requires 407.

**Description 51:(RS)-2-(5-Bromo-benzofuran-2-ylmethyl)-piperidine**

The title compound (0.53g) was prepared from (RS)- 2-[1-(5-bromo-benzofuran-2-yl)-methanoyl]-piperidine-1-carboxylic acid *tert*-butyl ester, D50 (1.00g) according to a procedure similar to that for Description 3 and used without purification.

Mass Spectrum (API$^+$): Found 294 (MH$^+$). $C_{14}H_{16}{}^{79}BrNO$ requires 293.

**Description 52: (RS)- 2-[1-(4-Bromo-benzofuran-2-yl)-methanoyl]-piperidine-1-carboxylic acid *tert*-butyl ester**

The title compound (1.45g) was prepared from 4-bromo-benzofuran (3.65g, WO0109111) and (RS)-1-(*tert*-butyloxycarbonyl)-2-(N-methoxy-N-methylcarbamoyl)-piperidine, D1 (5.17g) according to a procedure similar to that for Description 2.

Mass Spectrum (API$^+$); Found 308 (MH$^+$-$^t$BOC). $C_{19}H_{21}{}^{79}BrNO_4$ requires 407.

**Description 53: (RS)-2-(4-Bromo-benzofuran-2-ylmethyl)-piperidine**

The title compound (0.90g) was prepared from (RS)- 2-[1-(4-bromo-benzofuran-2-yl)-methanoyl]-piperidine-1-carboxylic acid *tert*-butyl ester, D52 (1.40g) according to a procedure similar to that for Description 3 and used without purification.

**Description 54:(RS)- 2-[1-(3-Methyl-benzofuran-2-yl)-methanoyl]-piperidine-1-carboxylic acid *tert*-butyl ester**

The title compound (300mg) was prepared from 3-methyl-benzofuran (390mg) and (RS)-1-(*tert*-butyloxycarbonyl)-2-(N-niethoxy-N-methylcarbarnoyl)-piperidine, D1 (820mg) according to a procedure similar to that for Description 2.

Mass Spectrum (API$^+$): Found 244 (MH$^+$-$^t$BOC). $C_{20}H_{25}NO_4$ requires 343.

**Description 55: (RS)-2-(3-Methyl-benzofuran-2-ylmethyl)-piperidine**

The title compound (184mg) was prepared from (RS)- 2-[1-(3-methyl-benzofuran-2-yl)-methanoyl]-piperidine-1-carboxylic acid *tert*-butyl ester, D54 (300mg) according to a procedure similar to that for Description 3 and used without purification.

**Description 56: 2-[1-(4-Fluoro-benzofuran-2-yl)-methanoyl]-piperidine-1-carboxylic acid-*tert*-butyl ester**

The title compound (500mg) was prepared from 4-fluoro-benzofuran (450mg) and (RS)-1-(*tert*-butyloxycarbonyl)-2-(N-methoxy-N-methylcarbamoyl)-piperidine, D1 (900mg) according to a procedure similar to that for Description 2.

Mass Spectrum (API$^+$): Found 248 (MH$^+$-$^t$BOC). $C_{19}H_{22}FNO_4$ requires 347.

**Description 57: (RS)- 2-(4-Fluoro-benzofuran-2-ylmethyl)-piperidine**

The title compound (310mg) was prepared from (RS)- 2-[1-(4-fluoro-benzofuran-2-yl)-methanoyl]-piperidine-1-carboxylic acid *tert*-butyl ester, D56 (500mg) according to a procedure similar to that for Description 3 and used without purification.

**Description 58: (RS)-2-(1- Benzo[b]thiophen-2-yl-methanoyl)-piperidine-l-carboxylic acid *tert*-butyl ester**

The title compound (1.60g) was prepared from benzo[b]thiophene(0.80g) and (RS)-1-(*tert*-butyloxycarbonyl)-2-(N-methoxy-N-methylcarbamoyl)-piperidine, D1 (1.62g) according to a procedure similar to that for Description 2.

**Description 59: (RS)- 2-Benzo[b]thiophen-2-ylmetbyl-piperidine**

The tide compound (410mg) was prepared from (RS)-2-(1-benzo[b]thiophen-2-yl-methanoyl)-piperidine-1-carboxylic acid *tert*-butyl ester, D58 (666mg) according to a procedure similar to that for Description 3 and used without purification.

**Example 1: (RS)-2-(2-Benzofuranylmethyl)-1-((5-(4-fluorophenyl)-2-methyl-thiazol-4-yl)-carbonyl)-piperidine**

5-(4-(Fluorophenyl)-2-methyl-thiazole-4-carbonyl chloride (136 mg, 0.53 mmol) in dichloromethane (1 ml) was added to a solution of (RS)-2-(2-benzofuranyhnethyl)piperidine (104 mg, 0.48 mmol) and triethylamine (0.20 ml, 1.45 mmol) in dichloromethane (4 ml) and the mixture shaken at ambient temperature for 30 min. The resultant was washed with saturated aqueous sodium hydrogen carbonate (8 ml). The organic layer was applied directly onto a pre-packed silica gel column and chromatographed eluting with ethyl acetate hexane mixtures to give the title compound (50 mg, 24 %) as an off-white solid.

Mass spectrum (API$^+$): found 435 (MH$^+$): $C_{25}H_{23}FN_2O_2S$ requires 434.

**Example 2: (RS)-1-(2-Benzooxazol-2-ylmethyl-piperidin-1-yl)-1-[5-(4-fluoro-phenyl)-2-methyl-thiazol-4-yl]-methanone**.

5-(4-Fluoro-phenyl)-2-methyl-thiazole-4-carbonylic acid (0.05g) in dimethylformamide (2.5ml) was treated sequentially with diisopropylethylamine (0.12ml), [O-(7-azabenzotriazol-1-yl)-1,1,3,3-tetramethyluronium hexafluorophosphate] (0.08g) and the amine of Description 6 (0.047g) in dimethylformaide (2.5ml). The mixture was stirred for 16h, diluted with ethyl acetate and the organic phase washed with water, dried (MgS04) and solvent removed at reduced pressure. The residue was chromatographed (silica gel, 0 → 30% ethyl acetate:pentane) to give the title compound (0.06g)

Mass spectrum (API$^+$): found 436 (MH$^+$); $C_{24}H_{22}FN_3O_2S$ requires 435.

**Example 3: (RS)-1-(2-Benzooxazol-2-ylmethyl-piperidin-1-yl)-1-[2-(3-methyl-[1,2,4]oxadiazol-5-yl)-phenyl]-methanone**

The title compound (0.02g) was prepared from the amine of Description 6 (0.047g) and 2-(3-methyl-[1,2,4] oxadiazol-5-yl)-benzoic acid (0.045g) according to the method of Example 2.

Mass spectrum (API$^+$): found 403 (MH$^+$): $C_{23}H_{22}N_4O_3$ requires 402.

**Example 4: (RS)-1-(2-Benzofuran-2-yhnethyl-pipendin-1-yl)-1-[4-(4-fluoro-phenyl)-1-methyl-1-*H*-pyrazol-3-yl]-methanone**

A mixture of (RS)-2-(2-benzofuranylmethyl)piperidine, D3 (0.108g, 0.5mmol), 4-(4-fluorophenyl)-1-methyl-1*H*-pyrazole-3-carboxylic acid (0.11g, 0.5mmol), EDC.HCl(0.106g, 0.55 mmol) and 1-hydroxybenzotriazole (0.01g, 0.07mmol) in dichloromethane (4ml) was shaken for 20h. The resultant was washed with saturated aqueous sodium bicarbonate (8ml) and the organic layer was added directly onto a dry 10g prepacked silica gel cartridge. Elution with 10-100% ethyl acetate in hexane gradient then 1-20% methanol in ethyl acetate gradient afforded the title compound as a colourless amorphous solid (0.059g, 28%).

$^1$H NMR ($CDCl_3$) δ: 1.05 - 1.30 (1H, m), 1.35 - 1.80 (5H, m), 2.80 - 3.20 (3H, m), 3.53 (0.55 H, broad m), 3.81 and 3.93 (3H, 2 x s), 4.13 (0.45 H, broad m), 4.74 (0.45 H, broad m), 5.41 (0.55 H, broad m), 6.26 and 6.60 (1H, 2 x s), 6.85 and 6.95 (2H, 2 x t, J = 8.6 Hz), 7.10 - 7.55 (7H, m). Mass spectrum (Electrospray LC/MS): Found 418 (MH$^+$). $C_{25}H_{24}FN_3O_2$ requires 417.

The compounds of Examples 5-22 and 81-82 in Table 1 were prepared from the appropriate amine and acid using similar procedures to that described in Example 4. The compound of Example 13 was prepared as the hydrochloride salts.

Table 1

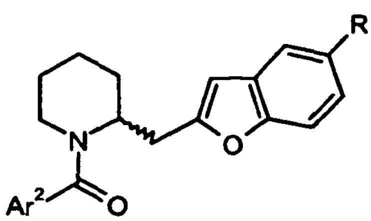

Table continued

| Example | $Ar^2$ | R | Mass Spectrum (Electrospray LC/MS) |
|---|---|---|---|
| 5 | 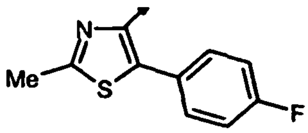 | F | Found $MH^+$453. $C_{25}H_{22}F_2N_2O_2S$ requires 452 |
| 6 | 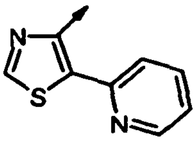 | F | Found $MH^+$422. $C_{23}H_{20}FN_3O_2S$ requires 421 |
| 7 | 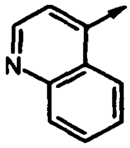 | F | Found $MH^+$389. $C_{24}H_{21}FN_2O_2$ requires 388 |
| 8 | 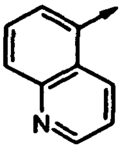 | F | Found $MH^+$389. $C_{24}H_{21}FN_2O_2$ requires 388 |
| 9 | 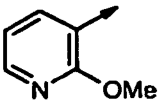 | F | Found $MH^+$369. $C_{21}H_{21}FN_2O_3$ requires 368. |
| 10 | 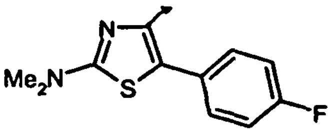 | F | Found $MH^+$482. $C_{26}H_{25}F_2N_3O_2S$ requires 481. |
| 11 | 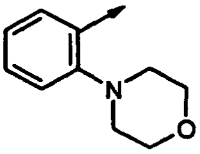 | F | Found $MH^+$423. $C_{25}H_{27}FN_2O_3$ requires 422. |
| 12 | 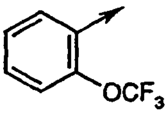 | F | Found $MH^+$422. $C_{22}H_{19}F_4NO_3$ requires 421. |
| 13 | 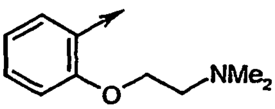 | H | Found $MH^+$407. $C_{25}H_{30}N_2O_3$ requires 406 |
| 14 | 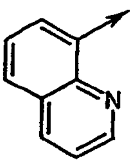 | H | Found $MH^+$371. $C_{24}H_{22}N_2O_2$ requires 370. |

Table continued

| Example | $Ar^2$ | R | Mass Spectrum (Electrospray LC/MS) |
|---|---|---|---|
| **15** | 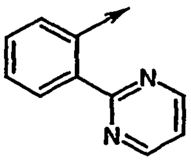 | H | Found $MH^+$398. $C_{25}H_{23}N_3O_2$ requires 397. |
| **16** | 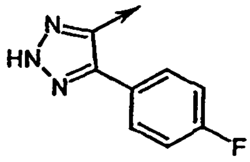 | H | Found $MH^+$405. $C_{23}H_{21}FN_4O_2$ requires 404. |
| **17** | 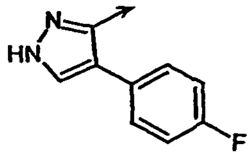 | H | Found $MH^+$404. $C_{24}H_{22}FN_3O_2$ requires 403. |
| **18** | 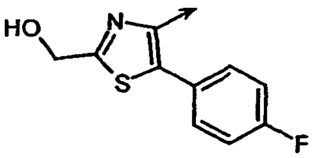 | H | Found $MH^+$ 451. $C_{25}H_{23}FN_2O_3S$ requires 450. |
| **19** | 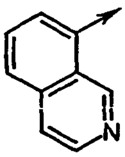 | H | Found $MH^+$371. $C_{24}H_{22}N_2O_3$ requires 370 |
| **20** | 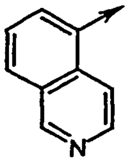 | H | Found $MH^+$371. $C_{24}H_{22}N_2O_2$ requires 370. |
| **21** | 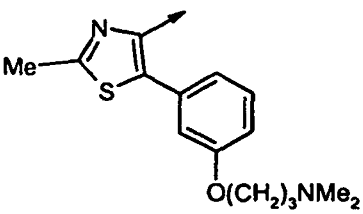 | H | Found $MH^+$518. $C_{30}H_{35}N_3O_3S$ requires 517. |
| **22** | 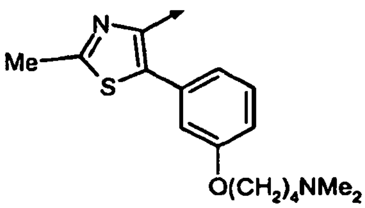 | H | Found $MH^+$532. $C_{31}H_{37}N_3O_3S$ requires 531. |

Table continued

| Example | $Ar^2$ | R | Mass Spectrum (Electrospray LC/MS) |
|---|---|---|---|
| **81** | 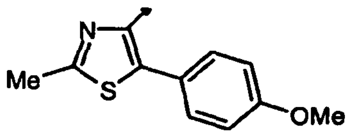 | H | Found $MH^+$447. $C_{26}H_{26}N_2O_3S$ requires 446. (API LC/MS) |
| **82** | 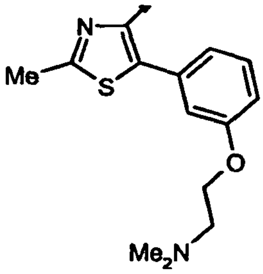 | H | Found $MH^+$ 504. $C_{29}H_{33}N_3O_3S$ requires 436. |

**Example 23: (RS)-1-[5-(4-Fluoro-phenyl)-2-methyl-thiazol-4-yl]-1-(2-furo[2,3-*b*]pyridin-2-ylmethyl-piperidin-1-yl)-methanone**

The title compound was prepared, using the method of Example 4, from 5-(4-fluoro-phenyl)-2-methyl-thiazole-4-carboxylic acid (0.171g, 0.72 mmol) and (RS)-2-piperidin-2-ylmethyl-furo[2,3-*b*]pyridine, D11 (0.13g, 0.60 mmol) as a colourless solid (0.129g, 49%).

$^1$H NMR δ: 0.90 - 1.20 (1H, m), 1.30 - 1.85 (5H, m), 2.49 and 2.70 (3H, 2 x s), 2.80 - 3.20 (3H, m), 3.40 (0.45H, m), 4.10 (0.55H, m), 4.70 (0.55, m), 5.35 (0.45H, m), 6.34 and 6.65 (1H, 2 x s), 6.92 and 7.00 (2H, 2 x t, J = 8.6 Hz), 7.15 (1H, m), 7.35 - 7.50 (2H, m), 7.80 (1H, m), 8.23 (1H, m).

Mass spectrum (Electrospray LC/MS): Found 436 ($MH^+$). $C_{24}H_{22}FN_3O_2S$ requires 435.

**Example 24: (RS)-1-[4-(4-Fluoro-phenyl)-1-methyl-1*H*-pyrazole-3-yl]-(2-furo[2,3-*b*]pyridin-2-ylmethyl-piperidin-1-yl)methanone**

The title compound was prepared using the method of Example 4, from 4-(4-(fluoro-phenyl)-1-methyl-1*H*-pyrazole-3-carboxylic acid (0.159g, 0.72mmol) and (RS)-2-piperidin-2-ylmethyl-furo[2,3-*b*]pyridine, D11(0.13g, 0.60mmol) as a colourless solid (0.12g, 48%).

Mass Spectrum (Electrospray LC/MS): Found 419 ($MH^+$). $C_{24}H_{23}FN_4O_2$ requires 418.

**Example 25: (RS)-1-[5-(4-Fluoro-phenyl)-2-methyl-thiazol-4-yl]-1-(2-quinolin-2-ylmethyl-piperidin-1-yl)-methanone**

The title compound was prepared, using the method of Example 1, from 5-(4-fluoro-phenyl)-2-methyl-thiazole-4-carbonyl chloride (0.09g, 0.35mmol) and 2-piperidin-2-ylmethyl-quinoline, D13 (0.053g, 0.23mmol) as a colourless solid (0.038g, 37%).

$^1$H NMR (DMSO-$d_6$) δ: 1.00-1.90 (6H, m), 2.37 and 2.66 (3H, 2 x s), 2.90-3.30 (3.5H, m), 4.15 (0.5H, m), 4.45 (0.5H, m), 5.25 (0.5H, m), 7.05 (2H, m), 7.10 - 7.85 (6H, m), 7.90 (1H, m), 8.05 - 8.30 (1H, 2xd, J = 8.4Hz).

Mass spectrum (Electrospray LC/MS): Found 446 ($MH^+$). $C_{26}H_{24}FN_3OS$ requires 445.

**Example 26: (RS)-1-Benzofuran-2-yl-1-(1-{1-[5-(4-fluoro-phenyl)-2-methyl-thiazol-4-yl]-methanoyl}-piperidin-2-yl)-methanone**

The title compound was prepared, using the method of Example 1, from 5-(4-fluoro-phenyl)-2-methyl-thiazole-4-carbonyl chloride (0.1g, 0.39 mmol) and (RS)-1-benzofuran-2-yl-1-piperidin-2-yl-methanone, D9 (0.1g, 0.43 mmol) as a colourless solid (0.074g, 43%)

Mass spectrum (Electrospray LC/MS): Found 449 ($MH^+$). $C_{25}H_{21}FN_2O_3S$ requires 448.

**Example 27: (RS)-1-[2-(1H-benzoimidazol-2-ylmethyl)-piperidin-1-yl]-1-[5-(4-fluoro-phenyl)-2-methyl-thiazol-4-yl]-methanone**

(RS)-(1-{1-[5-(4-Fluoro-phenyl)-2-methyl-thiazol-4-yl]-methanoyl}-piperidin-2yl)acetic acid, D17 (110mg, 0.3mmol) and benzene-1,2-diamine (33mg, 0.3mmol) in PPA (2g) were heated at 140°C for 3.5h. The cooled reaction mixture was poured onto a mixture of crushed ice and $K_2CO_3$. The basic aqueous solution was extracted with ethyl acetate (3X). The combined organics were washed with brine, dried ($MgSO_4$) and the solvent removed *in vacuo.* Chromatography (silica gel, ethyl acetate) afforded the title compound as a dark yellow gum (72mg, 52%).
Mass Spectrum (Electrospray LC/MS): Found 435 ($MH^+$). $C_{24}H_{23}FN_4OS$ requires 434.
The compounds of the Examples 28-34 in Table 2 below were prepared from the appropriate benzene- 1,2-diamine and (RS)-(1-{1-[5-(4-fluoro-phenyl)2-methyl-thiazol-4-yl]-methanoyl}-piperidin-2yl)-acetic acid, D17 using similar procedures to that described in Example 27.

**Table 2**

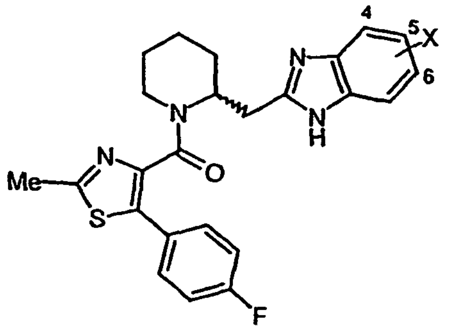

| Example I | X | Mass Spectrum (Electrospray LC/MS) |
|---|---|---|
| **28** | 5-Cl | Found $MH^+$469. $C_{24}H_{22}{}^{35}ClFN_4OS$ requires 468. |
| **29** | 5-F | Found $MH^+$453. $C_{24}H_{22}F_2N_4OS$ requires 452. |
| **30** | 5-C1,6-F | Found $MH^+$487. $C_{24}H_{21}{}^{35}ClF_2N_4OS$ requires 486. |
| **31** | 4-Me | Found $MH^+$ 449. $C_{25}H_{25}FN_4OS$ requires 448. |
| **32** | 4,6-di-F | Found $MH^+$ 471. $C_{24}H_{21}F_3N_4OS$ requires 470. |
| **33** | 4-$CH_2NMe_2$ | Found $MH^+$492. $C_{27}H_{30}FN_5OS$ requires 491. |
| **34** | 5-Br | Found $MH^+$ 513. $C_{24}H_{22}{}^{79}BrFN_4OS$ requires 512. |

**Example 35: (RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone**

A solution of 5-(4-fluoro-phenyl)-2-methyl-thiazole-4-carboxylic acid (700mg, 3.0 mmol), HATU (1.12g, 3.0mmol) and N,N-diisopropylethylamine (1.54ml, 9.0mmol) in DMF (20ml) was stirred at room temperature under argon for 2 min. The mixture was then treated with (RS)-5,6-difluoro-2 piperidin-2-ylmethyl-1*H*-benzoimidazole, D18 (742 mg, 3.0mmol) and stirring was continued for 16h. The solvent was removed *in vacuo* and the residue partitioned between water and ethyl acetate. The organic phase was dried ($Na_2SO_4$) and the solvent removed *in vacuo.* Chromatography (silica gel, 0-5% methanol-ethyl acetate) afforded the title compound as a pale yellow solid (1.35g, 97%).
Mass spectrum (Electrospray LC/MS): Found 471 ($MH^+$). $C_{24}H_{21}F_3N_4OS$ requires 470
$^1H$ NMR δ: 0.80-1.90 (6H + $H_2O$, bm), 2.71 and 2.75(3H, 2Xs), 3.04 (1H, dt, J = 3 and 13Hz), 3.17 (0.8H, dd, J = 5 and 13Hz), 3.42-3.58 (1.8H, bm), 3.74 (0.2H, m), 4.43 (0.4H, m), 5.35 (1H, 0.8H, m), 6.75 (1.6H, t, J = 8Hz), 7.00-7.20 (3H, bm), 7.40-7.57 (1.4H, m), 11.30 and 12.38 (1H, 2Xbs).
The title compound (900mg) was dissolved in methanol and treated with 1M HCl in diethyl ether (excess). The volatiles were removed *in vacuo* and the residue was triturated with ethyl acetate to give a white solid (805mg). A sample (450mg) was crystallised form ethyl acetate to give the hydrochloride salt of the title compound as white solid (420mg).
Mass spectrum (Electrospray LC/MS) Found 471.$C_{24}H_{21}F_3N_4OS$ requires 470.

**Example 36: (RS)-1-[2-(4,5-Difluoro-1*H*-benzoimidazol-2-ylmethyl)-piperidin-1-yl]-1-[5-(4-fluoro-phenyl)-2-methyl-thiazol-4-yl]-methanone**

A solution of 5-(4-fluoro-phenyl)-2-methyl-thiazole-4-carboxylic acid (1.00g, 4.2mmol), HATU (1.60g, 4.2mmol) and N,N-diisopropylethylamine (2.00ml, 11.6mmol) in DMF (20ml) was stirred at room temperature under argon for 2 min. The mixture was then treated with (RS)-4,5-difluoro-2 piperidin-2-ylmethyl-1*H* benzoimidazole, D21 (1.06g, 4.2mmol) and stirring was continued for 16h. The solvent was removed *in vacuo* and the residue was washed with water then was triturated with ethyl acetate to afford the title compound as an off-white solid (1.68g, 85%).

Mass spectrum (Electrospray LC/MS): Found 471 ($MH^+$) $C_{24}H_{21}F_3N_4OS$ requires 470.

The title compound (650mg) was dissolved in methanol and treated with 1M HCl in diethyl ether (excess). The volatiles were removed *in vacuo* and the residue was triturated with ethyl acetate to afford the hydrochloride salt of the title compound as a white solid (615mg).

Mass spectrum (Electrospray LC/MS) Found 471 ($MH^+$). $C_{24}H_{21}F_3N_4OS$ requires 470.

**Example 37: (RS)-1-[2-(5,6-Difluoro-1*H*-benzoimidazol-2-ylmethyl)-piperidin-1 yl]-1-[5-(4-fluoro-phenyl)-2-hydroxymethyl-thiazol-4-yl]-methanone**

A strirring solution of 5-(4-fluoro-phenyl)-2-hydroxymethyl-thiazole-4-carboxylic acid (632mg, 2.0mmol, 80% pure), EDC.HCl (311mg, 2.0mmol) and 1-hydroxybenzotriazole (270mg, 2.0mmol) in DMF (20ml) was treated with (RS)-5,6-difluoro-2-piperidin-2-ylmethyl-1*H*-benzoimidazole, D18 (500mg, 2.0mmol). The reaction mixture was stirred at room temperature, under argon for 16h. The DMF was removed *in vacuo* and the residue was partitioned between ethyl acetate and water. The organic phase was dried ($Na_2SO_4$) and the solvent removed *in vacuo.* Chromatography (silica gel, 0-10% methanol-ethyl acetate) afforded the title compound as a pale yellow solid (350mg, 36%).

Mass spectrum (Electrospray LC/MS): Found 487 ($MH^+$). $C_{24}H_{21}F_3N_4O_2S$ requires 486.

$^1H$ NMR (DMSO-$d_6$) δ: 0.90-1.80 (6H, bm), 2.92-3.30 (3H + DMSO, bm), 4.15 (0.4H, m), 4.40-4.60 (1H, bm), 4.73 (1H, d, J = 9Hz), 5.30 (0.6H, m), 6.20 (1H, m), 7.05-7.65 (6H, bm), 12.24 and 12.56 (1H, 2Xbs).

The title compound (350mg) was dissolved in methanol and treated with 1M HCl in diethyl ether (excess). The volatiles were removed *in vacuo* and the residue was triturated with EtOAc to afford the hydrochloride salt of the title compound as a white solid (215mg). Mass spectrum (Electrospray LC/MS) Found 487 ($MH^+$)$C_{24}H_{21}F_3N_4O_2S$ requires 486.

**Example 38: (RS)-1-[2-(4,5-Difluoro-1*H*-benzoimidazol-2-ylmethyl)-piperidin-1-yl]-1-[5-(4-fluoro-phenyl)-2-hydroxymethyl-thiazol-4-yl]-methanone**

A strirring solution of 5-(4-fluoro-phenyl)-2-hydroxymethyl-thiazole-4-carboxylic acid (320mg, 1.0mmol, 80% pure) and (RS)-4,5-difluoro-2-piperidin-2-ylmethyl-1*H*-benzoimidazole, D21 (250mg, 1.0mmol) in DMF (10ml) was treated with EDC.HCl (194mg, 1.0mmol) and 1-hydroxybenzotriazole (50mg, 0.4mmol). The reaction mixture was stirred at room temperature, under argon for 16h. The resulting solution was diluted with diethyl ether then washed with saturated aqueous $NaHCO_3$, water (3X) and brine. Drying ($MgSO_4$) and removal of the solvent *in vacuo* afforded an orange gum. Chromatography (silica gel, 0-2% methanol-dichloromethane) afforded the title compound as an orange powder (160mg, 33%).

Mass spectrum (Electrospray LC/MS) Found 487 ($MH^+$). $C_{24}H_{21}F_3N_4O_2S$ requires 486.

The title compound (80mg) was dissolved in methanol and treated with 1M HCl in diethyl ether (excess). The volatiles were removed *in vacuo* and the residue was triturated with diethyl ether to afford the hydrochloride salt of the title compound as a pale orange solid (65mg).

Mass spectrum (Electrospray LC/MS): Found 487 ($MH^+$). $C_{24}H_{21}F_3N_4O_2S$ requires 486.

**Example 39: (RS)-1-[2-(5,6-Difluoro-1*H*-benzoimidazol-2-ylmethyl)-piperidin-1-yl]-1-[4-(4-fluoro-phenyl)-1-methyl-1H-pyrazol-3-yl]-methanone**

A solution of 4-(4-fluoro-phenyl)-1-methyl-1*H*-pyrazole-3-carboxylic acid (73mg, 0.33mmol), HATU (124mg, 0.33mmol) and *N,N*-diisopropylethylamine (0.35ml) in DMF (6ml) was stirred at room temperature under argon for 0.5h. The mixture was then treated with (RS)-5,6-difluoro-2-piperidin-2-ylmethyl-1*H*-benzoimidazole.2HCl, D20 (105mg, 0.33mmol) and stirring, under argon was continued for 16h. The reaction mixture was diluted with diethyl ether and washed with water (3X), dried ($MgSO_4$) and the solvent removed *in vacuo.* Chromatography (silica gel, ethyl acetate) afforded the title compound (88mg, 57%).

Mass spectrum (Electrospray LC/MS): Found 454 ($MH^+$). $C_{24}H_{22}F_3N_5O$ requires 453.

**Example 40: (RS)-1-[2-(5-Methoxy-1*H*-benzoimidazol-2-ylmethyl)-piperidin-1-yl]-1-[5-(4-fluoro-phenyl)-2-methyl-thiazol-4-yl]-methanone**

A stirring mixture of (1-{1-[5-(4-Fluoro-phenyl)-2-methyl-thiazol-4-yl]-methanoyl}-piperidin-2yl)-acetic acid, D17 (150mg, 0.42mmol) and 4-methoxy-benzene-1,2-diamine (58mg, 0.42mmol) was heated at 120°C for 4h. The cooled reaction mixture was dissolved in ethyl acetate, washed with sodium hydrogen carbonate (2X) then brine. The solvent was removed in *vacuo* and the residue was chomatographed (silica gel, ethyl acetate) to afford the title compound as a brown gum (25mg, 13%).

Mass spectrum (Electrospray LC/MS): Found 465 ($MH^+$). $C_{25}H_{25}FN_4O_2S$ requires 464.

The compounds of the Examples 41-50 and 83-99 were prepared from the appropriate carboxylic acid or acid chloride and (RS)-4,5-difluoro-2-piperidin-2-ylmethyl-1*H*-benzoimidazole, D21 using a similar procedure to that described in Example 2 or a procedure similar to that described in Examples 1 or 4. The compounds of Examples 47, 48 and 49 were converted to hydrochloride salts by treatment with 1M HCl in diethyl ether. The compound of Example 96 was prepared as the trifluoroacetate salt.

**Table 3**

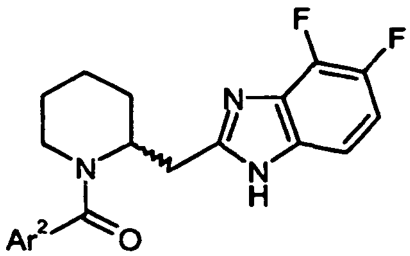

| Example | $Ar^2$ | Method | Mass Spectrum (Electrospray LC/MS) |
|---|---|---|---|
| **41** | 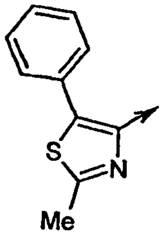 | E2 | Found $MH^+$453. $C_{24}H_{22}F_2N_4OS$ requires 452. |
| **42** | 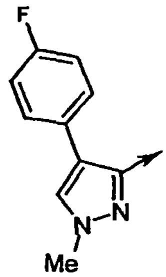 | E2 | Found $MH^+$454. $C_{24}H_{22}F_3N_5O$ requires 453. |
| **43** | 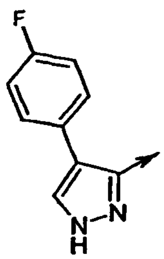 | E2 | Found $MH^+$440. $C_{23}H_{20}F_3N_5O$ requires 439. |

Table continued

| Example | $Ar^2$ | Method | Mass Spectrum (Electrospray LC/MS) |
|---|---|---|---|
| **44** | 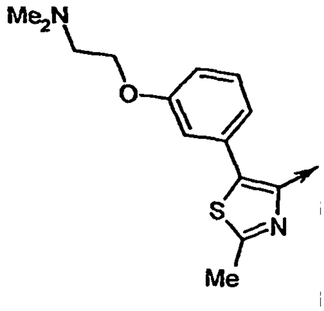 | E2 | Found $MH^+$540. $C_{28}H_{31}F_2N_5O_2S$ requires 539. |
| **45** | 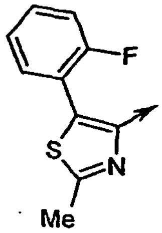 | E4 | Found $MH^+$471. $C_{24}H_{21}F_3N_4OS$ requires 470. |
| **46** | 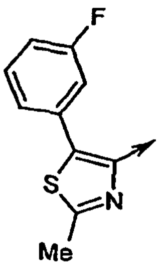 | E4 | Found $MH^+$471. $C_{24}H_{21}F_3N_4OS$ requires 470. |
| **47** | 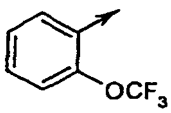 | E2 | Found $MH^+$440. $C_{21}H_{18}F_5N_3O_2$ requires 439. |
| **48** | 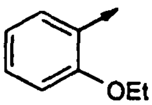 | E2 | Found $MH^+$400. $C_{22}H_{23}F_2N_3O_2$ requires 399. |
| **49** | 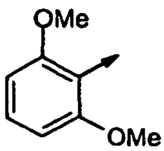 | E2 | Found $MH^+$ 416. $C_{22}H_{23}F_2N_3O$ requires 415. |
| **50** | 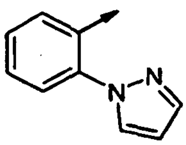 | E2 | Found $MH^+$422. $C_{23}H_{21}F_2N_5O$ requires 421. |
| **83** | 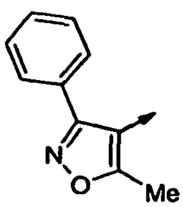 | E2 | Found $MH^+$ 437. $C_{24}H_{22}F_2N_4O_2$ requires 436. |

Table continued

| Example | Ar2 | Method | Mass Spectrum (Electrospray LC/MS) |
|---|---|---|---|
| **84** | 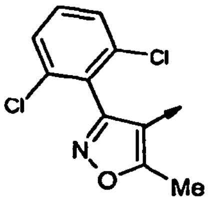 | E2 | Found MH+ 505. $C_{24}H_{20}{}^{35}Cl_2F_2N_4O_2$ requires 504. |
| **85** | 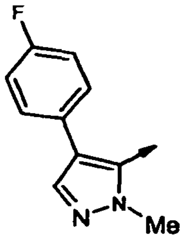 | E2 | Found MH+ 454. $C_{24}H_{22}F_3N_5O$ requires 453. |
| **86** | 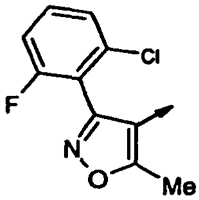 | E1 | Found MH+ 489. $C_{24}H_{20}\ {}^{35}ClF_3N_4O_2$ requires 488. |
| **87** | 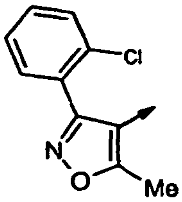 | E2 | Found MH+ 471. $C_{24}H_{21}\ {}^{35}ClF_2N_4O_2$ requires 470. |
| **88** | 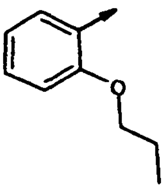 | E2 | Found MH+ 414. $C_{23}H_2F_2N_3O_2$ requires 413. |
| **89** | 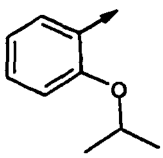 | E2 | Found MH+ 414. $C_{23}H_{25}F_2N_3O_2$ requires 413. |
| **90** | 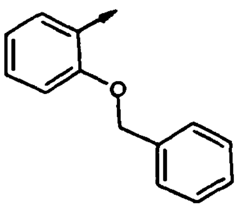 | E2 | Found MH+ 462. $C_{27}H_{25}F_2N_3O_2$ requires 461. |
| **91** | 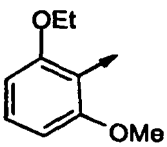 | E2 | Found MH+ 430. $C_{23}H_{25}F_2N_3O_3$ requires 429. |

Table continued

| Example | $Ar^2$ | Method | Mass Spectrum (Electrospray LC/MS) |
|---|---|---|---|
| 92 | 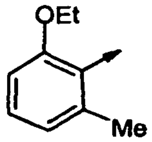 | E2 | Found $MH^+$ 414. $C_{23}H_{25}F_2N_3O_2$ requires 413. |
| 93 | 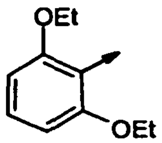 | E2 | Found $MH^+$ 444. $C_{24}H_{27}F_2N_3O_3$ requires 443. |
| 94 | 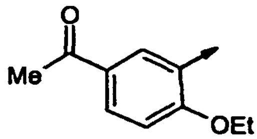 | E2 | Found $MH^+$ 442. $C_{24}H_{25}F_2N_3O_3$ requires 441. |
| 95 | 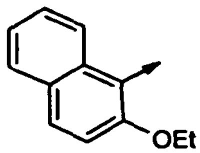 | E2 | Found $MH^+$ 450. $C_{26}H_{25}F_2N_3O_2$ requires 449. |
| 96 | 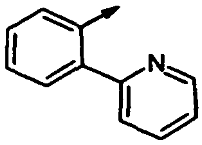 | E2 | Found $MH^+$ 433. $C_{25}H_{22}F_2N_4O$ requires 432. |
| 97 | 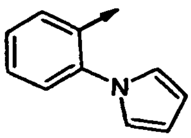 | E2 | Found $MH^+$ 421. $C_{24}H_{22}F_2N_4O$ requires 420. |
| 98 | 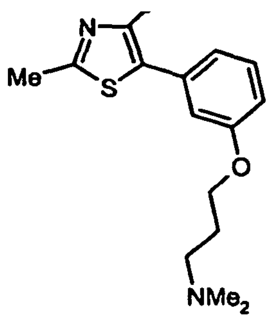 | E2 | Found $MH^+$ 554. $C_{29}H_{33}F_2N_5O_2S$ requires 553. |
| 99 | 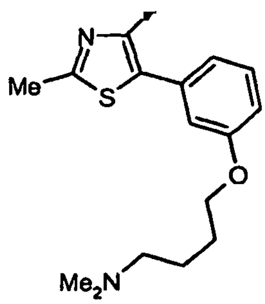 | E2 | Found $MH^+$ 568. $C_{30}H_{35}F_2N_5O_2S$ requires 567. |

The compounds of the Examples 51-55 and 100-103 in Table 4 were prepared from the appropriate carboxylic acid and (RS)-5,6-difluoro-2-piperidin-2-ylmethyl-177-benzoimidazole, D18 using a similar procedure to that described in Example 2 or Example 4 or from the appropriate acid chloride and (RS)-5,6-difluoro-2-piperidin-2-ylmethyl-1*H*-benzoirnidazole, D18 using a procedure similar to that described for Example 1. The compound of Example 55 was converted to the hydrochloride salt by treatment with 1M HCl in diethyl ether. The compound of Example 100 was prepared as the trifluoroacetate salt.

**Table 4**

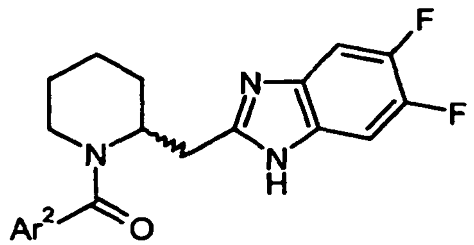

| Example | $Ar^2$ | Method | Mass Spectrum (Electrospray LC/MS) |
|---|---|---|---|
| **51** | 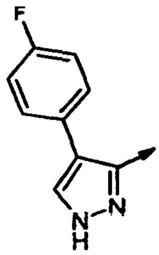 | E2 | Found $MH^+$ 440. $C_{23}H_{20}F_3N_5O$ requires 439. |
| **52** | 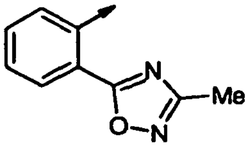 | E2 | Found $MH^+$438. $C_{23}H_{21}F_2N_5O_2$ requires 437 |
| **53** | 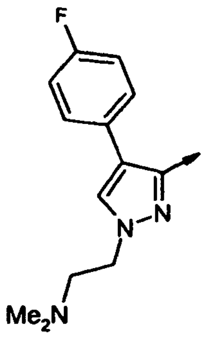 | E2 | Found $MH^+$511. $C_{27}H_{29}F_3N_6O$ requires 510 |
| **54** | 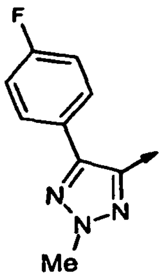 | E2 | Found $MH^+$455. $C_{23}H_{21}F_3N_6O$ requires 454 |

Table continued

| Example | $Ar^2$ | Method | Mass Spectrum (Electrospray LC/MS) |
|---|---|---|---|
| **55** | 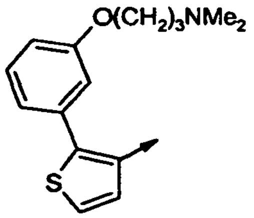 | E1 | Found $MH^+$539. $C_{29}H_{32}F_2N_4O_2S$ requires 538. |
| **100** | 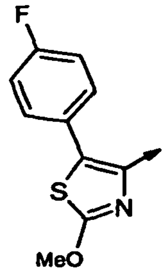 | E4 | Found $MH^+$ 487. $C_{24}H_{21}F_3N_4O_2S$ requires 486. |
| **101** | 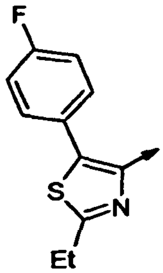 | E2 | Found $MH^+$ 485. $C_{25}H_{23}F_3N_4OS$ requires 484. |
| **102** | 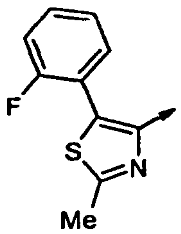 | E4 | Found $MH^+$ 471. $C_{24}H_{21}F_3N_4OS$ requires 470. |
| **103** | 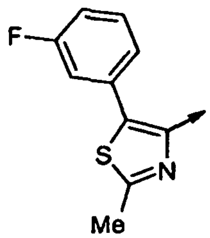 | E4 | Found $MH^+$ 471. $C_{24}H_{21}F_3N_4OS$ requires 470. |

**Example 56: (RS)-1-[2-(5,6-Difluoro-1-methyl-1*H*-benzoimldazol-2-ylmethyl)-piperidin-1-yl]-1-[5-(4-fluoro-phenyl)-2-methyl-thiazol-4-yl]-methanone**

(RS)-1-[2-(5,6-Difluoro-1*H* benzoimidazol-2-ylmethyl)-piperidin-1-yl]-1-[5-(4-fluoro-phenyl)-2-methyl-thiazol-4-yl]-methanone, E35 (400mg, 0.85mmol) was slowly added to an ice-cooled slurry of NaH (68mg, 60% dispersion on mineral oil, 1.7mmol) in DMF (10ml). After 0.5h iodomethane (0.90mmol) was added. The reaction mixture was stirred at room temperature, under argon for 16h. 2N HCl (9 drops) then water (100ml) were added. The aqueous phase was extracted with EtOAc(2X) and the combined organics washed with brine, dried ($Na_2SO_4$) and the solvent removed in *vacuo.* The residue was chromatographed (silica gel, 50-100% pentane-ethyl acetate) . Residual DMF was removed by dissolving in ethyl acetate/diethyl ether and washing with water (2X). Drying ($Na_2SO_4$) and removal of the solvent *in vacuo* afforded the title compound as a pale yellow solid (142mg, 34%).

Mass spectrum (Electrospray LC/MS): Found 485 ($MH^+$). $C_{25}H_{23}F_3N_4OS$ requires 484.

**Example 57: (RS)-1-(2-Benzothiazol-2-ylmetbylpiperidin-1-yl)-1-[5-(4-fluorophenyl)-2-methylthiazol-4-yl]-methanone**

2-Piperidin-2-ylmethylbenzothiazole, D28 (0.135 g), HATU (0.228 g), diisopropylethylamine (0.300 ml) and 5-(4-fluorophenyl)-2-methylthiazol-4-carboxylic acid (0.144g) were dissolved in dry DMF and shaken at room temperature for 16 hours. The solvent was then evaporated and the residue partitioned between dichloromethane and brine. The organic layer was dried ($MgSO_4$), evaporated and the residue chromatographed over silica gel. Elution with diethyl ether provided the title compound as a foam (220 mg).

Mass spectrum ($API^+$): found 452($MH^+$): $C_{24}H_{22}FN_3OS_2$ requires 451.

**Example 58: (RS)-1-(2-Benzothiazol-2-ylmethylpiperidin-1-yl)-1-[4-(4-fluorophenyl)-1-methyl-1-*H*-pyrazol-3-yl]-methanone**

The title compound (0.210 g) was prepared from 2-piperidin-2-ylmethyl-benzothiazole, D28 (0.135 g) and 4-(4-fluorophenyl)-1-methyl-1-*H*-pyrazol-3-carboxylic acid (0.135 g) using the procedure described in Example 57.

Mass spectrum ($API^+$): found 435($MH^+$): $C_{24}H_{23}FN_4OS$ requires 434.

**Example 59: (RS)-1-(2-Benzothiazol-2-ylmethylpiperidin-1-yl)-1-[2-(3-methyl-[1,2,4]oxadiazol-5-yl)phenyl]-methanone**

The title compound (0.170 g) was prepared from 2-piperidin-2-ylmethyl-benzothiazole, D28 (0.135 g), and 2-(3-methyl-[1,2,4]oxadiazol-5-yl)benzoic acid (0.126 g) using the procedure described in Example 57.

Mass spectrum ($API^+$): found 419($MH^+$): $C_{23}H_{22}N_4O_2S$ requires 418.

**Example 60 and Example 61: (RS)-1-[5-(4-Fluorophenyl)-2-methylthiazol4-yl]-1-[2-(5,6,7,8-tetrahydro-[1,2,4]-triazolo[1,5-a]pyridin-2-ylmethyl)-piperidin-1-yl]-methanone and (RS)-1-[5-(4-Fluorophenyl)-2-methylthiazol-4-yl]-1-(2-[1,2,4] triazolo [1,5a] pyridin-2-ylmethylpiperidin-1-yl)-methanone**

The mixture of 2-piperidin-2-ylmethyl-5,6,7,8-tetrahydro-[1,2,4]-triazolo[1,5-a]pyridine and 2-piperidin-2-ylmethyl-[1,2,4]-triazolo[1,5-a]pyridine, D32 (0.220 g) was treated with HATU (0.380 g), *N,N*-diisopropylethylamine (1.0 ml) and 5-(4-fluorophenyl)-2-methylthiazole-4-carboxylic acid (0.240g) in dry DMF, according to the procedure described in Example 57. The crude product was chromatographed (silica gel , 4% methanol-diethyl ether) to afford 1-[5-(4-fluorophenyl)-2-methylthiazol-4-yl]-1-(2-[1,2,4]-triazolo[1,5-a]pyridin-2-ylmethylpiperidin-1-yl)-methanone as a colourless foam (0.070 g) (Mass spectrum ($API^+$): found 436($MH^+$): $C_{23}H_{22}FN_5OS$ requires 435). Continued elution (10% methanol-diethyl ether) afforded 1-[5-(4-fluoro-phenyl)-2-methyl-thiazol-4-yl]-1-[2-(5,6,7,8-tetrahydro-[1,2,4]-triazolo[1,5-a]pyridin-2-ylmethyl)-piperidin-1-yl]-methanone as a colourless foam (0.230 g) (Mass spectrum ($API^+$): found 440($MH^+$): $C_{23}H_{26}FN_5OS$ requires 439).

**Example 62a and b : 1-[(RS)-2-((RS)-2-Benzofuran-2-yl-2-hydroxy-ethyl)-piperidin-1-yl]-1-[5-(4-fluoro-phenyl)-2-methyl-thiazol-4-yl]-methanone (as separate diastereoisomers)**

A solution of (RS)-1-Benzofuran-2-yl-2-(1-{1-[5 -(4-fluoro-phenyl)-2-methyl-thiazol-4-yl]-methanoyl}-piperidin-2-yl)-ethanone, D36 (50mg, 0.11 mmol) in methanol was treated with sodium borohydride (50mg). The reaction mixture was stirred at room temperature, under argon for 16h. The volatiles were removed in *vacuo* and the residue was triturated with dichloromethane. The dichloromethane soluble material was chromatographed (silica gel, diethyl ether) to afford, separately the two diastereoisomers of the title compound.

Mass spectrum ($API^+$) : found 447 ($MH^+$. $H_2O$): $C_{26}H_{25}FN_2O_3S$ requires 464 (first eluting diastereoisomer).

Mass spectrum ($API^+$): found 447 ($MH^+$- $H_2O$): $C_{26}H_{25}FN_2O_3S$ requires 464 (second eluting diastereoisomer)

**Example 63: (RS)-1-[2-(4-Bromo-1*H*-benzoimidazol-2-ylmethyl)-piperidin-1-yl]-1-[5-(4-fluorophenyl)-2-methylthiazol-4-yl]methanone**

2-Methyl-5-(4-fluorophenyl)thiazole-4-carbonyl chloride (383mg, 1.5mmol) was added portionwise over 5 min. to a stirred solution of (RS)-4-bromo-2-piperidin-2-ylmethyl-1*H*-benzoimidazole, D37 (400mg, 1.4mmol) and triethylamine (0.57ml, 5.0mmol) in dichloromethane (20ml) at room temperature. After 20h the mixture was washed with saturated sodium hydrogen carbonate. The organic layer was dried ($Na_2SO_4$) and the solvent removed *in vacuo.* Chromatography (silica gel, 10-100% ethyl acetate-hexane then 1-10% methanol-ethyl acetate) afforded the title compound as a pale brown amorphous solid (620mg, 89%).

Mass spectrum (Electrospray LC/MS): Found 513 ($MH^+$). $C_{24}H_{22}{}^{79}BrFN_4OS$ requires 512.

**Example 64: (RS)-1-[2-(4-Cyano-1*H*-benzoimidazol-2-ylmethyl)-piperidin-1-yl]-1-[5-(4-fluorophenyl)-2-methylthiazol-4-yl]methanone**

A mixture of (RS)-1-[2-(4-bromo-1*H*-benzoimidazol-2-ylmethyl)-piperidin-1-yl]-1-[5-(4-fluorophenyl)-2-methylthiazol-4-yl]methanone, E63 (216mg, 0.42mmol) and copper(I)cyanide (76mg, 0.84mmol) in N-methylpyrrolidinone (15ml) was heated at reflux for 3h. The reaction mixture was cooled, poured into water and extracted with ethyl acetate (2X). Solid sodium chloride was added to the aqueous layer which was further extracted with ethyl acetate (2X). The combined organic extracts were washed with brine, dried ($Na_2SO_4$) and the solvent removed *in vacuo.* The residue was Chromatograped (silica gel, 10-100% ethyl acetate-hexane then 1-20% methanol-ethyl acetate). Further purification by HPLC (Supercosil ABZ+, 5-95% acetonitrile containing 0.1% trifluoroacetic acid-water containing 0.1% trifluoroacetic acid) afforded the title compound as a pale brown amorphous solid (5.3mg, 3%).

Mass spectrum (Electrospray LC/MS): Found 460 ($MH^+$). $C_{25}H_{22}FN_5OS$ requires 459.

**Example 65: (RS)-1-[2-(4-Acetyl-1*H*-benzoimidazol-2-ylmethyl)-piperidin-1-yl]-1- [5-(4-fluoropbenyl)-2-methylthiazol-4-yl]methanone**

To a deoxygenated solution of (RS)-1-[2-(4-bromo-1*H*-benzoimidazol-2-ylmethyl)-piperidin-1-yl]-1-[5-(4-fluorophenyl)-2-methylthiazol-4-yl]methanone, E63 (200mg, 0.39mmol) in anhydrous 1,4-dioxane (10ml) was added tributyl (1-ethoxyvinyl)tin (155mg, 0.43mmol) and tetrakis(triphenylphosphine)palladium(0) (30mg, 0.026mmol). The resultant mixture was refluxed under argon for 20h. Further tetrakis(triphenylphosphine)palladium(0) (60mg, 0.052mmol) was added and refluxing continued for a further 24h. On cooling, water (15ml) and 5N hydrochloric acid (10 drops) were added and the mixture stirred at room temperature for 2h. The reaction mixture was poured into water and extracted with ethyl acetate (3X). The combined organics were dried ($Na_2SO_4$) and the solvent removed *in vacuo.* Chromatography (silica gel, 0-100% ethyl acetate-hexane then 2-5% methanol-ethyl acetate) afforded the title compound as a pale orange solid (62mg, 33%).

Mass spectrum (Electrospray LC/MS): Found 475 (M-H). $C_{26}H_{25}FN_4O_2S$ requires 476.

**Example 66: (RS)-2-(1-{1-[5-(4-Fluoro-phenyl)-2-methyl-thiazol-4-yl]-methanoyl}-piperidin-2-ylmethyl)-1H-benzoimidazole-5-carbonitrile**

A stirring mixture of (RS)-1-[2-(5-bromo-1H-benzoimidazol-2-ylmethyl)-piperidin-1-yl]-1-[5-(4-fluoro-phenyl)-2-methyl-thiazol-4-yl]-methanone, E34 (25mg, 0.44mmol) and copper(I)cyanide (79mg, 0.88mmol) in N-methylpyrrolidinone (7ml) under argon was heated at 200°C for 5h. The cooled reaction mixture was diluted with water/ethyl acetate and filtered through Kieselghur. The organic phase was washed with water (2X), brine (2X), dried ($MgSO_4$) and the solvent removed *in vacuo.* The residue was chromatograped (silica gel, 0-4% methanol (containing 10% .0.880 ammonia)- dichloromethane). Further purification by HPLC (Supercosil ABZ+, 5-95% acetonitrile containing 0.1% trifluoroacetic acid-water containing 0.1% trifluoroacetic acid) afforded the title compound as a pale brown gum (2mg, 1%).

Mass spectrum (Electrospray LC/MS): Found 460 ($MH^+$). $C_{25}H_{22}FN_5OS$ requires 459.

**Example 67: (RS)-1-[2-(5,6-Difluoro-1-propyl-1*H*-benzoimidazol-2-ylmethyl)-piperidin-1-yl]-1-[5-(4-fluoro-phenyl)-2-methyl-thiazol-4-yl]-methanone**

(RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone, E35 (150mg, 0.32mmol) was added slowly to an ice-cooled slurry of sodium hydride (26mg, 0.64mmol, 60% dispersion in mineral oil) in DMF (10ml). After stirring for a further 0.5h, 1-iodopropane (60mg, 0.35mmol) was added. The mixture was stirred at room temperature for 16h then partitioned between water and ethyl acetate. The organic phase was washed with water, brine, dried ($Na_2SO_4$) and the solvent removed *in vacuo.*

Chromatography (silica gel, 50-100% ethyl acetate-pentane then 0-5% methanol-ethyl acetate) afforded the title compound as a white solid.

Mass spectrum (Electrospray LC/MS): Found 513 ($MH^+$). $C_{27}H_{27}F_3N_4OS$ requires 512.

The product was dissolved in methanol and treated with 1M HCl in diethyl ether (excess). The volatiles were removed *in vacuo* to afford the hydrochloride salt of the title compound as pale green solid (170mg).

Mass spectrum (Electrospray LC/MS): Found 513. $C_{27}H_{27}F_3N_4OS$ requires 512.

**Example 68: (RS)-1-{2-[5,6-Difluoro-1-(2-methoxy-ethyl)-1*H*-benzoimidazol-2-ylmethyl]-piperidin-1-yl}-1-[5-(4-fluoro-phenyl)-2-methyl-thiazol-4-yl]-methanone**

(RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone, E35 (300mg, 0.64mmol) was added slowly to an ice-cooled slurry of sodium hydride (51mg, 1.3mmol, 60% dispersion in mineral oil) in DMF (10ml). After stirring for 0.5h 1-bromo-2-methoxy-ethane (139mg, 1.0mmol), *N,N*-diisopropylethylamine (0.3ml) and potassium iodide (5mg) was added. The mixture was stirred at room temperature for 0.5h then 100°C for 16h. The volatiles were removed *in vacuo* from the cooled reaction mixture. The residue was partitioned between ethyl acetate and water. The aqueous phase was extracted with ethyl acetate (2X). The combined organics were dried ($Na_2SO_4$) and the solvent removed in *vacuo.* The residue was triturated with ethyl acetate to afford the title compound as a solid (306mg, 65%).

Mass spectrum (Electrospray LC/MS) Found 529 ($MH^+$)- $C_{27}H_{27}F_3N_4O_2S$ requires 528.

The product was dissolved in methanol and treated with 1M HCl in diethyl ether (excess). The volatiles were removed *in vacuo* and the residue triturated with methanol-ethyl acetate to afford the hydrochloride salt of the title compound as a white solid (66mg).

Mass spectrum (Electrospray LC/MS): Found 529 ($MH^+$). $C_{27}H_{27}F_3N_4O_2S$ requires 528.

**Example 69: (RS)-1-{2-[1-(2-Dimethylamino-ethyl)-5,6-difluoro-1*H*-benzoimidazol-2-ylmethyl]-piperidin-1-yl}-1-[5-(4-fluoro-phenyl)-2-methyl-thiazol-4-yl]-methanone**

The title compound was prepared from (RS)-1-[2-(5,6-difluoro-1*H*-benzoimidazol-2-ylmethyl)-piperidin-1-yl]-1-[5-(4-fluoro-phenyl)-2-methyl-thiazol-4-yl]-methanone, E35 (300mg) and (2-chloro-ethyl)-dimethyl-amine, according to a procedure similar to that described for Example 68 as a white solid (88mg, 26%).

Mass spectrum (Electrospray LC/MS): Found 542 ($MH^+$). $C_{28}H_{30}F_3N_5OS$ requires 541.

The hydrochloride salt of the title compound was prepared as described for E71

Mass spectrum (Electrospray LC/MS): Found 542 ($MH^+$). $C_{28}H_{30}F_3N_5OS$ requires 541.

**Example 70: (RS)-1-{2-[5,6-difluoro-1-(hydroxy-ethyl)-1*H*-benzoimidazol-2-ylmethyl]-piperidin-1-yl}-1-[5-(4-fluoro-phenyl)-2-methyl-thiazol-4-yl]-methanone**

(RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone, E35 (250mg, 0.53mmol) was added slowly to an ice-cooled slurry of sodium hydride (43mg, 1.1mmol, 60% dispersion in mineral oil) in DMF (10ml), under argon. After stirring for a further 0.5h, 2-bromoethanol (0.045ml, 0.64mmol) was added. The mixture was heated at 110°C for 48h, but only a minor amount of product had formed (MS). Potassium carbonate (1.00g), N,N-diisopropylethylamine (1.0ml) and a further portion of 2-bromoethanol (0.5ml) were added and heating was continued for 16h. The volatiles were removed in *vacuo* from the cooled reaction mixture. The residue was partitioned between ethyl acetate and water. The organic phase was dried ($Na_2SO_4$) and the solvent removed *in vacuo.* Chromatography (silica gel, 0-5% methanol-ethyl acetate) afforded the title compound as a white solid (78mg, 29%).

Mass spectrum (Electrospray LC/MS): Found 515 ($MH^+$). $C_{26}H_{25}F_3N_4O_2S$ requires 514.

**Example 71 and Example 72: (RS)-1-[2-(6,7-Difluoro-1-methyl-1*H*-benzoimidazol-2-ylmetbyl)-plperidin-1-yl]-1-[5-(4-fluorophenyl)-2-methyl-thiazol-4-yl]-methanone and (RS)-1-[2-(4,5-Difluoro-1-methyl-1H benzoimidazol-2-ylmethyl)-piperidin-1-yl]-1-[5-(4-fluorophenyl)-2-methyl-thiazol-4-yl]-methanone**

(RS)-1-[2-(4,5-Difluoro-1*H*-benzoimidazol-2-ylmethyl)-piperidin-1-yl]-1-[5-(4-fluoro-phenyl)-2-methyl-thiazol-4-yl]-methanone, E36 (400mg, 0.85mmol) was added slowly to an ice-cooled slurry of sodium hydride (68mg, 1.7mmol, 60% dispersion in mineral oil) in DMF (10ml). After stirring for a further 0.5h, iodomethane (266mg, 0.87mmol) was added. The mixture was stirred at room temperature for 72h then partitioned between water and ethyl acetate. The organic phase was washed with water, brine, dried ($Na_2SO_4$) and the solvent removed *in vacuo.* Purification by HPLC (Supercosil ABZ$^+$, 5-95% acetonitrile containing 0.1% trifluoroacetic acid-water containing 0.1% trifluoroacetic acid) afforded the title compounds (structures assignment based on nOe experiments):

1-[2-(6,7-Difluoro-1-methyl-1*H*-benzoimidazol-2-ylmethyl)-piperidin-1-yl]-1-[5-(4-fluorophenyl)-2-methyl-thiazol-4-yl]-methanone (76mg)

Mass spectrum (Electrospray LC/MS): Found 485 ($MH^+$). $C_{25}H_{23}F_3N_4OS$ requires 484.

$^1$H NMR δ: 0.75-1.85 (6H, bm), 2.42 and 2.72 (3H, s), 0.85-3.15 (2H, bm), 3.30 (1H, m), 3.42 (0.7H, m), 3.70

and 4.15 (3H, s), 4.25 (0.3H, m), 4.75 (0.3H, m), 5.10 (0.7H, m), 7.00 (3H, m), 7.35 (3H, m).

1-[2-(4,5-Difluoro-1-methyl-1*H*-benzoimidazol-2-ylmethyl)-piperidin-1-yl]-1-[5-(4-fluorophenyl)-2-methyl-thiazol-4-yl]-methanone (106mg)

Mass spectrum (Electrospray LC/MS): Found 485 ($MH^+$). $C_{25}H_{23}F_3N_4OS$ requires 484.

$^1H$ NMR δ: 0.75-1.90 (6H, bm), 2.25 and 2.70 (3H, s), 2.9-3.2 (2H, bm), 3.15-3.45 (1.7H, bm), 3.56 and 3.94 (3H, s), 4.25 (0.3H, m), 4.76 (0.3H, m), 5.09 (0.7H, m), 6.98 (3H, m), 7.08(1H, m), 7.36 (2H, m).

**Example 73: (RS)-2-(1-{1-[5-(4-Fluorophenyl)-2-methyl-thiazol-4-yl]-methanoyl}-piperidin-2-ylmethyl)-benzofuran-3-carboxylic acid amide**

To a solution of (RS)-2-(1-(2,2,2-trifluoro-ethanoyl)-piperidin-2-yl-methyl)-benzofuran-3-carbonitrile, D40 (480mg, 1.4mmol) in methanol (15ml) and water (3ml) was added potassium carbonate (700mg, 4.9mmol) and the mixture heated at reflux for 1.25h. The solvent was removed in vacuo and the residue was partitioned between dichloromethane and water and basified to pH 14. The aqueous phase was re-extracted with dichloromethane (3X). The combined organics were dried ($Na_2SO_4$) and the solvent removed *in vacuo* to afford a solid (0.34g, 99%). To a portion of this material (67mg, 0.28mmol) in dichloromethane was added 5-(4-fluorophenyl)-2-methyl-thiazole-4-carboxylic acid (73mg, 0.30mmol), EDC.HCl (60mg, 0.30mml) and 1-hydroxybenzotriazole (10mg). After 16h at room temperature the reaction mixture was washed with saturated aqueous sodium hydrogen carbonate and the organic phase applied directly onto a pre-packed silica gel column. Elution with ethyl acetate- methanol mixtures afforded the title compound as an amorphous solid (8mg, 6%).

Mass spectrum (Electrospray LC/MS): Found 478 ($MH^+$). $C_{26}H_{24}FN_3O_3S$ requires 477.

**Example 74: (RS)-2-(1-{1-[4-(4-Fluorophenyl)-1-methyl-1*H*-pyrazol-3-yl]-methanoyl}-piperidin-2-ylmethyl)-benzofuran-3-carboxylic acid amide**

The title compound (7mg, 5%), was prepared as described for E73 from (RS)-2-(1-(2,2,2-trifluoro-ethanoyl)-piperidin-2-yl-methyl)-benzofuran-3-carbonitrile, D40 and 4-(4-fluorophenyl)-1-methyl-1H-pyrazole carboxylic acid (61mg, 0.3mmol)

Mass spectrum (Electrospray LC/MS): Found 461 ($MH^+$). $C_{26}H_{25}FN_4O_3$ requires 460.

**Example 75:(RS)-1-[5-(4-Fluoro-phenyl)-2-methyl-thiazol-4-yl]-1-(2-furo[3,2-b]-pyridin-2-ylmethyl-piperidin-1-yl)methanone**

The title compound was prepared using the method of Example 4, from 5-(4-fluoro-phenyl)-2-methyl-thiazole-4-carboxylic acid (120mg, 0.5mmol) and (RS)-2-piperidin-2-ylmethyl-furo[3,2-b]pyridine, D42 (108mg, 0.5mmol), as a colourless solid (95mg, 43%).

Mass spectrum (Electrospray LC/MS): Found 436 (NOT). $C_{24}H_{22}FN_3O_2S$ requires 435.

**Example 76:(RS)-1-[4-(4-Fluoro-phenyl)-1-methyl-1H-pyrazole-3-yl)-(2-furo[3,2-b]pyridin-2-ylmethyl-piperidin-1-yl)methanone**

The title compound was prepared using the method of Example 4, from 4-(4-fluoro-phenyl)-1-methyl-1H-pyrazole-3-carboxylic acid (110mg, 0.5mmol) and (RS)-2-Piperidin-2-ylmethyl-furo[3,2-b]pyridine, D42(108mg, 0.5mmol), as a colourless solid (130mg, 62%).

Mass spectrum (Electrospray LC/MS): Found 419 ($MH^+$). $C_{24}H_{23}FN_4O_2$ requires 418.

**Example 77: (RS)-1-[2-(3-Bromo-benzofuran-2-ylmethyl)-piperidin-1-yl]-1-[5-(4-fluorophenyl)-2-methyl-thiazol-4-yl]-methanone**

To (RS)-2-(2-benzofuranylmethyl)-1-((5-(4-fluorophenyl)-2-methyl-thiazol-4-yl)-carbonyl)-piperidine, E1 (0.90g, 2mmol) in dry diethyl ether (10ml) and dichloromethane (20ml) cooled to - 12°C under argon, was added dropwise a solution of bromine (0.36g, 2mmol) in dichloromethane (10ml) over 0.5h. The resulting solution was warmed to ambient temperature over 1.5h, then stirred for a further 18h. The reaction mixture was evaporated then re-evaporated from dichloromethane (3X). The resulting amorphous solid was chromatographed (silica gel with ethyl acetate-hexane mixtures) to afford the title compound as an amorphous solid (0.27g, 25%). Mass spectrum (Electrospray LC/MS) Found 513 ($MH^+$). $C_{25}H_{22}{}^{79}BrFN_2O_2S$ requires 512.

**Example 78: (RS)-2-(1-{1- [5-(4-Fluoro-phenyl)-2-methyl-thiazol-4-yl]-methanoyl}-piperidin-2-ylmethyl)-benzofuran-3-carbonitrile**

To (RS)-1 -[2-(3-bromo-benzofuran-2-ylmethyl)-pipehdin-1 -yl]-1-[5-(4-fluoro-phenyl)-2-methyl-thiazol-4-yl]-methanone, E77 (270mg, 0.53mmol) in N-methyl-pyrrolidinone (8ml) was added copper(I)cyanide (96mg, 1.06mmol) and the mixture stirred under argon at reflux for 6h. The reaction mixture was cooled, poured into water (80ml) and ethyl acetate (50ml) and then filtered through kieselguhr. The filtrate was separated and the aqueous phase extracted with ethyl acetate (2X). The combined organics were washed with water (4X50ml), dried and the solvent removed *in vacuo* . Chromatography (silica gel) afforded the title compound as a white amorphous solid (94mg, 39%).

Mass spectrum (Electrospray LC/MS) Found 460 ($MH^+$). $C_{26}H_{22}FN_3O_2S$ requires 459.

**Example 79: (RS)-1-[2-(1-(1-[5-(4-fluoro-phenyl)-2-methyl-thiazol-4-yl]-methanoyl)-piperidin-2-ylmethyl)-benzofuran-3-yl]-ethanone**

The title compound (120mg, 41%) was obtained from (RS)-1-[2-(3-bromo-benzofuran-2-ylmethyl)-piperidin-1-yl]-1-[5-(4-fluoro-phenyl)-2-methyl-thiazol-4-yl]-methanone, E77 (320mg, 0.62mmol) using the method of Example 65.

Mass spectrum (Electrospray LC/MS): Found 477 ($MH^+$). $C_{27}H_{25}FN_2O_3S$ requires 476.

**Example 80a and Example 80b: (R)-1-[2-(5,6-Difluoro-1*H*-benxoimidazol-2-ylmethyl)-piperidin-1-yl]-1-[5-(4-fluoro-phenyl)-2-methyl-thiazol-4-yl]-methanone and(S)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone**

(RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone E35 (380 mg) was separated to give the title compounds (stereochemistry not assigned) on a Chiralpak AD column (250mm x 19mm i.d.; 10 micron particle size); Mobile Phase: n-hexane, HiPerSiolv:ethanol, 99.7%v/v-100%v/v, Analar: triethylamine, Analar (90:10:0.25 v/v/v): pre-mixed: isocratic procedure; injecting at 2.5ml at concentation of 20mg/ml racemate in ethanol, 99.7%v/v-100%v/v, Analar.

Faster eluting enantiomer (132mg, >99.8% e.e.):
Mass spectrum (Electrospray LC/MS): Found 471 ($MH^+$). $C_{24}H_{21}F_3N_4OS$ requires 470
Slower eluting enantiomer (173mg, >99.8%e.e.):
Mass spectrum (Electrospray LC/MS): Found 471 ($MH^+$). $C_{24}H_{21}F_3N_4OS$ requires 470

**Table 5**

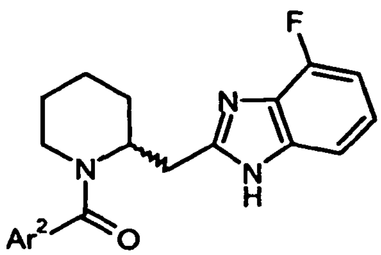

The compounds of Examples 104-109 were prepared from the appropriate carboxylic acid and (RS)-4-fluoro-2-piperidin-2-ylmethyl-1H-benzoimidazole, D43 using a procedure similar to that described in Example 2 or Example 4.

Table continued

| Example | $Ar^2$ | Method | Mass Spectrum (Electrospray LC/MS) |
|---|---|---|---|
| 104 | 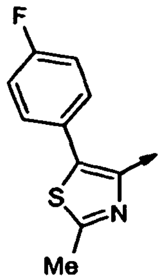 | E2 | Found $MH^+$ 453. $C_{24}H_{22}F_2N_4OS$ requires 452. |
| 105 | 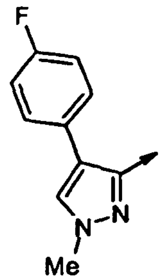 | E2 | Found $MH^+$ 436. $C_{24}H_{23}F_2N_5O$ requires 435. |
| 106 | 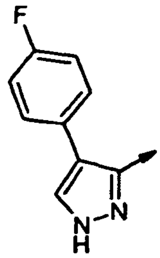 | E2 | Found $MH^+$ 422. $C_{23}H_{21}F_2N_5O$ requires 421. |
| 107 | 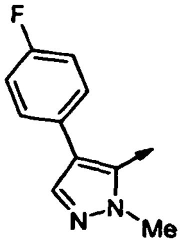 | E2 | Found $MH^+$ 436. $C_{24}H_{23}F_2N_5O$ requires 435. |
| 108 | 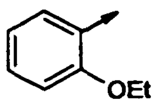 | E2 | Found $MH^+$ 382. $C_{22}H_{24}FN_3O_2$ requires 381. |
| 109 | 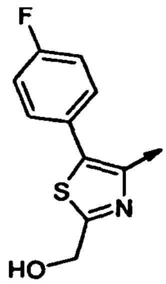 | E4 | Found M-1 467. $C_{24}H_{22}F_2N_4O_2S$ requires 468. (API LC/MS) |

**Example 110: (RS)-1-[S-(4-Fluoro-phenyl)-2-methyl-thiazol-4-yl]-1-{2-[4-(1-hydroxy-ethyl)-1H-benzoimidazol-2-ylmethyl]-piperidin-1-yl}-methanone**

Sodium borohydride (145mg) was added portionwise over 2 min. to a cooled (0-10°C) solution of (RS)-1-[2-(4-acetyl-1H benzimidazol-2-ylmethyl)-piperidin-1-yl]-1-[5-(4-fluorophenyl)-2-methylthiazol-4-yl]methanone, E65 (365mg) in methanol (10ml). After stirring for a further 2.5h at room temperature, water (100ml) was added and the mixture was extracted with dichloromethane (2X). The combined organics were dried ($Na_2SO_4$), evaporated and the residue chromatographed (silica gel, 0-100% ethyl acetate-hexane then 2-10% methanol-ethyl acetate) to afford the title compound as a solid (320mg).

Mass spectrum (Electrospray LC/MS): Found 479 ($MH^+$). $C_{26}H_{27}FN_4O_2S$ requires 478.

**Example 111: (RS)- 1-(2-Benzofuran-2-ylmethyl-piperidin-1-yl)-1-[5-(4-chloro-phenyl)-2-methyl-thiazol-4-yl]-methanone**

The title compound (100mg) was prepared from (RS)-2-(2-benzofuranylmethyl)piperidine, D3 (64mg) and 5-(4-chloro-phenyl)-2-methyl-thiazole-4-carboxylic acid (76mg) by a procedure similar to that described for Example 2.

Mass spectrum (API LC/MS): Found 451 ($MH^+$). $C_{25}H_{23}{}^{35}ClN_2O_2S$ requires 450.

**Example 112: (RS)- 1-[2-(3-Chloro-furo[3,2-b]pyridin-2-ylmethyl)-piperidin-1-yl]-1-[5-(4-fluoro-phenyl)-2-methyl-thiazol-4-yl]-methanone**

A solution of chlorine (28mg) in dichloromethane (3ml) was added to a cooled (-12°C) solution (RS)-1-[5-(4-fluoro-phenyl)-2-methyl-thiazol-4-yl]-1-(2-furo[3,2-b]-pyridin-2-ylmethyl-piperidin-1-yl)methanone, E75 (170mg) in dichloromethane (4ml). After stirring at -12°C for 0.5h the reaction mixture was stirred at room temperature for 16h then the solution was washed with saturated aqueous $NaHCO_3$, dried ($Na_2SO_4$) and the solvent removed *in vacuo.* Chromatography (silica gel, 0-100% ethyl acetate-hexane then 0-10% methanol-ethyl acetate) afforded the title compound (24mg).

Mass spectrum (Electrospray LC/MS): Found 470 ($MH^+$). $C_{24}H_{21}{}^{35}ClFN_3O_2S$ requires 469.

**Example 113: (RS)-1-[2-(5,6- Difluoro-1-methyl-1H-benzoimidazol-2-ylmethyl)-piperidin-1-yl]-1-[5-(4-fluoro-phenyl)-2-hydroxymethyl-thiazol-4-yl]-methanone**

The title compound (15mg) was prepared from 5-(4-fluoro-phenyl)-2-hydroxymethyl-thiazole-4-carboxylic acid (84mg) and (RS)-5,6-dinuoro-1-methyl-2-piperidin-2-ylmethyl-1H-benzoimidazole, D46 (86mg) by a procedure similar to that described for Example 4.

Mass spectrum (Electrospray LC/MS): Found 501($MH^+$). $C_{23}H_{23}F_3N_4O_2S$ requires 500.

**Example 114: (RS)-1-[5-(4-Fluoro-phenyl)-2-methyl-thiazol-4-yl]-1-[2-(1H-indol-2-ylmethyl)-piperidin-1-yl]-methanone**

(RS)- 1-[5-(4-Fluoro-phenyl)-2-methyl-thiazol-4-yl]-1-{2-[1-(2-trimethylsilanyl-ethoxymethyl)-1H-indol-2ylmethyl]-piperidin-1-yl}-methanone (130mg) was prepared from (RS)- 2-piperidin-2-ylmethyl-1-(2-trimethylsilanyl-ethoxymethyl)-1*H*-indole, D49 (520mg) and 5-(4-fluoro-phenyl)-2-methyl-thiazole-4-carbonyl chloride (512mg) according to a procedure similar to that for Example 1. A stirring solution of the above amide (125mg) in dry THF (5ml) was treated with a solution of tetrabutylammonium fluoride (2.2mmol) in THF (5ml). Once tic indicated the reaction had gone to completion, the reaction mixture was poured into water and the product extracted with ethyl acetate. The combined organics were washed with brine, dried ($Na_2SO_4$) and the solvent removed *in vacuo.* Chromatography (silica gel, 0-100% pentane-ethyl acetate followed by preparative tlc, 40% ethyl acetate-pentane) afforded the title compound as a white solid (2.5mg).

Mass spectrum (Electrospray LC/MS): Found 434($MH^+$). $C_{25}H_{24}FN_3OS$ requires 433.

**Example 115: (RS)- 5-[1-(2-Benzofuran-2-ylmethyl-piperidin-1-yl)-methanoyl]-4*H* benzo [1,4] oxazin-3-one**

The title compound (65mg) was prepared from (RS)-2-(2-benzofuranylmethyl)piperidine, D3 (65mg) and 3-oxo-3,4-dihydro-2H-benzo[1,4]oxazine-5-carboxylic acid (39mg) by a procedure similar to that for Example 2.

Mass spectrum (Electrospray LC/MS): Found 391($MH^+$) $C_{23}H_{22}N_2O_4$ requires 390.

**Example 116: (RS)- 1-[2-(5-Bromo-benzofuran-2-ylmethyl)-piperidin-1-yl]-1-[5-(4-fluorophenyl)-2-methyl-thiazol-4-yl]-methanone**

The title compound (490mg) was prepared from 5-(4-fluoro-phenyl)-2-methyl-thiazole-4-carbonyl chloride (488mg) and (RS)-2-(5-bromo-benzofuran-2-ylmethyl)-piperidine, D51 (530mg) by a procedure similar to that described for Example 1.

Mass spectrum (Electrospray LC/MS): Found 513 ($MH^+$). $C_{25}H_{22}{}^{79}BrFN_2O_2S$ requires 512.

**Example 117: (RS)- 1-[2-(5-Cyano-benzofuran-2-ylmethyl)-piperidin-1-yl]-1-[5-(4-fluorophenyl)-2-methyl-thiazol-4-yl]-methanone**

The title compound (19mg) was prepared from (RS)- 1-[2-(5-bromo-benzofuran-2-ylmethyl)-pipendin-1-yl]-1-[5-(4-fluoro-phenyl)-2-methylthiazol-4-yl]-methanone, E116 (420mg) and copper(I)cyanide according to a procedure similar to that described for Example 64.

Mass spectrum (API$^+$ LC/MS): Found 460 ($MH^+$). $C_{26}H_{22}FN_3O_2S$ requires 459.

**Example 118: (RS)- 1-[2-(4-Bromo-benzofuran-2-ylmethyl)-piperidin-1-yl]-1-[5-(4-fluorophenyl)-2-methyl-thiazol-4-yl]-methanone**

The title compound (0.45g) was prepared from 5-(4-fluoro-phenyl)-2-methyl-thiazole-4-carbonyl chloride (0.89g) and 2-(4-bromo-benzofuran-2-ylmethyl)-piperidine, D53 (0.88g) by a procedure similar to that described for Example 1.

Mass spectrum (Electrospray LC/MS): Found 513 ($MH^+$). $C_{25}H_{22}{}^{79}BrFN_2O_2S$ requires 512.

**Example 119:(RS)- 1-[2-(4-Cyano-benzofuran-2-ylmethyl)-piperidin-1-yl]-1-[5-(4-fluorophenyl)-2-methyl-thiazol-4-yl]-methanone**

The title compound (80mg) was prepared from (RS)- 1-[2-(4-bromo-benzofuran-2-ylmethyl)-piperidin-1-yl]-1-[5-(4-fluoro-phenyl)-2-methyl-thiazol-4-yl]-methanone, E118 (400mg) and copper(I)cyanide according to a procedure similar to that described for Example 64.

Mass spectrum (Electrospray LC/MS): Found 460 ($MH^+$). $C_{26}H_{22}FN_3O_2S$ requires 459.

**Example 120: (RS) -1-[5-(4-Fluoro-phenyl)-2-methyl-thiazol-4-yl]-1-[2-(3-methyl-benzofuran-2-ylmethyl)-piperidin-1-yl]-methanone**

The title compound (18mg) was prepared from (RS)-2-(3-methyl-benzofüran-2-ylmethyl)-piperidine, D55 (92mg) and 5-(4-fluoro-phenyl)-2-methyl-thiazole-4-carboxylic acid (190mg) according to a procedure similar to that described for Example 4.

Mass spectrum (API LC/MS): Found 449 ($MH^+$). $C_{26}H_{25}FN_2O_2S$ requires 448.

**Example 121: (RS)- 1-[2-(4-Fluoro-benzofuran-2-ylmethyl)-piperidin-1-yl]-1-[5-(4-fluorophenyl)-2-methyl-thiazol-4-yl]-methanone**

The title compound (60mg) was prepared from (RS)-2-(4-fluoro-benzofuran-2-ylmethyl)-piperidine, D57 (157mg) and 5-(4-fluoro-phenyl)-2-methyl-thiazole-4-carboxylic acid (160mg) according to a procedure similar to that described for Example 4.

Mass spectrum (API LC/MS): Found 453 ($MH^+$). $C_{25}H_{22}F_2N_2O_2S$ requires 452.

**Example 122: (RS)- 1-[2-(4-Fluoro-benzofuran-2-ylmethyl)-piperidin-1-yl]-l-[4-(4-fluorophenyl)-1-methyl-1H-pyrazol-3 -yl]-methanone**

The title compound (45mg) was prepared from (RS)-2-(4-fluoro-benzofuran-2-ylmethyl)-piperidine, D57 (157mg) and 4-(4-fluoro-phenyl)-1-methyl-1*H*- pyrazole-3-carboxylic acid (160mg) according to a procedure similar to that described for Example 4.

Mass spectrum (API LC/MS): Found 436 ($MH^+$). $C_{25}H_{23}F_2N_3O_2$ requires 435.

**Example 123: (RS)- 1-(2-Benzo[b] thiophen-2-ylmethyl-piperidin-1-yl)-1-[5-(4-fluorophenyl)-2-methyl-thiazol-4-yl]-methanone**

The title compound (40mg) was prepared from 2-benzo[b]thiophen-2-ylmethyl-piperidine D59(133mg) and 5-(4-fluoro-phenyl)-2-methyl-thiazole-4-carboxylic acid (136mg) according to a procedure similar to that described for Example 4.

Mass spectrum (Electrospray LC/MS): Found 451 ($MH^+$). $C_{25}H_{23}FN_2OS_2$ requires 450.

**Example 124: (RS)- 1-(2-Benzo[b]thiophen-2-ylmethyl-piperidin-1-yl)-1-[4-(4-fluorophenyl)-1-methyl-1H-pyrazol-3-yl]-methanone**

The title compound (6mg) was prepared from 2-benzo[b]thiophen-2-ylmethyl-piperidine D59(133mg) and 4-(4-fluoro-phenyl)-1-methyl-1*H*-pyrazole-3-carboxylic acid (140mg) according to a procedure similar to that described for Example 4.

Mass spectrum (Electrospray LC/MS): Found 434 ($MH^+$). $C_{25}H_{24}FN_3OS$ requires 433.

**Example 125: (RS)- 1-(2-Benzo[b]thiophen-2-ylmethyl-piperidin-1-yl)-1-quinolin-8-yl-methanone**

The title compound (4mg) was prepared from 2-benzo[b]thiophen-2-ylmethyl-piperidine D59(133mg) and 8-quinoline carboxylic acid (109mg) according to a procedure similar to that described for Example 4.

Mass spectrum (Electrospray LC/MS): Found 387 ($MH^+$). $C_{24}H_{22}N_2OS$ requires 386

**Example 126: (RS)-1-(2-Benzofuran-2-ylmethyl-piperidin-1-yl)-1-(2-methyl-5-phenyl-thiazol-4-yl)-methanone**

The title compound (90mg) was prepared from (RS)-2-(2-benzofuranylmethyl) piperidine, D3 (100mg) and 5-phenyl-2-methyl-thiazole-4-carbonyl chloride (122mg) according to a procedure similar to that for Example 1.

Mass spectrum (Electrospray LC/MS): Found 417 ($MH^+$). $C_{25}H_{24}N_2O_2S$ requires 416.

**Example 127: (RS)- 1-(2-Benzofuran-2-yhmethyl-piperidin-1-yl)-1-[2-(3-methyl-[1,2,4]oxadiazol-5-yl)-phenyl]-methanone**

The title compound (33mg) was prepared from (RS)-2-(2-benzofuranylmethyl) piperidine, D3 (108mg) and 2-(3-methyl-[1,2,4]oxadiazol-5-yl)-benzoic acid (102mg) according to a procedure similar to that for Example 2.

Mass spectrum (Electrospray LC/MS): Found 402 ($MH^+$). $C_{24}H_{23}N_3O_3$ requires 401.

**Example 128a and Example 128b: (R)-1-[2-(4,6-Difluoro-1*H* benzoimidazol-2-ylmethyl)-piperidin-1-yl]-1-[5-(4-fluoro-phenyl)-2-methyl-thiazol-4-yl]-methanone and (S)-1-[2-(4,6-Difluoro-1*H*-benzoimidazol-2-ylmethyl)-piperidin-1-yl]-1-[5-(4-fluoro-phenyl)-2-methyl-thiazol-4-yl]-methanone (enantiomers of Example 32)**(RS)-1-[2-(4,6- Difluoro- 1*H*- benzoimidazol- 2- ylmethyl)-piperidin- 1- yl]- 1-[5-(4- fluoro- phenyl)- 2- methyl- thiazol- 4- yl]-methanone, E32 was separated to give the title compounds (stereochemistry not assigned) using the following procedures:

**Analytical separation**: 10uL of diluted sample (compound in methanol) was separated on a 4.6x250mm,10 micron, analytical ChiralCel OD column. A Berger Analytical SFC (Super Critical Fluid) instrument was used with system parameters as follows: mobile phase= 90% CO2: 10% organic modifier, organic modifier = 90%methanol:10% chloroform v/v, 3000psi, 2mL/min, 40C $\lambda$=254 nm

The enantiomers eluted at 6.4 min. and 8.1 min.

**Preparative separation**: Scale-up was preformed on a Prochrom Super C.20 instrument. The instrument parameters were as follows : 40C, 21 MPa, 40g/min CO2+ 4.5ml/min organic modifier (as above), $\lambda$=300nm, ChiralCel OD 20x250mm, 10 micron column. 30 mg of racemate was introduced per cycle. Sample concentration= 875mg/13ml solvent (sample solvent = 5ml chloroform and 8mL methanol). Cycle time (run time)= 17minutes. The enantiomers eluted at 10.2 and 13.6 min. Elution times measured at peak maxima.

It is understood that the present invention covers all combinations of particular and preferred groups described herein above.

**Determination of Oresin-1 Receptor Antagonist Activity**

The orexin-1 receptor antagonist activity of the compounds of formula (I) was determined in accordance with the following experimental method.

**Experimental Method**

CHO-DG44 cells expressing the human orexin-1 receptor were grown in cell medium (MEM medium with Earl's salts) containing 2 mM L-Glutamine, 0.4 mg/mL G418 Sulphate from GIBCO BRL and 10% heat inactivated fetal calf serum from Gibco BRL. The cells were seeded at 20,000 cells/100 μl/well into 96-well black clear bottom sterile plates from Costar which had been pre-coated with 10 μg/well of poly-L-lysine from SIGMA. The seeded plates were incubated overnight at 37C in 5% $CO_2$.

Agonists were prepared as 1 mM stocks in water:DMSO (1:1). EC50 values (the concentration required to produce 50% maximal response) were estimated using 11 x half log unit dilutions (Biomek 2000, Beckman) in Tyrode's buffer containing probenecid (10 mM HEPES with 145mM NaCl, 10mM glucose, 2.5 mM KCl, 1.5 mM $CaCl_2$, 12 mM $MgCl_2$ and 2.5mM probenecid; pH7.4). Antagonists were prepared as 10 mM stocks in DMSO (100%). Antagonist IC50 values (the concentration of compound needed to inhibit 50% of the agonist response) were determined against 3.0 nM human orexin-A using 11x half log unit dilutions in Tyrode's buffer containing 10% DMSO and probenecid.

On the day of assay 50 μl of cell medium containing probenecid (Sigma) and Fluo3AM (Texas Fluorescence Laboratories) was added (Quadra, Tomtec) to each well to give final concentrations of 2.5 mM and 4 μM, respectively. The 96-well plates were incubated for 60 min at 37C in 5% CO2. The loading solution containing dye was then aspirated and cells were washed with 4x150 μl Tyrode's buffer containing probenecid and 0.1% gelatin (Denley Cell Wash). The volume of buffer left in each well was 125 μl. Antagonist or buffer (25 μl) was added (Quadra) the cell plates gently shaken and incubated at 37C in 5% $CO_2$ for 30 minutes. Cell plates were then transferred to the Fluorescent Imaging Plate Reader (FLIPR, Molecular Devices) instrument. Prior to drug addition a single image of the cell plate was taken (signal test), to evaluate dye loading consistency. The run protocol used 60 images taken at 1 second intervals followed by a further 24 images at 5 second intervals. Agonists were added (by the FLIPR) after 20 seconds (during continuous reading). From each well, peak fluorescence was determined over the whole assay period and the mean of readings 1-19 inclusive was subtracted from this figure. The peak increase in fluorescence was plotted against compound concentration and iteratively curve fitted using a four parameter logistic fit (as described by Bowen and Jerman, *TiPS,* 1995, 16, 413-417) to generate a concentration effect value. Antagonist Kb values were calculated using the equation:

$$Kb = IC50/(1+([3/EC50])$$

where EC50 was the potency of human orexin-A determined in the assay (in nM terms) and IC50 is expressed in molar terms.

Compounds of Examples tested according to this method had pKb values >7.0 to 9.4 at the human cloned orexin-1 receptor.

The orexin-2 receptor antagonist activity of the compounds of formula (I) was determined in accordance with the following experimental method.

**Experimental Method**

CHO-DG44 cells expressing the human orexin-2 receptor were grown in cell medium (MEM medium with Earl's salts) containing 2 mM L-Glutamine, 0.4 mg/mL G418 Sulphate from GIBCO BRL and 10% heat inactivated fetal calf serum from Gibco BRL. The cells were seeded at 20,000 cells/100 μl/well into 96-well black clear bottom sterile plates from Costar which had been pre-coated with 10 μg/well of poly-L-lysine from SIGMA. The seeded plates were incubated overnight at 37C in 5% $CO_2$.

Agonists were prepared as 1 mM stocks in water.DMSO (1:1). EC50 values (the concentration required to produce 50% maximal response) were estimated using 11x half log unit dilutions (Biomek 2000, Beckman) in Tyrode's buffer containing probenecid (10 mM HEPES with 145mM NaCl, 10mM glucose, 2.5 mM KCl, 1.5 mM $CaCl_2$, 1.2 mM $MgCl_2$ and 2.5mM probenecid; pH7.4). Antagonists were prepared as 10 mM stocks in DMSO (100%). Antagonist IC50 values (the concentration of compound needed to inhibit 50% of the agonist response) were determined against 10.0 nM human orexin-A using 11x half log unit dilutions in Tyrode's buffer containing 10% DMSO and probenecid.

On the day of assay 50 μl of cell medium containing probenecid (Sigma) and Fluo3AM (Texas Fluorescence Laboratories) was added (Quadra, Tomtec) to each well to give final concentrations of 2.5 mM and 4 μM, respectively. The 96-well plates were incubated for 60 min at 37C in 5% $CO_2$. The loading solution containing dye was then aspirated and cells were washed with 4x150 μl Tyrode's buffer containing probenecid and 0.1% gelatin (Denley Cell Wash). The volume of buffer left in each well was 125 μl. Antagonist or buffer (25 μl) was added (Quadra) the cell plates gently shaken and incubated at 37C in 5% $CO_2$ for 30 min. Cell plates were then transferred to the Fluorescent Imaging Plate Reader (FLIPR, Molecular Devices) instrument. Prior to drug addition a single image of the cell plate was taken (signal

test), to evaluate dye loading consistency. The run protocol used 60 images taken at 1 second intervals followed by a further 24 images at 5 second intervals. Agonists were added (by the FLIPR) after 20 sec (during continuous reading). From each well, peak fluorescence was determined over the whole assay period and the mean of readings 1-19 inclusive was subtracted from this figure. The peak increase in fluorescence was plotted against compound concentration and iteratively curve fitted using a four parameter logistic fit (as described by Bowen and Jerman, *TiPS,* 1995, 16, 413-417) to generate a concentration effect value. Antagonist Kb values were calculated using the equation:

$$Kb = IC50/(1+([3/EC50])$$

where EC50 was the potency of human orexin-A determined in the assay (in nM terms) and IC50 is expressed in molar terms.

Compounds of Examples tested according to this method had pKb values in the range 6.5 - 8.4 at the human cloned orexin-2 receptor.

## Claims

**1.** A compound of formula (I):

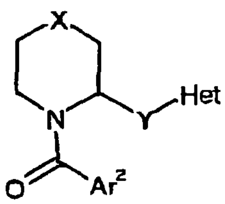

wherein

X represents $CH_2$;
Y represents $CH_2$, CO, CH(OH), or $CH_2CH(OH)$;
Het is an optionally substituted bicyclic heteroaryl group selected from the group consisting of quinoxalinyl, quinazolinyl, pyridopyrazinyl, benzoxazolyl, benzothienyl, benzofuranyl, benzimidazolyl, naphthyridinyl, benzothiazolyl, indolyl, triazolopyridinyl, furopyridinyl, pyridopyrimidinyl, isoquinolinyl, quinolinyl, oxazolylpyridinyl, tetrahydrobenzimidazolyl, tetrahydrobenzofuranyl, or tetrahydrotriazolopyridinyl;
$Ar^2$ represents an optionally substituted phenyl or a 5- or 6-membered heterocyclyl group selected from the group consisting of furanyl, thienyl, pyrrolyl, oxazolyl, thiazolyl, imidazalyl, oxadiazolyl, thiadiazolyl, triazolyl, triazinyl, pyridazinyl, pyridinyl, pyrimidinyl, isothiazolyl, isoxazolyl, pyrazinyl or pyrazolyl, wherein the phenyl or heterocyclyl group is substituted by $R^1$; or $Ar^2$ represents an optionally substituted bicyclic aromatic or bicyclic heteroaromatic group containing up to 3 heteroatoms selected from N, O and S;
$R^1$ represents hydrogen, optionally substituted($C_{1-4}$)alkoxy, halo, cyano, optionally substituted($C_{1-6}$)alkyl, optionally substituted phenyl, or an optionally substituted 5- or 6-membered heterocyclyl group containing up to 4 heteroatoms selected from N, O and S;

wherein the optional substituents for Het, $Ar^2$ and $R^1$ are selected from the group consisting of halogen, hydroxy, oxo, cyano, nitro, ($C_{1-4}$)alkyl, ($C_{1-4}$)alkoxy, hydroxy($C_{1-4}$)alkyl, hydroxy($C_{1-4}$)alkoxy, halo($C_{1-4}$)alkyl, halo($C_{1-4}$) alkoxy, aryl($C_{1-4}$)alkoxy, ($C_{1-}$ 4)alkylthio, hydroxy($C_{1-4}$)alkyl, ($C_{1-4}$)alkoxy($C_{1-4}$)alkyl, ($C_{3-6}$)cyckoalkyl($C_{1-4}$)alkoxy, ($C_{1-4}$)alkanoyl, ($C_{1-4}$)alkoxycarbonyl, ($C_{1-4}$)alkylsulfonyl, ($C_{1-4}$)alkylsuffonyloxy, ($C_{1-4}$)alkylsnlfonyl($C_{1-4}$)alkyl, arylsulfonyl, arylsulfonyloxy, arylsulfonyl($C_{1-4}$)alkyl, ($C_{1-4}$)alkylsulfonamido, ($C_{1-4}$alkylamido, ($C_{1-4}$)alkylsulfonamido ($C_{1-4}$)alkyl, ($C_{1-4}$)alkyhunido($C_{1-4}$)alkyl, arylsulfonamido, arylcarboxamido, arylsulfonamido($C_{1-4}$)alkyl, arylcarboxamido($C_{1-4}$)alkyl, aroyl, aroyl($C_{1-4}$)alkyl, aryl($C_{1-4}$)alkanoyl; ($C_{1-4}$)acyl; or a group $R^aR^bN$-, $R^aOCC)(CH_2)_r$, $R^aCON(R^a)(CH_2)_r$, $R^aR^bNCO(CH_2)_r$, $R^aR^bNSO_2(CH_2)_r$ or $R^aSO_2NR^b(CH_2)_r$ where each of $R^a$ and $R^b$ independently represents a hydrogen atom or a ($C_{1-4}$)alkyl group, or $R^aR^b$ forms part of a ($C_{3-6}$)azacyloalkane or ($C_{3-6}$)(2-oxo)azacycloalkane ring, and r represents zero or an integer from 1 to 4; or $R^aR^bN(CH_2)n$ or $R^aR^bN(CH_2)nO$-, wherein n represents an integer from 1 to 4 and where $R^a$ with at least one $CH_2$ of the $(CH_2)n$ portion of the group form a

$(C_{3-6})$azacycloalkane and $R^b$ represents hydrogen, a $(C_{1-4})$alkyl group or with the nitrogen to which it is attached forms a second $(C_{3-6})$azacycloalkane fused to the first $(C_{3-6})$azacycloalkane;
or a pharmaceutically acceptable salt thereof.

**2.** A compound according to claim 1 wherein $Ar^2$ represents optionally substituted phenyl, pyridinyl, thiazolyl, pyrazolyl, pyridazinyl, thienyl, naphthyl, triazolyl isoxazolyl, quinolinyl, or isoquinolinyl.

**3.** A compound according to claim 1 or 2 wherein $R^1$ represents optionally substituted phenyl, pyridinyl, pyrazolyl pyrimidinyl or oxadiazolyl group.

**4.** A compound according to any one of claims 1 to 3 wherein $Ar^2$ is optionally substituted by $C_{1-4})$alkyl, hydroxy$(C_{1-4})$alkyl, $R^aR^bN$, $(C_{1-4})$alkoxy, $R^aR^bN(CH_2)n$, $(C_{1-4})$acyl, and $(C_{1-4})$alkylamido.

**5.** A compound according to any one of claims 1 to 4 wherein Het is benzimidazolyl, benzofuranyl or benzoxazolyl.

**6.** A compound according to any one of claims 1 to 5 wherein Y represents $CH_2$.

**7.** A compound of formula (I) selected from the group consisting of:

| | |
|---|---|
| 1 | (RS)-2-(2-Benzofuranylmethyl)-1-((5-(4-fluorophenyl)-2-methyl-thiazol-4-yl)-carbonyl)-piperidine |
| 2 | (RS)-1-(2-Benzooxazol-2-ylmethyl-piperidin-1-yl)-1-[5-(4-fluoro-phenyl)-2-methyl-thiazol-4-yl]-methanone |
| 3 | (RS)-1-(2-Benzooxazol-2-ylmethyl-piperidin-1-yl)-1-[2-(3-methyl-[1,2,4]oxadiazol-5-yl)-phenyl]-methanone |

| | |
|---|---|
| 4 | (RS)-1-(2-Benzofuran-2-ylmethyl-piperidin-1-yl)-1-[4-(4-fluoro-phenyl)-1-methyl-1-*H*-pyrazol-3-yl]-methane |
| 5 | (RS)-1-[2-(5-Fluoro-benzofuran-2-ylmethyl)-piperidin-1-yl]-1-[5-(4-fluoro-phenyl)-2-methyl-thiazol-4-yl]-methanone |
| 6 | (RS)-1-[2-(5-Fluoro-benzofuran-2-ylmethyl)-piperidin-1-yl]-1-(5-pyridin-2-yl-thiazol-4-yl)-methanone |
| 7 | (RS)-1-[2-(5-Fluoro-benzofuran-2-ylmethyl)-piperidin-1-yl]-1-quinolin4-yl-methanone |
| 8 | (RS)-1-[2-(5-Fluoro-benzofuran-2-ylmethyl)-piperidin-1-yl]-1-quinolin-5-yl-methanone |
| 9 | (RS)-1-[2-(5-Fluoro-benzofuran-2-ylmethyl)-piperidin-1-yl]-1-(2-methoxy-pyridin-3-yl)-methanone |
| 10 | (RS)-1-[2-Dimethylamino-5-(4-fluoro-phenyl)-thiazol-4-yl]-1-[2-(5-fluoro-benzofuran-2-ylmethyl)-piperidin-1-yl]-methanone |
| 11 | (RS)-1-[2-(5-Fluoro-benzofuran-2-ylmethyl)-piperidin-1-yl]-1-(2-morpholin-4-yl-phenyl)-methanone |
| 12 | (RS)-1-[2-(5-Fluoro-benzofiran-2-ylmethyl)-piperidin-1-yl]-1-(2-trifluoromethoxy-phenyl)-methanone |
| 13 | (RS)-1-(2-Benzofuran-2-ylmethyl-piperidin-1-yl)-1-[2-(2-dimethylamino-ethoxy)-phenyl]-methanone |
| 14 | (RS)-1-(2-Benzofuran-2-ylmethyl-piperidin-1-yl)-1-quinolin-8-yl-methanone |
| 15 | (RS)-1-(2-Benzofuran-2-ylmethyt-piperidin-1-yl)-1-(2-pyrimidin-2-yl-phenyl)-methanone |
| 16 | (RS)-1-(2-Benzofuran-2-ylmethyl-piperidin-1-yl)-1-[5-(4-fluoro-phenyl)-2H-[1,2,3]triazol-4-yl]-methanone |
| 17 | (RS)-1-(2-Benzofuran-2-ylmethyl-piperidin-1-yl)-1-[4-(4-fluoro-phenyl)-1H-pyrazol-3-yl]-methanone |
| 18 | (RS)-1-(2-Benzofuran-2-ylmethyl-piperidin-1-yl)-1-[5-(4-fluoro-phenyl)-2-hydroxymethyl-thiazol-4-yl]-methanone |
| 19 | (RS)-1-(2-Benzofuran-2-ylmethyl-piperidin-1-yl)-1-isoquinolin-8-yl-methanone |
| 20 | (RS)-1-(2-Benzofuran-2-ylmethyl-piperidin-1-yl)-1-isoquinolin-5-yl-methanone |
| 21 | (RS)-1 -(2-Benzofuran-2-ylmethyl-piperidin-1 -yl)-1-{5-[3-(3-dimethylamino-propoxy)-phenyl]-2-methyl-thiazol-4-yl} -methanone |

Table continued

| | |
|---|---|
| 22 | (RS)-1-(2-Benzofuran-2-ylmethyl-piperidin-1-yl)-1-{5-[3-(4-dimethylamino-butoxy)-phenyl]-2-methyl-thiazol-4-yl}-methanone |
| 23 | (RS)-1-[5-( 4-Fluoro-phenyl)-2-methyl-thiazol-4-yl]-1-(2-furo[2,3-*b*]pyridin-2-ylmethy 1-piperidin-1-yl)-methanone |
| 24 | (RS)-1-[4-(4-Fluoro-phenyl)-1-methyl-1*H*-pyrazole-3-yl]-(2-furo[2,3-b]pyridin-2-ylmethyl-piperidin-1-yl) methanone |
| 25 | (RS)-1-[5-(4-Fluoro-phenyl)-2-methyl-thiazol-4-yl]-1-(2-quinolin-2-ylmethyl-piperidin-1-yl)-methanone |
| 26 | (RS)-1-Benzofuran-2-yl-1-(1-{1-[5-(4-fluoro-phenyl)-2-methyl-thiazol-4-yl]-methanoyl}-piperidin-2-yl)-methanone |
| 27 | (RS)-1-[2-(1H-benzoimidazol-2-ylmethyl)-piperidin-1-yl]-1-[5-(4-fluoro-phenyl)-2-methyl-thiazol-4-yl]-methanone |
| 28 | (RS)-1-[2-(5-Chloro-1H-benzoimidazol-2-ylmethyl)-piperidin-1-yl]-1-[5-(4-fluoro-phenyl)-2-methyl-thiazol-4-yl]-methanone |
| 29 | (RS)-1-[2-(5-Fluoro-1H-benzoimidazol-2-ylmethyl)-piperidin-1-yl}-1 -[5-(4-fluoro-phenyl)-2-methyl-thiazol-4-yl]-methanone |
| 30 | (RS)-1 -[2-(5-Chloro-6-fluoro-1H-benzoimidazol-2-ylmethyl)-piperidin-1 -yl]-1 -[5-(4-fluoro-phenyl)-2-methyl-thiazol-4-yl]-methanone |
| 31 | (RS)-1-[5-(4-Fluoro-phenyl)-2-methyl-thiazol-4-yl]-1-[2-(4-methyl-1H-benzoimidazol-2-ylmethyl)-piperidin-1-yl]-methanone |
| 32 | (RS)-1-[2-(4,6-Difluoro-1H-benzoimidazol-2-ylmethyl)-piperidin-1-yl]-1-[5-(4-fluoro-phenyl)-2-methyl-thiazol-4-yl]-methanone |
| 33 | (RS)-1-[2-(4-Dimethylaminomethyl-1H-benzoimidazol-2-ylmethyl)-piperidin-1-yl]-1[5-(4-fluoro-phenyl)-2-methyl-thiazol-4-yl]-methanone |
| 34 | (RS)-1-[2-(5-Bromo-1H-benzoimidazol-2-ylmethyl)-piperidin-1-yl]-1-[5-(4-fluoro-phenyl)-2-methyl-thiazol-4-yl]-methanone |
| 35 | (RS)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone |
| 36 | (RS)-1-[2-(4,5-Difluoro-1*H*-benzoimidazol-2-ylmethyl)-piperidin-1-yl]-1-[5-(4-fluoro-phenyl)-2-methyl-thiazol-4-yl]-methanone |
| 37 | (RS)-1-[2-(5 6-Difluoro-1N-benzoimidazol-2-ylmethyl)-piperidin-1-yl]-1-[5-(4-fluoro-phenyl)-2-hydroxymethyl-thiazol-4-yl]-methanone |
| 38 | (RS)-1-[2-(4,5-Difluoro-1*H*-benzoimidazol-2-ylmethyl)-piperidin-1-yl]-1-[5-(4-fluoro-phenyl)-2-hydroxymethyl-thiazol-4-yl]-methanone |
| 39 | (RS)-1-[2-(5,6-Difluoro-1*H*-benzoimidazol-2-ylmethyl)-piperidin-1-yl]-1-[4-(4-fluoro-phenyl)-1-methyl-1H-pyrazol-3-yl]-methanone |
| 40 | (RS)-1-[2-(5-Methoxy-1*H*-benzoimidazol-2-ylmethyl)-piperidin-1-yl]-1-[5-(4-fluoro-phenyl)-2-methyl-thiazol-4-yl]-methanone |
| 41 | (RS)-1-[2-(6,7-Difluoro-1H-benzoimidazol-2-ylmethyl)-piperidin-1-yl]-1-(2-methyl-5-phenyl-thiazol-4-yl)-methanone |
| 42 | (RS)-1-[2-(6,7-Difluoro-1H-benzoimidazol-2-ylmethyl)-piperidin-1-yl]-1-[4-(4-fluoro-phenyl)-1-methyl-1H-pyrazol-3-yl]-methanone |
| 43 | (RS)-1-[2-(6,7-Difluoro-1H-benzoimidazol-2-ylmethyl)-piperidin-1-yl]-1-[4-(4-fluoro-phenyl)-1H-pyrazol-3-yl]-methanone |
| 44 | (RS)-1-[2-(4,5-Difluoro-1H-benzoimidazol-2-ylmethyl)-piperidin-1-yl]-1-{5-[3-(2-dimethylamino-ethoxy)-phenyl]-2-methyl-thiazol-4-yl}-methanone |

Table continued

| | |
|---|---|
| 45 | (RS)-1-[2-(4,5-Difluoro-1H-benzoimidazol-2-ylmethyl)-piperidin-1-yl]-1-[5-(2-fluoro-phenyl)-2-methyl-thiazol-4-yl]-methanone |
| 46 | (RS)-1-[2-(4,5-Difluoro-1H-benzoimidazol-2-ylmethyl)-piperidin-1-yl]-1-[5-(3-fluoro-phenyl)-2-methyl-thiazol-4-yl]-methanone |
| 47 | (RS)-1-[2-(4,5-Difluoro-1H-benzoimidazol-2-ylmethyl)-piperidin-1-yl]-1-(2-trifluoromethoxy-phenyl)-methanone |
| 48 | (RS)-1-[2-(4,5-Difluoro-1H-benzoimidazol-2-ylmethyl)-piperidin-1-yl]-1-(2-ethoxy-phenyl)-methanone |
| 49 | (RS)-1-[2-(4,5-Difluoro-1H-benzoimidazol-2-ylmethyl)-piperidin-1-yl]-1-(2,6-dimethoxy-phenyl)-methanone |
| 50 | (RS)-1-[2-(6,7-Difluoro-1H-benzoimidazol-2-ylmethyl)-piperidin-1-yl]-1-(2-pyrazol-1-yl-phenyl)-methanone |
| 51 | (RS)-1-[2-(5,6-Difluoro-1H-benzoimidazol-2-ylmethyl)-piperidin-1-yl]-1-[4-(4-fluoro-phenyl)-1H-pyrazol-3-yl]-methanone |
| 52 | (RS)-1-[2-(5,6-Difluoro-1H-benzoimidazol-2-ylmethyl)-piperidin-1-yl]-1-[2-(3-methyl-[1,2,4]oxadiazol-5-yl)-phenyl]-methanone |
| 53 | (RS)-1-[2-(Difluoro-1H-benzoimidazol-2-ylmethyl)-piperidin-1-yl]-1-[1-(2-dimethylamino-ethyl)-4-(4-fluoro-phenyl)-1H-pyrazol-3-yl]-methanone |
| 54 | (RS)-1-[2-(5,6-Difluoro-1H-benzoimidazol-2-ylmethyl)-piperidin-1-yl]-1-[5-(4-fluoro-phenyl)-2-methyl-2H-[1,2,3]triazol-4-yl]-methanone |
| 55 | (RS)-1-[2-(5,6-Difluoro-1H-benzoimidazol-2-ylmethyl)-piperidin-1-yl]-1-{2-[3-(3-dimethylamino-propoxy)-phenyl]-thiophen-3-yl}-methanone |
| 56 | (RS)-1-[2-(5,6-Difluoro-1-methyl-1*H*-benzoimidazol-2-ylmethyl)-piperidin-1-yl]-1-[5-(4-fluoro-phenyl)-2-methyl-thiazol-4-yl]-methanone |
| 57 | (RS)-1-(2-Benzothiazol-2-ylmethylpiperidin-1-yl)-1-[5-(4-fluorophenyl)-2-methylthiazol-4-yl]-methanone |
| 58 | (RS)-1-(2-Benzothiazol-2-ylmethylpiperidin-1-yl)-1-[4-(4-fluorophenyl)-1-methyl-1-*H*-pyrazol-3-yl]-methanone |
| 59 | (RS)-1-(2-Benzothiazol-2-ylmethylpiperidin-1-yl)-1-[2-(3-methyl-[1,2,4]oxadiazol-5-yl)phenyl]-methanone |
| 60 | (RS)-1-[5-(4-Fluorophenyl)-2-methylthiazol-4-yl]-1-[2-(5,6,7,8-tetrahydro-[1,2,4]-triazolo[1,5-a]pyridin-2-ylmethyl)-piperidin-1-yl]-methanone |
| 61 | (RS)-1-[5-(4-Fluorophenyl)-2-methylthiazol-4-yl]-1-(2-[1,2,4]triazolo[1,5-a]pyridin-2-ylmethylpiperidin-1-yl)-methanone |
| 62 | 1-[(RS)-2-((RS)-2-Benzofuran-2-yl-2-hydroxy-ethyl)-piperidin-1-yl]-1-[5-(4-nuoro-phenyl)-2-methyl-thiazol-4-yl]-methanone (as separate diastereoisomers) |
| 63 | (RS)-1-[2-(4-Bromo-1*H*-benzoimidazol-2-ylmethyl)-piperidin-1-yl]-1-[5-(4-fluorophenyl)-2-methylthiazol-4-yl] methanone |
| 64 | (RS)-1-[2-(4-Cyano-1*H*-benzoimidazol-2-ylmethyl)-piperidin-1-yl]-1-[5-(4-fluorophenyl)-2-methylthiazol-4-yl] methanone |
| 65 | (RS)-1-[2-(4-Acetyl-1H-benzoimidazol-2-ylmethyl)-piperidin-1-yl]-1-[5-(4-fluorophenyl)-2-methylthiazol-4-yl] methanone |
| 66 | (RS)-2-(1-{1-[5-(4-Fluoro-phenyl)-2-methyl-thiazol-4-yl]-methanoyl}-piperidin-2-ylmethyl)-1 H-benzoimidazole-5-carbonitrile |
| 67 | (RS)-1-[2-(5,6-Difluoro-1-propyl-1H-benzoimidazol-2-ylmethyl)-piperidin-1-yl]-1-[5-(4-fluoro-phenyl)-2-methyl-thiazol-4-yl]-methanone |
| 68 | (RS)-1-{2-[5,6-Difluoro-1-(2-methoxy-ethyl)-1*H*-benzoimidazol-2-ylmethyl]-piperidin-1-yl}-1-[5-(4-fluoro-phenyl)-2-methyl-thiazol-4-yl]-methanone |
| 69 | (RS)-1-{2-[1-(2-Dimethylamino-ethyl)-5,6-difluoro-1*H*-benzoimidazol-2-ylmethyl]-piperidin-1-yl}-1-[5-(4-fluoro-phenyl)-2-methyl-thiazol-4-yl]-methanone |

Table continued

| | |
|---|---|
| 70 | (RS)-1-{2-[5,6-difluoro-1-(2-hydroxy-ethyl)-1*H*-benzoimidazol-2-ylmethyl]-piperidin-1-yl}-1-[5-(4-fluoro-phenyl)-2-methyl-thiazol-4-yl]-methanone |
| 71 | (RS)-1-[2-(6,7-Difluoro-1-methyl-1H-benzoimidazol-2-ylmethyl)-piperidin-1-yl]-1-[5-(4-fluorophenyl)-2-methyl-thiazol-4-yl]-methanone |
| 72 | (RS)-1-[2-(4,5-Difluoro-1-methyl-1H-benzoimidazol-2-ylmethyl)-piperidin-1-yl]-1-[5-(4-fluorophenyl)-2-methyl-thiazol-4-yl]-methanone |
| 73 | (RS)-2-(1-{1-[5-(4-Fluorophenyl)-2-methyl-thiazol-4-yl]-methanoyl}-piperidin-2-ylmethyl)-benzofuran-3-carboxylic acid amide |
| 74 | (RS)-2-(1-{1-[4-(4-Fluorophenyl)-1-methyl-1*H*-pyrazol-3-yl]-methanoyl}-piperidin-2-ylmethyl)-benzofuran-3-carboxylic acid amide |
| 75 | (RS)-1-[5-(4-Fluoro-phenyl)-2-methyl-thiazol-4-yl]-1-(2-furo[3,2-b]-pyridin-2-ylmethyl-piperidin-1-yl) methanone |
| 76 | (RS)-1-[4-(4-Fluoro-phenyl)-1-methyl-1H-pyrazole-3-yl)-(2-furo[3,2-b]pyridin-2-ylmethyl-piperidin-1-yl) methanone |
| 77 | (RS)-1-[2-(3-Bromo-benzofuran-2-ylmethyl)-piperidin-1-yl]-1-[5-(4-fluoro-phenyl)-2-methyl-thiazol-4-yl]-methanone |
| 78 | (RS)-2-(1-{1-[5-(4-Fluoro-phenyl)-2-methyl-thiazol4-yl]-methanoyl}-piperidin-2-ylmethyl)-benzofuran-3-carbonitrile |
| 79 | (RS)-1-[2-(1-{1-[5-(4-fluoro-phenyl)-2-methyl-thiazol-4-yl]-methanoyl}-piperidin-2-ylmethyl)-benzofuran-3-yl]-ethanone |
| 80 | (R)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanoneand(S)-1-[2-(5,6-Difluoro-1*H*-benzoimidazol-2-ylmethyl)-piperidin-1-yl]-1-[5-(4-fluoro-phenyl)-2-methyl-thiazol-4-yl]-methanone |

| | |
|---|---|
| 81 | (RS)-1-(2-Benzofuran-2-ylmethyl-piperidin-1-yl)-1-[5-(4-methoxy-phenyl)-2-methyl-thiazol-4-yl]-methanone |
| 82 | (RS)-1-(2-Benzofuran-2-ylmethyl-piperidin-1-yl)-1-{5-[3-(2-dimethylamino-ethoxy)-phenyl]-2-methyl-thiazol-4-yl}-methanone |
| 83 | (RS)-1-[2-(4,5-Difluoro-1H-benzoimidazol-2-ylmethyl)-piperidin-1-yl]-1-(5-methyl-3-phenyl-isoxazol-4-yl)-methanone |
| 84 | 1-[3-(2,6-Dichloro-phenyl)-5-methyl-isoxazol-4-yl]-1-[2-(4,5-difluoro-1H-benzoimidazol-2-ylmethyl)-piperidin-1-yl]-methanone |
| 85 | (RS)-1-[2-(4,5-Difluoro-1H-benzoimidazol-2-ylmethyl)-piperidin-1-yl]-1-[4-(4-fluoro-phenyl)-2-methyl-2H-pyrazol-3-yl]-methanone |
| 86 | (RS)-1-[3-(2-Chloro-6-fluoro-phenyl)-5-methyl-isoxazol-4-yl]-1-[2-(4,5-difluoro-1H-benzoimidazol-2-ylmethyl)-piperidin-1-yl]-methanone |
| 87 | (RS)-1-[3-(2-Chloro-phenyl)-5-methyl-isoxazol-4-yl]-1-[2-(4,5-difluoro-1H. benzoimidazol-2-ylmethyl)-piperidin-1-yl]-methanone |
| 88 | (RS)-1-[2-(4,5-Difluoro-1H-benzoimidazol-2-ylmethyl)piperidin-1-yl]-1-(2-propoxy-phenyl)-methanone |
| 89 | (RS)-1-[2-(4,4-Difluoro-1H-benzoimidazol-2-ylmethyl)-piperidin-1-yl]-1-(2-isopropoxy-phenyl)-methanone |
| 90 | (RS)-1-(2-Benzyloxy-phenyl)-1-[2-(4,S-difluoro-1H-benzoimidazol-2-ylmethyl)-piperidin-1-yl]-methanone |
| 91 | (RS)-1-[2-(4,S-Difluoro-1H-benzoimidazol-2-ylmethyl)-piperidin-1-yl]-1-(2-ethoxy-6-methoxy-phenyl)-methanone |
| 92 | (RS)-1-[2-(4,5-Difluoro-1H-benzoimidazol-2-ylmethyl)-piperidin-1 -yl]-1-(2-ethoxy-6-methyl-phenyl)-methanone |

Table continued

| | |
|---|---|
| 93 | (RS)-1-(2,6-Diethoxy-phenyl)-1-[2-(4,5-difluoro-1H-benzoimidazol-2-ylmethyl)-piperidin-1-yl]-methanone |
| 94 | 1-(3-{ 1-[2-(4,5-Difluoro-1H-benzoimidazol-2-ylmethyl)-piperidin-1-yl]-methanoyl}-4-ethoxy-phenyl)-ethanone |
| 95 | (RS)-1-[2-(4,5-Difluoro-1H-benzoimidazol-2-ylmethyl)-piperidin-1-yl]-1-(2-ethoxy-naphthalen-1-yl)-methanone |
| 96 | (RS)-1-[2-(4,S-Difluoro-1H-benzoimidazol-2-ylmethyl)-piperidin-1-yl]-1-(2-pyridin-2-yl-phenyl)-methanone |
| 97 | 1-[2-(6,7-Difluoro-1H-benzoimidazol-2-ylmethyl)-piperidin-1-yl]-1-(2-pyrrol-1-yl-phenyl)-methanone |
| 98 | (RS)-1-[2-(4,5-Difluoro-1H-benzoimidazol-2-ylmethyl)-piperidin-1-yl]-1-{5-[3-(3-dimethylamino-propoxy)-phenyl]-2-methyl-thiazol-4-yl}-methanone |
| 99 | (RS)-1-[2-(4,5-Difluoro-1H-benzoimidazol-2-ylmethyl)-piperidin-1-yl]-1-{5-[3-(4-dimethylamino-butoxy)-phenyl]-2-methyl-thiazol-4-yl}-methanone |
| 100 | (RS)-1-[2-(5,6-Difluoro-1H-benzoimidazol-2-ylmethyl)-piperidin-1-yl]-1-[5-(4-fluoro-phenyl)-2-methoxy-thiazol-4-yl]-methanone |
| 101 | (RS)-1-[2-(5,6-Difluoro-1H-benzoimidazol-2-ylmethyl)-piperidin-1-yl]-1-[2-ethyl-5-(4-fluoro-phenyl)-thiazol-4-yl]-methanone |
| 102 | (RS)-1-[2-(5,6-Difluoro-1H-benzoimidazol-2-ylmethyl)-piperidin-1-yl]-1-[5-(2-fluoro-phenyl)-2-methyl-thiazol-4-yl]-methanone |
| 103 | (RS)-1-[2-(5,6-Difluoro-1H-benzoimidazol-2-ylmethyl)-piperidin-1-yl]-1-[5-(3-fluoro-phenyl)-2-methyl-thiazol-4-yl]-methanone |
| 104 | (RS)-1-[2-(4-Fluoro-1H-benzoimidazol-2-ylmethyl)-piperidin-1-yl]-1-[5-(4-fluoro-phenyl)-2-methyl-thiazol-4-yl]-methanone |
| 105 | (RS)-1-[2-(4-Fluoro-1H-benzoimidazol-2-ylmethyl)-piperidin-1-yl]-1-[4-(4-fluoro-phenyl)-1-methyl-1H-pyrazol-3-yl]-methanone |
| 106 | (RS)-1-[2-(4-Fluoro-1H-benzoimidazol-2-ylmethyl)-piperidin-1-yl]-1-[4-(4-fluoro-phenyl)-1H-pyrazol-3-yl]-methanone |
| 107 | (RS)-1-[2-(4-Fluoro-1H-benzoimidazol-2-ylmethyl)-piperidin-1-yl]-1-[4-(4-fluoro-phenyl)-2-methyl-2H-pyrazol-3-yl]-methanone |
| 108 | (RS)-1-(2-Ethoxy-phenyl)-1-[2-(4-fluoro-1H-benzoimidazol-2-ylmethyl)-piperidin-1-yl]-methanone |
| 109 | (RS)-1-[2-(4-Fluoro-1H-benzoimidazol-2-ylmethyl)-piperidin-1-yl]-1-[5-(4-fluoro-phenyl)-2-hydroxymethyl-thiazol-4-yl]-methanone |
| 110 | (RS)-1-[5-(4-Fluoro-phenyl)-2-methyl-thiazol-4-yl]-1-{2-[4-(1-hydroxy-ethyl)-1*H* benzoimidazol-2-ylmethyl]-piperidin-1-yl}-methanone |
| 111 | (RS)- 1-(2-Benzofuran-2-ylmethyl-piperidin-1-yl)-1-[5-(4-chloro-phenyl)-2-methyl-thiazol-4-yl]-methanone |
| 112 | (RS)-1-[2-(3-Chloro-furo[3,2-b]pyridin-2-ylmethyl)-piperidin-1-yl]-1-[5-(4-fluoro-phenyl)-2-methyl-thiazol-4-yl]-methanone |
| 113 | (RS)-1-[2-(5,6-Ditluoro-1-methyl-1H-benzoimidazol-2-ylmethyl)-piperidin-1-yl]-1-[5-(4-fluoro-phenyl)-2-hydroxymethyl-thiazol-4-yl]-methanone |
| 114 | (RS)-1-[5-(4-Fluoro-phenyl)-2-methyl-thiazol-4-yl]-1-[2-(1H-indol-2-ylmethyl)-piperidin-1-yl]-methanone |
| 115 | (RS)-5-[1-(2-Benzofuran-2-ylmethyl-piperidin-1-yl)-methanoyl]-4H-benzo[1,4]oxazin-3-one |
| 116 | (RS)-1-[2-(5-Bromo-benzofuran-2-ylmethyl)-piperidin-1-yl]-1-[5-(4-fluoro-phenyl)-2-methyl-thiazol-4-yl]-methanone |
| 117 | (RS)-1-[2-(5-Cyano-benzofuran-2-ylmethyl)-piperidin-1-yl]-1-[5-(4-fluoro-phenyl)-2-methyl-thiazol-4-yl]-methanone |

Table continued

| | |
|---|---|
| 118 | (RS)-1-[2-(4-Bromo-benzofuran-2-ylmethyl)-piperidin-1-yl]-1-[5-(4-fluoro-phenyl)-2-methyl-thiazol-4-yl]-methanone |
| 119 | (RS)-1-[2-(4-Cyano-benzofuran-2-ylmethyl)-piperidin-1-yl]-1-[5-(4-fluoro-phenyl)-2-methyl-thiazol-4-yl]-methanone |
| 120 | (RS)-1-[5-(4-Fluoro-phenyl)-2-methyl-thiazol-4-yl]-1-[2-(3-methyl-benzofuran-2-ylmethyl)-piperidin-1-yl]-methanone |
| 121 | (RS)-1-[2-(4-Fluoro-benzofuran-2-ylmethyl)-piperidin-1-yl]-1-[5-(4-fluoro-phenyl)-2-methyl-thiazol-4-yl]-methanone |
| 122 | (RS)-1-[2-(4-Fluoro-benzofuran-2-ylmethyl)-piperidin-1-yl]-1-[4-(4-fluoro-phenyl)-1-methyl-1 H- pyrazol-3 -yl]-methanone |
| 123 | (RS)- 1-(2-Benzo[b] thiophen-2-ylmethyl-piperidin-1-yl)-1-[5-(4-fluoro-phenyl)-2-methyl-thiazol-4-yl]-methanone |
| 124 | (RS)-1-(2-Benzo[b]thiophen-2-ylmethyl-piperidin-1-yl)-1-[4-(4-fluoro-phenyl)-1-methyl-1H-pyrazol-3-yl]-methanone |
| 125 | (RS)-1-(2-Benzo[b]thiophen-2-ylmethyl-piperidin-1-yl)-1-quinolin-8-yl-methanone |
| 126 | (RS)-1-(2-Benzofuran-2-ylmethyl-piperidin-1-yl)- 1-(2-methyl-5-phenyl-thiazol-4-yl)-methanone |
| 127 | (RS)-1-(2-Benzofuran-2-ylmethyl-piperidin-1-yl)-1-[2-(3-methyl-[1,2,4]oxadiazol-5-yl)-phenyl]-methanone |
| 128 | (R)-1-[2-(4,6-Difluoro-1*H*-benzoimidazol-2-ylmethyl)-piperidin-1-yl]-1-[5-(4-fluoro-phenyl)-2-methyl-thiazol-4-yl]-methanone and (S)-1-[2-(4,6-Difluoro-1 *H*-benzoimidazol-2-ylmethyl)-piperidin-1-yl]-1-[5-(4-fluoro-phenyl)-2-methyl-thiazol-4-yl]-methanone |

or a pharmaceutically acceptable salt of any one thereof.

**8.** A pharmaceutical composition comprising a compound of formula (1) as defined in any one of claims 1 to 7, or a pharmaceutically acceptable salt thereof, and a pharmaceutically acceptable carrier.

**9.** Use of a compound of any one of claims 1 to 7 for the manufacture of a medicament for the treatment or prevention of obesity, diabetes, sleep disorders, insomnia, parasomnia or stroke.

**Patentansprüche**

**1.** Verbindung der Formel (1):

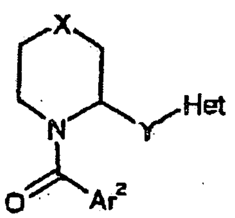

wobei
X für $CH_2$ steht;
Y für $CH_2$, CO, CH(OH) oder $CH_2CH(OH)$ steht;
Het ein gegebenenfalls substituierter bicyclischer Heteroarylrest, ausgewählt aus Chinoxalinyl, Chinazolinyl, Pyridopyrazinyl, Benzoxazolyl, Benzothienyl, Benzofuranyl, Benzimidazolyl, Naphthyridinyl, Benzothiazolyl, Indolyl, Triazolopyridinyl, Furopyridinyl, Pyridopyrimidinyl, Isochinolinyl, Chinolinyl, Oxazolylpyridinyl, Tetrahydrobenzimidazolyl, Tetrahydrobenzofuranyl oder Tetrahydrotriazolopyridinyl, ist; $Ar^2$ einen gegebenenfalls substituierten Phe-

nyl- oder einen 5- oder 6-gliedrigen Heterocyclylrest, ausgewählt aus Furanyl, Thienyl, Pyrrolyl, Oxazolyl, Thiazolyl, Imidazolyl, Oxadiazolyl, Thiadiazolyl, Triazolyl, Triazinyl, Pyridazinyl, Pyridinyl, Pyrimidinyl, Isothiazolyl, Isoxazolyl, Pyrazinyl oder Pyrazolyl, bedeutet, wobei der Phenyl- oder Heterocyclylrest mit $R^1$ substituiert ist; oder $Ar^2$ einen gegebenenfalls substituierten bicyclischen aromatischen oder bicyclischen heteroaromatischen Rest, der bis zu drei Heteroatome, ausgewählt aus N, O und S, enthält, bedeutet;

$R^1$ Wasserstoff, gegebenenfalls substituiertes ($C_{1-4}$)-Alkoxy, Halogen, Cyano, gegebenenfalls substituiertes ($C_{1-6}$)-Alkyl, gegebenenfalls substituiertes Phenyl, oder einen gegebenenfalls substituierten 5- oder 6-gliedrigen Heterocyclylrest, der bis zu 4 Heteroatome, ausgewählt aus N, O und S, enthält, bedeutet;

wobei die optionalen Substituenten für Het, $Ar^2$ und $R^1$ aus Halogen, Hydroxy, Oxo, Cyano, Nitro, ($C_{1-4}$)-Alkyl, ($C_{1-4}$)-Alkoxy, Hydroxy-($C_{1-4}$)-alkyl, Hydroxy-($C_{1-4}$)-alkoxy, Halogen-($C_{1-4}$)-alkyl, Halogen-($C_{1-4}$)-alkoxy, Aryl-($C_{1-4}$)-alkoxy, ($C_{1-4}$)-Alkylthio, Hydroxy-($C_{1-4}$)-alkyl, ($C_{1-4}$)-Alkoxy-($C_{1-4}$)-alkyl, ($C_{3-6}$)-Cycloalkyl-($C_{1-4}$)-alkoxy, ($C_{1-4}$)-Alkanoyl, ($C_{1-4}$)-Alkoxycarbonyl, ($C_{1-4}$)-Alkylsulfonyl, ($C_{1-4}$)-Alkylsulfonyloxy, ($C_{1-4}$)-Alkylsulfonyl-($C_{1-4}$)-alkyl, Arylsulfonyl, Arylsulfonyloxy, Arylsulfonyl-($C_{1-4}$)-alkyl, ($C_{1-4}$)-Alkylsulfonamido, ($C_{1-4}$)-Alkylamido, ($C_{1-4}$)-Alkylsulfonamido-($C_{1-4}$)-alkyl, ($C_{1-4}$)-Alkylamido-($C_{1-4}$)-alkyl, Arylsulfonamido, Arylcarboxamido, Arylsulfonamido-($C_{1-4}$)-alkyl, Arylcarboxamido-($C_{1-4}$)-alkyl, Aroyl, Aroyl-($C_{1-4}$)-alkyl, Aryl-($C_{1-4}$)-alkanoyl, ($C_{1-4}$)-Acyl oder einem Rest $R^aR^bN$-, $R^aOCO(CH_2)_r$, $R^aCON(R^a)(CH_2)_r$, $R^aR^bNCO(CH_2)_r$, $R^aR^bNSO_2(CH_2)_r$ oder $R^aSO_2NR^b(CH_2)_r$, wobei jeder der Reste $R^a$ und $R^b$ unabhängig ein Wasserstoffatom oder einen ($C_{1-4}$)-Alkylrest bedeutet oder $R^aR^b$ Teil eines ($C_{3-6}$)-Azacycloalkan- oder ($C_{3-6}$)-(2-Oxo)azacycloalkanrings bildet und r Null oder eine ganze Zahl von 1 bis 4 bedeutet; oder $R^aR^bN(CH_2)n$ oder $R^aR^bN(CH_2)nO$-, wobei n eine ganze Zahl von 1 bis 4 bedeutet und wobei $R^a$ mit mindestens einem $CH_2$ des $(CH_2)$n-Anteils des Restes ein ($C_{3-6}$)-Azacycloalkan bildet und $R^b$ Wasserstoff, einen ($C_{1-4}$)-Alkylrest bedeutet oder mit dem Stickstoff, an das es gebunden ist, ein zweites ($C_{3-6}$)-Azacycloalkan bildet, das an das erste ($C_{3-6}$)-Azacycloalkan kondensiert ist, ausgewählt sind;

oder ein pharmazeutisch verträgliches Salz davon.

**2.** Verbindung nach Anspruch 1, wobei $Ar^2$ gegebenenfalls substituiertes Phenyl, Pyridinyl, Thiazolyl, Pyrazolyl, Pyridazinyl, Thienyl, Naphthyl, Triazolyl, Isoxazolyl, Chinolinyl oder Isochinolinyl bedeutet.

**3.** Verbindung nach Anspruch 1 oder 2, wobei $R^1$ einen gegebenenfalls substituierten Phenyl-, Pyridinyl-, Pyrazolyl-, Pyrimidinyl- oder Oxadiazolylrest bedeutet.

**4.** Verbindung nach einem der Ansprüche 1 bis 3, wobei $Ar^2$ gegebenenfalls mit ($C_{1-4}$)-Alkyl, Hydroxy-($C_{1-4}$)-alkyl, $R^aR^bN$, ($C_{1-4}$)-Alkoxy, $R^aR^bN(CH_2)n$, ($C_{1-4}$)-Acyl und ($C_{1-4}$)-Alkylamido substituiert ist.

**5.** Verbindung nach einem der Ansprüche 1 bis 4, wobei Het Benzimidazolyl, Benzofuranyl oder Benzoxazolyl ist.

**6.** Verbindung nach einem der Ansprüche 1 bis 5, wobei Y für $CH_2$ steht.

**7.** Verbindung der Formel (I), ausgewählt aus:

| | |
|---|---|
| 1 | (RS)-2-(2-Benzofuranylmethyl)-1-((5-(4-fluorphenyl)-2-methyl-thiazol-4-yl)-carbonyl)-piperidin |
| 2 | (RS)-1 -(2-Benzooxazol-2-ylmethyl-piperidin-1 -yl)-1 -[5-(4-fluorphenyl)-2-methyl-thiazol-4-yl]-methanon |
| 3 | (RS)-1-(2-Benzooxazol-2-ylmethyl-piperidin-1 -yl)-1-[2-(3-methyl-[1,2,4]oxadiazol-5-yl)-phenyl]-methanon |
| 4 | (RS)-1 -(2-Benzofuran-2-ylmethyl-piperidin-1 -yl)-1-[4-(4-fluorphenyl)-1-methyl-1*H*-pyrazol-3-yl]-methan |
| 5 | (RS)-1-[2-(5-Fluor-benzofuran-2-ylmethyl)-piperidin-1-yl]-1-[5-(4-fluorphenyl)-2-methyl-thiazol-4-yl]-methanon |
| 6 | (RS)-1-[2-(5-Fluor-benzofuran-2-ylmethyl)-piperidin-1-yl]-1-(5-pyridin-2-yl-thiazol-4-yl)-methanon |
| 7 | (RS)-1-[2-(5-Fluor-benzofuran-2-ylmethyl)-piperidin-1-yl]-1-chinolin-4-yl-methanon |
| 8 | (RS)-1-[2-(5-Fluor-benzofuran-2-ylmethyl)-piperidin-1-yl]-1-chinolin-5-yl-methanon |
| 9 | (RS)-1-[2-(5-Fluor-benzofuran-2-ylmethyl)-piperidin-1-yl]-1-(2-methoxy-pyridin3-yl)-methanon |
| 10 | (RS)-1-[2-Dimethylamino-5-(4-fluorphenyl)-thiazol-4-yl]-1-[2-(5-fluorbenzofuran-2-ylmethyl)-piperidin-1-yl]-methanon |
| 11 | (RS)-1-[2-(5-Fluorbenzofuran-2-ylmethyl)-piperidin-1-yl]-1-(2-morpholin-4-yl-phenyl)-methanon |
| 12 | (RS)-1-[2-(5-Fluor-benzofuran-2-ylmethyl)-piperidin-1-yl]-1-(2-trifluormethoxyphenyl)-methanon |

Tabelle fortgesetzt

| | |
|---|---|
| 13 | (RS)-1-(2-Benzofuran-2-ylmethyl-piperidin-1-yl)-1-[2-(2-dimethylamino-ethoxy)-phenyl]-methanon |
| 14 | (RS)-1-(2-Benzofuran-2-ylmethyl-piperidin-1-yl)-1-chinolin-8-yl-methanon |
| 15 | (RS)-1-(2-Benzofuran-2-ylmethyl-piperidin-1-yl)-1-(2-pyrimidin-2-yl-phenyl)-methanon |
| 16 | (RS)-1-(2-Benzofuran-2-ylmethyl-piperidin-1-yl)-1-[5-(4-fluorphenyl)-2H-[1,2,3]-triazol-4-yl]-methanon |
| 17 | (RS)-1-(2-Benzofuran-2-ylmethyl-piperidin-1-yl)-1-[4-(4-fluorphenyl)-1*H*-pyrazol-3-yl]-methanon |
| 18 | (RS)-1-(2-Benzofuran-2-ylmethyl-piperidin-1-yl)-1-[5-(4-fluorphenyl)-2-hydroxymethyl-thiazol-4-yl]-methanon |
| 19 | (RS)-1-(2-Benzofuran-2-ylmethyl piperidin-1-yl)-1-isochinolin-8-yl-methanon |
| 20 | (RS)-1-(2-Benzofuran-2-ylmethyl-piperidin-1-yl)-1-isochinolin-5-yl-methanon |
| 21 | (RS)-1-(2-Benzofuran-2-ylmethyl-piperidin-1-yl)-1-{5-[3-(3-dimethylamino-propoxy)-phenyl]-2-methyl-thiazol-4-yl}-methanon |
| 22 | (RS)-1-(2-Benzofuran-2-ylmethyl-piperidin-1-yl)-1-{5-[3-(4-dimethylamino-butoxy)-phenyl]-2-methyl-thiazol-4-yl}-methanon |
| 23 | (RS)-1-[5-(4-Fluorphenyl)-2-methyl-thiazol-4-yl]-1-(2-furo[2,3-b]pyridin-2-ylmethyl-piperidin-1-yl)-methanon |
| 24 | (RS)-1-[4-(4-Fluorphenyl)-1-methyl-1*H*-pyrazol-3-yl]-(2-furo[2,3-b]pyridin-2-ylmethyl-piperidin-1-yl)-methanon |
| 25 | (RS)-1-[5-(4-Fluorphenyl)-2-methyl-thiazol-4-yl]-1-(2-chinolin-2-ylmethyl-piperidin-1-yl)-methanon |
| 26 | (RS)-1-Benzofuran-2-yl-1-(1-{1-[5-(4-fluorphenyl)-2-methyl-thiazol-4-yl]-methanoyl}-piperidin-2-yl)-methanon |
| 27 | (RS)-1-[2-(1*H*-Benzimidazol-2-ylmethyl)-piperidin-1-yl]-1-[5-(4-fluorphenyl)-2-methyl-thiazol-4-yl]-methanon |
| 28 | (RS)-1-[2-(5-Chlor-1*H*-benzimidazol-2-ylmethyl)-piperidin-1-yl]-1-[5-(4-fluorphenyl)-2-methyl-thiazol-4-yl]-methanon |
| 29 | (RS)-1-[2-(5-Fluor-1*H*-benzimidazol-2-ylmethyl)-piperidin-1-yl]-1-[5-(4-(fluorphenyl)-2-methyl-thiazol-4-yl]-methanon |
| 30 | (RS)-1-[2-(5-Chlor-6-fluor-1*H*-benzimidazol-2-ylmethyl)-piperidin-1-yl]-1-[5-(4-fluorphenyl)-2-methyl-thiazol-4-yl]-methanon |
| 31 | (RS)-1-[5-(4-Fluorphenyl)-2-methyl-thiazol-4-yl]-1-[2-(4-methyl-1*H*-benzimidazol-2-ylmethyl)-piperidin-1-yl]-methanon |
| 32 | (RS)-1-[2-(4,6-Difluor-1*H*-benzimidazol-2-ylmethyl)-piperidin-1-yl]-1-[5-(4-fluorphenyl)-2-methyl-thiazol-4-yl]-methanon |
| 33 | (RS)-1-[2-(4-Dimethylaminomethyl-1*H*-benzimidazol-2-ylmethyl)-piperidin-1-yl]-1-[5-(4-fluorphenyl)-2-methyl-thiazol-4-yl]-methanon |
| 34 | (RS)-1-[2-(5-Brom-1*H*-benzimidazol-2-ylmethyl)-piperidin-1-yl]-1-[5-(4-fluorphenyl)-2-methyl-thiazol-4-yl]-methanon |
| 35 | (RS)-1-[2-(5,6-Difluor-1*H*-benzimidazol-2-ylmethyl)-piperidin-1 -yl]-1-[5-(4-fluorphenyl)-2-methyl-thiazol-4-yl]-methanon |
| 36 | (RS)-1-[2-(4,5-Difluor-1*H*-benzimidazol-2-ylmethyl)-piperidin-1-yl]-1-[5-(4-fluorphenyl)-2-methyl-thiazol-4-yl]-methanon |
| 37 | (RS)-1-[2-(5,6-Difluor-1*H*-benzimidazol-2-ylmethyl)-piperidin-1-yl]-1-[5-(4-fluorphenyl)-2-hydroxymethyl-thiazol-4-yl]-methanon |
| 38 | (RS)-1-[2-(4,5-Difluor-1*H*-benzimidazol-2-ylmethyl)-piperidin-1-yl]-1-[5-(4-fluorphenyl)-2-hydroxymethyl-thiazol-4-yl]-methanon |
| 39 | (RS)-1-[2-(5,6-Difluor-1*H*-benzimidazol-2-ylmethyl)-piperidin-1-yl]-1-[4-(4-fluorphenyl)-1-methyl-1*H*-pyrazol-3-yl]-methanon |

Tabelle fortgesetzt

| | |
|---|---|
| 40 | (RS)-1-[2-(5-Methoxy-1*H*-benzimidazol-2-ylmethyl)-piperidin-1-yl]-1-[5-(4-fluorphenyl)-2-methyl-thiazol-4-yl]-methanon |
| 41 | (RS)-1-[2-(6,7-Difluor-1*H*-benzimidazol-2-ylmethyl)-piperidin-1-yl]-1-(2-methyl-5-phenyl-thiazol-4-yl)-methanon |
| 42 | (RS)-1-[2-(6,7-Difluor-1*H*-benzimidazol-2-ylmethyl)-piperidin-1-yl]-1-[4-(4-fluorphenyl)-1-methyl-1*H*-pyrazol-3-yl]-methanon |
| 43 | (RS)-1-[2-(6,7-Difluor-1*H*-benzimidazol-2-ylmethyl)-piperidin-1-yl]-1-[4-(4-fluorphenyl)-1*H*-pyrazol-3-yl]-methanon |
| 44 | (RS)-1-[2-(4,5-Difluor-1*H*-benzimidazol-2-ylmethyl)-piperidin-1-yl]-1-{5-[3-(2-dimethylamino-ethoxy)-phenyl]-2-methyl-thiazol-4-yl}-methanon |
| 45 | (RS)-1-[2-(4,5-Difluor-1*H*-benzimidazol-2-ylmethyl)-piperidin-1-yl]-1-[5-(2-fluorphenyl)-2-methyl-thiazol-4-yl]-methanon |
| 46 | (RS)-1-[2-(4,5-Difluor-1*H*-benzimidazol-2-ylmethyl)-piperidin-1-yl]-1-[5-(3-fluorphenyl)-2-methyl-thiazol-4-yl]-methanon |
| 47 | (RS)-1-[2-(4,5-Difluor-1*H*-benzimidazol-2-ylmethyl)-piperidin-1-yl]-1-(2-trifluormethoxyphenyl)-methanon |
| 48 | (RS)-1-[2-(4,5-Difluor-1*H*-benzimidazol-2-ylmethyl)-piperidin-1-yl]-1-(2-ethoxyphenyl)-methanon |
| 49 | (RS)-1-[2-(4,5-Difluor-1*H*-benzimidazol-2-ylmethyl)-piperidin-1-yl]-1-(2,6-dimethoxyphenyl)-methanon |
| 50 | (RS)-1-[2-(6,7-Difluor-1*H*-benzimidazol-2-ylmethyl)-piperidin-1-yl]-1-(2-pyrazol-1-ylphenyl)-methanon |
| 51 | (RS)-1-[2-(5,6-Difluor-1*H*-benzimidazol-2-ylmethyl)-piperidin-1-yl]-1-[4-(4-fluorphenyl)-1*H*-pyrazol-3-yl]-methanon |
| 52 | (RS)-1-[2-(5,6-Difluor-1*H*-benzimidazol-2-ylmethyl)-piperidin-1-yl]-1-[2-(3-methyl-[1,2,4]oxadiazol-5-yl)-phenyl]-methanon |
| 53 | (RS)-1-[2-(5,6-Difluor-1*H*-benzimidazol-2-ylmethyl)-piperidin-1-yl]-1-[-1-(2-dimethylamino-ethyl)-4-(4-fluorphenyl)-1*H*-pyrazol-3-yl]-methanon |
| 54 | (RS)-1-[2-(5,6-Difluor-1*H*-benzimidazol-2-ylmethyl)-piperidin-1-yl]-1-[5-(4-fluorphenyl)-2-methyl-2*H*-[1,2,3] triazol-4-yl]-methanon |
| 55 | (RS)-1-[2-(5,6-Difluor-1*H*-benzimidazol-2-ylmethyl)-piperidin-1-yl]-1-{2-[3-(3-dimethylamino-propoxy)-phenyl]-thiophen-3-yl}-methanon |
| 56 | (RS)-1-[2-(5,6-Difluor-1-methyl-1*H*-benzimidazol-2-ylmethyl)-piperidin-1-yl]-1-[5-(4-fluorphenyl)-2-methyl-thiazol-4-yl]-methanon |
| 57 | (RS)-1-(2-Benzothiazol-2-ylmethylpiperidin-1-yl)-1-[5-(4-fluorphenyl)-2-methylthiazol-4-yl]-methanon |
| 58 | (RS)-1-(2-Benzothiazol-2-ylmethylpiperidin-1-yl)-1-[4-(4-fluorphenyl)-1-methyl-1*H*-pyrazol-3-yl]-methanon |
| 59 | (RS)-1-(2-Benzothiazol-2-ylmethylpiperidin-1-yl)-1-[2-(3-methyl-[1,2,4]oxadiazol-5-yl)phenyl]-methanon |
| 60 | (RS)-1-[5-(4-Fluorphenyl)-2-methylthiazol-4-yl]-1-[2-(5,6,7,8-tetrahydro-[1,2,4]-triazolo[1,5-a]pyridin-2-ylmethyl)-piperidin-1-yl]-methanon |
| 61 | (RS)-1-[5-(4-Fluorphenyl)-2-methylthiazol-4-yl]-1-(2-[1,2,4]triazolo[1,5-a]pyridin-2-ylmethylpiperidin-1-yl)-methanon |
| 62 | 1-[(RS)-2-((RS)-2-Benzofuran-2-yl-2-hydroxy-ethyl)-piperidin-1-yl]-1-[5-(4-fluorphenyl)-2-methyl-thiazol-4-yl]-methanon (als getrennte Diastereoisomere) |
| 63 | (RS)-1-[2-(4-Brom-1*H*-benzimidazol-2-ylmethyl)-piperidin-1-yl]-1-[5-(4-fluorphenyl)-2-methylthiazol-4-yl]-methanon |
| 64 | (RS)-1-[2-(4-Cyano-1*H*-benzimidazol-2-ylmethyl)-piperidin-1-yl]-1-[5-(4-fluorphenyl)-2-methylthiazol-4-yl]-methanon |

Tabelle fortgesetzt

| | |
|---|---|
| 65 | (RS)-1-[2-(4-Acetyl-1*H*-benzimidazol-2-ylmethyl)-piperidin-1-yl]-1-[5-(4-fluorphenyl)-2-methylthiazol-4-yl]-methanon |
| 66 | (RS)-2-(1-{1-[5-(4-Fluorphenyl)-2-methyl-thiazol-4-yl]-methanoyl}-piperidin-2-ylmethyl)-1*H*-benzimidazol-5-carbonitril |
| 67 | (RS)-1-[2-(5,6-Difluor-1-propyl-1*H*-benzimidazol-2-ylmethyl)-piperidin-1-yl]-1-[5-(4-fluorphenyl)-2-methyl-thiazol-4-yl]-methanon |
| 68 | (RS)-1-(2-[5,6-Difluor-1-(2-methoxy-ethyl)-1*H*-benzimidazol-2-ylmethyl]-piperidin-1-yl}-1-[5-(4-fluorphenyl)-2-methyl-thiazol-4-yl]-methanon |
| 69 | (RS)-1-(2-[1-(2-Dimethylamino-ethyl)-5,6-difluor-1*H*-benzimidazol-2-ylmethyl]-piperidin-1-yl}-1-[5-(4-fluorphenyl)-2-methyl-thiazol-4-yl]-methanon |
| 70 | (RS)-1-{2-[5,6-Difluor-1-(2-hydroxy-ethyl)-1*H*benzimidazol-2-ylmethyl]-piperidin-1-yl} -1-[5-(4-fluorphenyl)-2-methyl-thiazol-4-yl]-methanon |
| 71 | (RS)-1-[2-(6,7-Difluor-1-methyl-1*H*-benzimidazol-2-ylmethyl)-piperidin-1-yl]-1-[5-(4-fluorphenyl)-2-methyl-thiazol-4-yl]-methanon |
| 72 | (RS)-1-[2-(4,5-Difluor-1-methyl-1*H*-benzimidazol-2-ylmethyl)-piperidin-1-yl]-1-[5-(4-fluorphenyl)-2-metliyl-thiazol-4-yl]-methänon |
| 73 | (RS)-2-(1 - {1-[5-(4-Fluorphenyl)-2-methyl-thiazol-4-yl]-methanoyl} -piperidin-2-ylmethyl)-benzofuran-3-carbonsäureamid |
| 74 | (RS)-2-(1-{1-[4-(4-Fluorphenyl)-1-methyl-1*H*pyrazol-3-yl]-methanoyl}-piperidin-2-ylmethyl)-benzofuran-3-carbonsäureamid |
| 75 | (RS)-1-[5-(4-Fluorphenyl)-2-methyl-thiazol-4-yl]-1-(2-furo[3,2-b]-pyridin-2-ylmethyl-piperidin-1-yl)-methanon |
| 76 | (RS)-1-[4-(4-Fluorphenyl)-1-methyl-1*H*-pyrazol-3-yl)-(2-furo[3,2-b]pyridin-2-ylmethyl-piperidin-1-yl)-methanon |
| 77 | (RS)-1-[2-(3-Brom-benzofuran-2-ylmethyl)-piperidin-1-yl]-1-[5-(4-fluorphenyl)-2-methyl-thiazol-4-yl]-methanon |
| 78 | (RS)-2-(1-{1-[5-(4-Fluorphenyl)-2-methyl-thiazol-4-yl]-methanoyl}-piperidin-2-ylmethyl)-benzofuran-3-carbonitril |
| 79 | (RS)-1-[2-(1-{1-[5-(4-Fluorphenyl)-2-methyl-thiazol-4-yl]-methanoyl}-piperidin-2-ylmethyl)-benzofuran-3-yl]-ethanon |
| 80 | (R)-1-[2-(5,6-Difluor-1*H*-benzimidazol-2-ylmethyl)-piperidin-1-yl]-1-[5-(4-fluorphenyl)-2-methyl-thiazol-4-yl]-methanon und (S)-1-[2-(5,6-Difluor-1*H*-benzimidazol-2-ylmethyl)-piperidin-1-yl]-1-[5-(4-fluorphenyl)-2-methyl-thiazol-4-yl]-methanon |
| 81 | (RS)-1-(2-Benzofuran-2-ylmethyl-piperidin-1-yl)-1-[5-(4-methoxyphenyl)-2-methyl-thiazol-4-yl]-methanon |
| 82 | (RS)-1-(2-Benzofuran-2-ylmethyl-piperidin-1-yl)-1-{5-[3-(2-dimethylamino-ethoxy)-phenyl]-2-methyl-thiazol-4-yl}-methanon |
| 83 | (RS)-1-[2-(4,5-Difluor-1*H*-benzimidazol-2-ylmethyl)-piperidin-1-yl]-1-(5-methyl-3-phenyl-isoxazol-4-yl)-methanon |
| 84 | 1-[3-(2,6-Dichlorphenyl)-5-methyl-isoxazol-4-yl]-1-[2-(4,5-difluor-1*H*-benzimidazol-2-ylmethyl)-piperidin-1-yl]-methanon |
| 85 | (RS)-1-[2-(4,5-Difluor-1*H*-benzimidazol-2-ylmethyl)-piperidin-1-yl]-1-[4-(4-fluorphenyl)-2-methyl-2*H*-pyrazol-3-yl]-methanon |
| 86 | (RS)-1-[3-(2-Chlor-6-fluorphenyl)-5-methyl-isoxazol-4-yl]-1-[2-(4,5-difluor-1*H*-benzimidazol-2-ylmethyl)-piperidin-1-yl]-methanon |
| 87 | (RS)-1-[3-(2-Chlorphenyl)-5-methyl-isoxazol-4-yl]-1-[2-(4,5-difluor-1*H* benzimidazol-2-ylmethyl)-piperidin-1-yl]-methanon |

Tabelle fortgesetzt

| | |
|---|---|
| 88 | (RS)-1-[2-(4,5-Difluor-1H-benzimidazol-2-ylmethyl)-piperidin-1-yl]-1-(2-propoxyphenyl)-methanon |
| 89 | (RS)-1-[2-(4,5-Difluor-1*H*-benzimidazol-2-ylmethyl)-piperidin-1-yl]-1-(2-isopropoxyphenyl)-methanon |
| 90 | (RS)-1-(2-Benzyloxyphenyl)-1-[2-(4,5-difluor-1*H*-benzimidazol-2-ylmethyl)-piperidin-1-yl]-methanon |
| 91 | (RS)-1-[2-(4,5-Difluor-1*H*-benzimidazol-2-ylmethyl)-piperidin-1-yl]-1-(2-ethoxy-6-methoxyphenyl)-methanon |
| 92 | (RS)-1-[2-(4,5-Difluor-1*H*-benzimidazol-2-ylmethyl)-piperidin-1-yl]-1-(2-ethoxy-6-methylphenyl)-methanon |
| 93 | (RS)-1-(2,6-Diethoxyphenyl)-1-[2-(4,5-difluor-1*H*-benzimidazol-2-ylmethyl)-piperidin-1-yl]-methanon |
| 94 | 1-(3-{1-[2-(4,5-Difluor-1*H*-benzimidazol-2-ylmethyl)-piperidin-1-yl]-methanoyl}-4-ethoxyphenyl)-ethanon |
| 95 | (RS)-1-[2-(4,5-Difluor-1*H*-benzimidazol-2-ylmethyl)-piperidin-1-yl]-1-(2-ethoxy-naphthalen-1-yl)-methanon |
| 96 | (RS)-1-[2-(4,5-Difluor-1*H*-benzimidazol-2-ylmethyl)-piperidin-1-yl]-1-(2-pyridin-2-yl-phenyl)-methanon |
| 97 | 1-[2-(6,7-Difluor-1*H*-benzimidazol-2-ylmethyl)-piperidin-1-yl]-1-(2-pyrrol-1-yl-phenyl)-methanon |
| 98 | (RS)-1-[2-(4,5-Difluor-1*H*-benzimidazol-2-ylmethyl)-piperidin-1-yl]-1-{5-[3-(3-dimethylamino-propoxy)-phenyl]-2-methyl-thiazol-4-yl}-methanon |
| 99 | (RS)-1-[2-(4,5-Difluor-1*H*-benzimidazol-2-ylmethyl)-piperidin-1-yl]-1-{5-[3-(4-dimethylamino-butoxy)-phenyl]-2-methyl-thiazol-4-yl}-methanon |
| 100 | (RS)-1-[2-(5,6-Difluor-1*H*-benzimidazol-2-ylmethyl)-piperidin-1-yl]-1-[5-(4-fluorphenyl)-2-methoxy-thiazol-4-yl]-methanon |
| 101 | (RS)-1-[2-(5,6-Difluor-1*H*-benzimidazol-2-ylmethyl)-piperidin-1-yl]-1-[2-ethyl-5-(4-fluorphenyl)-thiazol-4-yl]-methanon |
| 102 | (RS)-1-[2-(5,6-Difluor-1*H*-benzimidazol-2-ylmethyl)-piperidin-1-yl]-1-[5-(2-fluorphenyl)-2-methyl-thiazol-4-yl]-methanon |
| 103 | (RS)-1-[2-(5,6-Difluor-1*H* benzimidazol-2-ylmethyl)-piperidin-1-yl]-1-[5-(3-fluorphenyl)-2-methyl-thiazol-4-yl]-methanon |
| 104 | (RS)-1-[2-(4-Fluor-1*H*-benzimidazol-2-ylmethyl)-piperidin-1-yl]-1-[5-(4-fluorphenyl)-2-methyl-thiazol-4-yl]-methanon |
| 105 | (RS)-1-[2-(4-Fluor-1*H*-benzimidazol-2-ylmethyl)-piperidin-1-yl]-1-[4-(4-fluorphenyl)-1-methyl-1H-pyrazol-3-yl]-methanon |
| 106 | (RS)-1-[2-(4-Fluor-1H-benzimidazol-2-ylmethyl)-piperidin-1-yl]-1-[4-(4-fluorphenyl)-1*H*-pyrazol-3-yl]-methanon |
| 107 | (RS)-1-[2-(4-Fluor-1*H*-benzimidazol-2-ylmethyl)-piperidin-1-yl]-1-[4-(4-fluorphenyl)-2-methyl-2*H*-pyrazol-3-yl]-methanon |
| 108 | (RS)-1-(2-Ethoxyphenyl)-1-[2-(4-fluor-1*H*-benzimidazol-2-ylmethyl)-piperidin-1-yl]-methanon |
| 109 | (RS)-1-[2-(4-Fluor-1*H*-benzimidazol-2-ylmethyl)-piperidin-1-yl]-1-[5-(4-fluorphenyl)-2-hydroxymethyl-thiazol-4-yl]-methanon |
| 110 | (RS)-1-[5-(4-Fluorphenyl)-2-methyl-thiazol-4-yl]-1-{2-[4-(1-hydroxyethyl)-1H-benzimidazol-2-ylmethyl]-piperidin-1-yl} -methanon |
| 111 | (RS)-1-(2-Benzofuran-2-ylmethyl-piperidin-1-yl)-1-[5-(4-chlorphenyl)-2-methyl-thiazol-4-yl]-methanon |
| 112 | (RS)-1-[2-(3-Chlor-furo[3,2-b]pyridin-2-ylmethyl)-piperidin-1-yl]-1-[5-(4-fluorphenyl)-2-methyl-thiazol-4-yl]-methanon |
| 113 | (RS)-1-[2-(5,6-Difluor-1-methyl-1*H*-benzimidazol-2-ylmethyl)-piperidin-1-yl]-1-[5-(4-fluorphenyl)-2-hydroxymethyl-thiazol-4-yl]-methanon |
| 114 | (RS)-1-[5-(4-Fluorphenyl)-2-methyl-thiazol-4-yl]-1-[2-(1*H*-indol-2-ylmethyl)-piperidin-1-yl]-methanon |
| 115 | (RS)-5-[1-(2-Benzofuran-2-ylmethyl-piperidin-1-yl)-methanoyl]-4*H*-benzo[1,4]oxazin-3-on |

Tabelle fortgesetzt

| | |
|---|---|
| 116 | (RS)-1-[2-(5-Brom-benzofuran-2-ylmethyl)-piperidin-1-yl]-1-[5-(4-fluorphenyl)-2-methyl-thiazol-4-yl]-methanon |
| 117 | (RS)-1-[2-(5-Cyano-benzofuran-2-ylmethyl)-piperidin-1-yl]-1-[5-(4-fluorphenyl)-2-methyl-thiazol-4-yl]-methanon |
| 118 | (RS)-1-[2-(4-Brom-benzofuran-2-ylmethyl)-piperidin-1-yl]-1-[5-(4-fluorphenyl)-2-methyl-thiazol-4-yl]-methanon |
| 119 | (RS)-1 [2-(4-Cyano-benzofuran- 2- ylmethyl)-piperidin-1-yl]-1-[5-(4-fluorphenyl)-2-methyl-thiazol-4-yl]-methanon |
| 120 | (RS)-1- [5-(4-Fluorphenyl)-2-methyl-thiazol-4-yl]-1-[2-(3-methyl-benzokran-2-ylmethyl)-piperidin-1 -yl] -methanon |
| 121 | (RS)-1-[2-(4-Fluor-benzofuran-2-ylmethyl)-piperidin-1-yl]-1-[5-(4-fluorphenyl)-2-methyl-thiazol-4-yl]-methanon |
| 122 | (RS)-1-[2-(4-Fluor-benzomran-2-ylmethyl)-pipendin-1-yl]-1-[4-(4-fluorphenyl)-1-methyl- 1*H*-pyrazol-3-yl]-methanon |
| 123 | (RS)-1-(2-Benzo[b]thiophen-2-ylmethyl-piperidin-1-yl)-1-[5-(4-fluorphenyl)-2-methyl-thiazol-4-yl]-methanon |
| 124 | (RS)-1-(2-Benzo[b]thiophen-2-ylmethyl-piperidin-1-yl)-1-[4-(4-fluorphenyl)-1-methyl-1*H*-pyrazol-3-yl]-methanon |
| 125 | (RS)-1-(2-Benzo[b]thiophen-2-ylmethyl-piperidin-1-yl)-1-chinolin-8-yl-methanon |
| 126 | (RS)-1-(2-Benzofuran-2-ylmethyl-piperidin-1-yl)-1-(2-methyl-5-phenyl-thiazol-4-yl)-methanon |
| 127 | (RS)-1-(2-Benzofuran-2-ylmethyl-piperidin-l-yl)-1-[2-(3-methyl-[1,2,4]oxadiazol-5-yl)-phenyl]-methanon |
| 128 | (R)-1-[2-(4,6-Difluor-1*H*-benzimidazol-2-ylmethyl)-piperidin-1-yl]-1-[5-(4-fluorphenyl)-2-methyl-thiazol-4-yl]-methanon und (S)-1-[2-(4,6-Difluor-1*H*-benzimidazol-2-ylmethyl)-piperidin-1-yl]-1-[5-(4-fluorphenyl)-2-methyl-thiazol-4-yl]-methanon |

oder einem pharmazeutisch verträglichen Salz einer der vorstehenden Verbindungen.

**8.** Arzneimittel, umfassend eine Verbindung der Formel (I) wie in einem der Ansprüche 1 bis 7 definiert oder ein pharmazeutisch verträgliches Salz davon und einen pharmazeutisch verträglichen Träger.

**9.** Verwendung einer Verbindung gemäß einem der Ansprüche 1 bis 7 zur Herstellung eines Medikaments zur Behandlung oder Prävention von Obesität, Diabetes, Schlafstörungen, Schlaflosigkeit, Parasomnie oder Schlaganfall.

## Revendications

**1.** Composé de formule (I) :

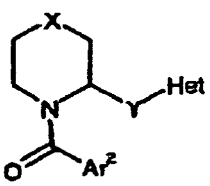

dans laquelle
X représente $CH_2$ ;
Y représente $CH_2$, CO, CH (OH) ou $CH_2CH$ (OH) ;
Het est un groupe hétéroaryle bicyclique facultativement substitué choisi dans la classe formée par les groupes quinoxalinyle, quinazolinyle, pyridopyrazinyle, benzoxazolyle, benzothiényle, benzofurannyle, benzimidazolyle, naphtyridinyle, benzothiazolyle, indolyle, triazolopyridinyle, furopyridinyle, pyridopyrimidinyle, isoquinolinyle, quinolinyle, oxazolylpyridinyle, tétrahydro-benzimidazolyle, tétrahydrobenzofurannyle ou tétrahydro-triazolopyridinyle ; $Ar^2$ représente un groupe phényle facultativement substitué ou un groupe hétérocyclyle penta- ou hexagonal facultativement substitué choisi dans la classe formée par les groupes furannyle, thiényle, pyrrolyle, oxazolyle, thiazolyle, imidazolyle, oxadiazolyle, thiadiazolyle, triazolyle, triazinyle, pyridazinyle, pyridinyle, pyrimidinyle, isothiazolyle, isoxazolyle, pyrazinyle ou pyrazolyle, où le groupe phényle ou hétérocyclyle est substitué par $R^1$ ; ou bien $Ar^2$ représente un groupe aromatique bicyclique ou hétéroaromatique bicyclique facultativement substitué contenant jusqu'à 3 hétéroatomes choisis parmi N, O et S ;
$R^1$ représente l'hydrogène, un groupe alkoxy en $C_1$ à $C_4$ facultativement substitué, halogéno, cyano, alkyle en $C_1$ à $C_6$ facultativement substitué, phényle facultativement substitué ou un groupe hétérocyclyle penta- ou hexagonal facultativement substitué contenant jusqu'à 4 hétéroatomes choisis parmi N, O et S ;
où les substituants facultatifs pour Het, $Ar^2$ et $R^1$ sont choisis dans la classe formée par les substituants halogéno, hydroxyle, oxo, cyano, nitro, alkyle en $C_1$ à $C_4$, alkoxy en $C_1$ à $C_9$, hydroxyalkyle en $C_1$ à $C_4$, hydroxyalkoxy en $C_1$ à $C_4$, halogénoalkyle en $C_1$ à $C_9$, halogénoalkoxy en $C_1$ à $C_4$, aryl(alkoxy en $C_1$ à $C_4$), alkylthio en $C_1$ à $C_4$, hydroxyalkyle en $C_1$ à $C_4$, (alkoxy en $C_1$ à $C_9$) alkyle en $C_1$ à $C_4$, (cycloalkyle en $C_3$ à $C_6$)alkoxy en $C_1$ à $C_9$, alcanoyle en $C_1$ à $C_9$, (alkoxy en $C_1$ à $C_4$) carbonyle, alkylsulfonyle en $C_1$ à $C_4$, alkylsulfonyloxy en $C_1$ à $C_4$, (alkyle en $C_1$ à $C_4$) - sulfonyle(alkyle en $C_1$ à $C_4$), arylsulfonyle, arylsulfonyloxy, arylsulfonyl(alkyle en $C_1$ à $C_9$), (alkyle en $C_1$ à $C_4$) sulfonamido, (alkyle en $C_1$ à $C_9$) amido, (alkyle en $C_1$ à $C_4$) sulfonamido (alkyle en $C_1$ à $C_4$) , (alkyle en $C_1$ à $C_4$) amido (alkyle en $C_1$ à $C_4$) , arylsulfonamido, arylcarboxamido, arylsulfonamido(alkyle en $C_1$ à $C_9$), arylcarboxamido (alkyle en $C_1$ à $C_4$) , aroyle, aroyl(alkyle en $C_1$ à $C_4$), aryl (alcanoyle en $C_1$ à $C_4$) ; acyle en $C_1$ à $C_4$ ; ou un groupe $R^aR^bN$-, $R^aOCO(CH_2)_r$, $R^aCON(R^a)(CH_2)_r$, $R^aR^bNCO(CH_2)_r$, $R^aR^bNSO_2(CH_2)_r$ ou $R^aSO_2NR^b$ $(CH_2)_r$, où chacun de $R^a$ et $R^b$ représente indépendamment un atome d'hydrogène ou un groupe alkyle en $C_1$ à $C_4$, ou bien $R^aR^b$ fait partie d'un cycle d'azacycloalcane en $C_3$ à $C_6$ ou de (2-oxo)azacycloalcane en $C_3$ à $C_6$, et r représente zéro ou un nombre entier de 1 à 4 ; ou $R^aR^bN$ $(CH_2)_n$ ou $R^aR^bN(CH_2)_nO$-, où n représente un nombre entier de 1 à 4 et où $R^a$ avec au moins un $CH_2$ de la portion $(CH_2)_n$ du groupe forme un azacycloalcane en $C_3$ à $C_6$ et $R^b$ représente l'hydrogène, un groupe alkyle en $C_1$ à $C_4$ ou, avec l'azote auquel il est attaché, forme un second azacycloalcane en $C_3$ à $C_6$ condensé au premier azacycloalcane en $C_3$ à $C_6$ ; ou un sel pharmaceutiquement acceptable de celui-ci.

**2.** Composé selon la revendication 1, dans lequel $Ar^2$ représente un groupe phényle, pyridinyle, thiazolyle, pyrazolyle, pyridazinyle, thiényle, naphtyle, triazolyle, isoxazolyle, quinolinyle ou isoquinolinyle facultativement substitué.

**3.** Composé selon la revendication 1 ou 2, dans lequel $R^1$ représente un groupe phényle, pyridinyle, pyrazolyle, pyrimidinyle ou oxadiazolyle facultativement substitué.

**4.** Composé selon l'une quelconque des revendications 1 à 3, dans lequel $Ar^2$ est facultativement substitué par un groupe alkyle en $C_1$ à $C_4$, hydroxyalkyle en $C_1$ à $C_4$, $R^aR^bN$, alkoxy en $C_1$ à $C_4$, $R^aR^bN$ $(CH_2)_n$, acyle en $C_1$ à $C_4$ ou (alkyle en $C_1$ à $C_4$) amido.

**5.** Composé selon l'une quelconque des revendications 1 à 4, dans lequel Het est un groupe benzimidazolyle, benzofurannyle ou benzoxazolyle.

**6.** Composé selon l'une quelconque des revendications 1 à 5, dans lequel Y représente $CH_2$.

**7.** Composé de formule (I) choisi dans le groupe formé par :

| | |
|---|---|
| 1 | (RS)-2-(2-benzofurannylméthyl)-1-((5-(4-fluorophényl)-2-méthylthiazole-4-yl)-carbonyl)pipéridine |
| 2 | (RS)-1-(2-benzooxazole-2-ylméthylpipéridine-1-yl)-1-[5-(4-fluorophényl)-2-méthylthiazole-4-yl]méthanone |
| 3 | (RS)-1-(2-benzooxazole-2-ylméthylpipéridine-1-yl)-1-[2-(3-méthyl-[1,2,4]oxadiazole-5-yl)phényl]méthanone |
| 4 | (RS)-1-(2-benzofuranne-2-ylméthylpipéridine-1-yl)-1-[4-(4-fluorophényl)-1-méthyl-1*H*-pyrazole-3-yl] méthane |
| 5 | (RS)-1-[2-(5-fluorobenzofuranne-2-ylméthyl)pipéidine-1-yl]-1-[5-(4-fluorophényl)-2-méthylthiazole-4-yl] méthanone |
| 6 | (RS)-1-[2-(5-fluorobenzofuranne-2-ylméthyl)pipéridine-1-yl]-1-(5-pyridine-2-ylthiazole-4-yl)méthanone |
| 7 | (RS)-1-[2-(5-fluorobenzofuranne-2-ylméthyl)pipéridine-1-yl]-1-quinoléine-4-yl-méthanone |
| 8 | (RS)-1-[2-(5-fluorobenzofuranne-2-ylméthyl)pipéridine-1-yl]-1-quinoléine-5-yl-méthanone |

Suite de tableau

| | |
|---|---|
| 9 | (RS)-1-[2-(5-fluorobenzofuranne-2-ylméthyl)pipéridine-1-yl]-1-(2-méthoxypyridine-3-yl)méthanone |
| 10 | (RS)-1-[2-diméthylamino-5-(4-fluorophényl)thiazole-4-yl]-1-[2-(5-fluorobenzofuranne-2-ylméthyl)pipéridine-1-yl]méthanone |
| 11 | (RS)-1-[2-(5-fluorobenzofuranne-2-ylméthyl)pipéridine-1-yl]-1-(2-morpholine-4-ylphényl)méthanone |
| 12 | (RS)-1-[2-(5-fluorobenzofuranne-2-ylméthyl)pipéridine-1-yl]-1-(2-trifluorométhoxyphényl)méthanone |
| 13 | (RS)-1-(2-benzofuranne-2-ylméthylpipéridine-1-yl)-1-[2-(2-diméthylamino-éthoxy)phényl]méthanone |
| 14 | (RS)-1-(2-benzofuranne-2-ylméthylpipéridine-1-yl)-1-quinoléine-8-ylméthanone |
| 15 | (RS)-1-(2-benzofuranne-2-ylméthylpipéridine-1-yl)-1-(2-pyrimidine-2-ylphényl)-méthanone |
| 16 | (RS)-1-(2-benzofuranne-2-ylméthylpipéridine-1-yl)-1-[5-(4-fluorophényl)-2H-[1,2,3]triazole-4-yl]méthanone |
| 17 | (RS)-1-(2-benzofuranne-2-ylméthylpipéridine-1-yl)-1-[4-(4-fluorophényl)1 H-pyrazole-3-yl]méthanone |
| 18 | (RS)-1-(2-benzofuranne-2-ylméthylpipéridine-1-yl)-1-[5-(4-fluorophényl)-2-hydroxyméthylthiazole-4-yl] méthanone |
| 19 | (RS)-1-(2-benzofuranne-2-ylméthylpipéridine-1-yl)-1-isoquinoléine-8-ylméthanone |
| 20 | (RS)-1-(2-benzofuranne-2-ylméthylpipéridine-1-yl)-1-isoquinoléine-5-ylméthanone |
| 21 | (RS)-1-(2-benzofuranne-2-ylméthylpipéridine-1-yl)-1-[5-[3-(3-diméthylamino-propoxy)phényl]-2-méthylthiazole-4-yl]méthanone |
| 22 | (RS)-1-(2-benzofuranne-2-ylméthylpipéridine-1-yl)-1-[5-[3-(4-diméthylamino-butoxy)phényl]-2-méthylthiazole-4-yl]méthanone |
| 23 | (RS)-1-[5-(4-fluorophényl)-2-méthylthiazole-4-yl]-1-(2-furo[2,3-b]pyridine-2-yl-méthylpipéridine-1-yl) méthanone |
| 24 | (RS)-1-[4-(4-fluorophényl)-1-méthyl-1*H*-pyrazole-3-yl]-(2-furo[2,3-*b*]pyridine-2-ylméthylpipéridine-1-yl) méthanone |
| 25 | (RS)-1-[5-(4-fluorophényl)-2-méthylthiazole-4-yl]-1-(2-quinoléine-2-ylméthylpipéridine-1-yl)méthanone |
| 26 | (RS)-1-benzofuranne-2-yl-1-(1-[1-[5-(4-fluorophényl)-2-méthylthiazole-4-yl]-méthanoyl]pipéridine-2-yl) méthanone |
| 27 | (RS)-1-[2-(1*H*-benzoimidazole-2-ylméthyl)pipéridine-1-yl]-1-[5-(4-fluorophényl)-2-méthylthiazole-4-yl] méthanone |
| 28 | (RS)-1-[2-(5-chloro-1H-benzoimidazole-2-ylméthyl)pipéridine-1-yl]-1-[5-(4-fluorophényl)-2-méthylthiazole-4-yl]méthanone |
| 29 | (RS)-1-[2-(5-fluoro-1H-benzoimidazole-2-ylméthyl)pipéridine-1-yl]-1-[5-(4-fluorophényl)-2-méthylthiazole-4-yl]méthanone |
| 30 | (RS)-1-[2-(5-chloro-6-fluoro-1H-benzoimidazole-2-ylméthyl)pipéridine-1-yl]-1-[5-(4-fluorophényl)-2-méthylthiazole-4-yl]méthanone |
| 31 | (RS)-1-[5-(4-fluorophényl)-2-méthylthiazole-4-yl]-1-[2-(4-méthyl-1H-benzoimidazole-2-ylméthyl)pipéridine-1-yl]méthanone |
| 32 | (RS)-1-[2-(4,6-difluoro-1H-benzoimidazole-2-ylméthyl)pipéridine-1-yl]-1-[5-(4-fluorophényl)-2-méthylthiazole-4-yl]méthanone |
| 33 | (RS)-1-[2-(4-diméthylaminométhyl-1H-benzoimidazole-2-ylméthyl)pipéridine-1-yl]-1-[5-(4-fluorophényl)-2-méthylthiazole-4-yl]méthanone |
| 34 | (RS)-1-[2-(5-bromo-1H-benzoimidazole-2-ylméthyl)pipéridine-1-yl]-1-[5-(4-fluorophényl)-2-méthylthiazole-4-yl]méthanone |
| 35 | (RS)-1-[2-(5,6-difluoro-1H-benzoimidazole-2-ylméthyl)pipéridine-1-yl]-1-[5-(4-fluorophényl)-2-méthylthiazole-4-yl]méthanone |

Suite de tableau

| | |
|---|---|
| 36 | (RS)-1-[2-(4,5-difluoro-1*H*-benzoimidazole-2-ylméthyl)pipéridine-1-yl]-1-[5-(4-fluorophényl)-2-méthylthiazole-4-yl]méthanone |
| 37 | (RS)-1-[2-(5,6-difluoro-1*H*-benzoimidazole-2-ylméthyl)pipéridine-1-yl]-1-[5-(4-fluorophényl)-2-hydroxyméthylthiazole-4-yl]méthanone |
| 38 | (RS)-1-[2-(4,5-difluoro-1*H*-benzoimidazole-2-ylméthyl)pipéridine-1-yl]-1-[5-(4-fluorophényl)-2-hydroxyméthylthiazole-4-yl]méthanone |
| 39 | (RS)-1-[2-(5,6-difluoro-1*H*-benzoimidazole-2-ylméthyl)pipéridine-1-yl]-1-[4-(4-fluorophényl)-1-méthyl-1H-pyrazole-3-yl]méthanone |
| 40 | (RS)-1-[2-(5-méthoxy-1*H*-benzoimidazole-2-ylméthyl)pipéridine-1-yl]-1-[5-(4-fluorophényl)-2-méthylthiazole-4-yl]méthanone |
| 41 | (RS)-1-[2-(6,7-difluoro-1H-benzoimidazole-2-ylméthyl)pipéridine-1-yl]-1-(2-méthyl-5-phénylthiazole-4-yl) méthanone |
| 42 | (RS)-1-[2-(6,7-difluoro-1H-benzoimidazole-2-ylméthyl)pipéridine-1-yl]-1-[4-(4-fluorophényl)-1-méthyl-1H-pyrazole-3-yl]méthanone |
| 43 | (RS)-1-[2-(6,7-difluoro-1H-benzoimidazole-2-ylméthyl)pipéridine-1-yl]-1-[4-(4-fluorophényl)-1 H-pyrazole-3-yl]méthanone |
| 44 | (RS)-1-[2-(4,5-difluoro-1H-benzoimidazole-2-ylméthyl)pipéridine-1-yl]-1-{5-[3-(2-diméthylaminoéthoxy) phényl]-2-méthylthiazole-4-yl}méthanone |
| 45 | (RS)-1-[2-(4,5-difluoro-1H-benzoimidazole-2-ylméthyl)pipéridine-1-yl]-1-[5-(2-fluorophényl)-2-méthylthiazole-4-yl]méthanone |
| 46 | (RS)-1-[2-(4,5-difluoro-1H-benzoimidazole-2-ylméthyl)pipéridine-1-yl]-1-[5-(3-fluorophényl)-2-méthylthiazole-4-yl]méthanone |
| 47 | (RS)-1-[2-(4,5-difluoro-1H-benzoimidazole-2-ylméthyl)pipéridine-1-yl]-1-(2-trifluorométhoxyphényl) méthanone |
| 48 | (RS)-1-[2-(4,5-difluoro-1H-benzoimidazole-2-ylméthyl)pipéridine-1-yl]-1-(2-éthoxyphényl)méthanone |
| 49 | (RS)-1-[2-(4,5-difluoro-1H-benzoimidazole-2-ylméthyl)pipéridine-1-yl]-1-(2,6-diméthoxyphényl)méthanone |
| 50 | (RS)-1-[2-(6,7-difluoro-1H-benzoimidazole-2-ylméthyl)pipéridine-1-yl]-1-(2-pyrazole-1-ylphényl)méthanone |
| 51 | (RS)-1-[2-(5,6-difluoro-1H-benzoimidazole-2-ylméthyl)pipéridine-1-yl]-1-[4-(4-fluorophényl)-1H-pyrazole-3-yl]méthanone |
| 52 | (RS)-1-[2-(5,6-difluoro-1H-benzoimidazole-2-ylméthyl)pipéridine-1-yl]-1-[2-(3-méthyl-[1,2,4]oxadiazole-5-yl) phényl]méthanone |
| 53 | (RS)-1-[2-(difluoro-1H-benzoimidazole-2-ylméthyl)pipéridine-1-yl]-1-[1-(2-diméthylaminoéthyl)-4-(4-fluorophényl)-1H-pyrazole-3-yl]méthanone |
| 54 | (RS)-1-[2-(5,6-difluoro-1H-benzoimidazole-2-ylméthyl)pipéridine-1-yl]-1-[5-(4-fluorophényl)-2-méthyl-2H-[1,2,3]triazole-4-yl]méthanone |
| 55 | (RS)-1-[2-(5,6-difluoro-1H-benzoimidazole-2-ylméthyl)pipéridine-1-yl]-1-{2-[3-(3-diméthylaminopropoxy) phényl]thiophène-3-yl}méthanone |
| 56 | (RS)-1-[2-(5,6-difluoro-1-méthyl-1H-benzoimidazole-2-ylméthyl)pipéridine-1-yl]-1-[5-(4-fluorophényl)-2-méthylthiazole-4-yl]méthanone |
| 57 | (RS)-1-(2-benzothiazole-2-ylméthylpipéridine-1-yl)-1-[5-(4-fluorophényl)-2-méthylthiazole-4-yl]méthanone |
| 58 | (RS)-1-(2-benzothiazole-2-ylméthylpipéridine-1-yl)-1-[4-(4-fluorophényl)-1-méthyl-1*H*-pyrazole-3-yl] méthanone |
| 59 | (RS)-1-(2-benzothiazole-2-ylméthylpipéridine-1-yl)-1-[2-(3-méthyl[1,2,4]oxadiazole-5-yl)méthyl]méthanone |
| 60 | (RS)-1-[5-(4-fluorophényl)-2-méthylthiazole-4-yl]-1-[2-(5,6,7,8-tétrahydro-[1,2,4]triazolo[1,5-a]pyridine-2-ylméthyl)pipéridine-1-yl]méthanone |

Suite de tableau

| | |
|---|---|
| 61 | (RS)-1-[5-(4-fluorophényl)-2-méthylthiazole-4-yl]-1-(2-[1,2,4]triazolo-[1,5-a]pyridine-2-ylméthylpipéridine-1-yl)méthanone |
| 62 | 1-[(RS)-2-((RS)-2-benzofuranne-2-yl-2-hydroxyéthyl)pipéridine-1-yl]-1-[5-(4-fluorophényl)-2-méthylthiazole-4-yl]méthanone (sous forme de diastéréoisomères séparés) |
| 63 | (RS)-1-[2-(4-bromo-1*H*-benzoimidazole-2-ylméthyl)pipéridine-1-yl]-1-[5-(4-fluorophényl)-2-méthylthiazole-4-yl]méthanone |
| 64 | (RS)-1-[2-(4-cyano-1*H*-benzoimidazole-2-ylméthyl)pipéridine-1-yl]-1-[5-(4-fluorophényl)-2-méthylthiazole-4-yl]méthanone |
| 65 | (RS)-1-[2-(4-acétyl-1H-benzoimidazole-2-ylméthyl)pipéridine-1-yl]-1-[5-(4-fluorophényl)-2-méthylthiazole-4-yl]méthanone |
| 66 | (RS)-2-(1-{1-[5-(4-fluorophényl)-2-méthylthiazole-4-yl]méthanoyl}pipéridine-2-ylméthyl)-1 H-benzoimidazole-5-carbonitrile |
| 67 | (RS)-1-[2-(5,6-difluoro-1-propyl-1*H*-benzoimidazole-2-ylméthyl]pipéridine-1-yl]-1-[5-(4-fluorophényl)-2-methylthiazole-4-yl]méthanone |
| 68 | (RS)-1-{2-[5,6-difluoro-1-(2-méthoxyéthyl)-1 H-benzoimidazole-2-ylméthyl]pipéridine-1-yl}-1-[5-(4-fluorophényl)-2-méthylthiazole-4-yl]méthanone |
| 69 | (RS)-1-{2-[1-(2-diméthylaminoéthyl)-5,6-difluoro-1*H*-benzoimidazole-2-ylméthyl]-pipéridine-1-yl}-1-[5-(4-fluorophényl)-2-méthylthiazole-4-yl]méthanone |
| 70 | (RS)-1-{2-[5,6-difluoro-1-(2-hydroxyéthyl)-1H-benzoimidazole-2-ylméthyl]pipéridine-1-yl}-1-[5-(4-fluorophényl)-2-méthylthiazole-4-yl]méthanone |
| 71 | (RS)-1-[2-(6,7-difluoro-1-méthyl-1*H*-benzoimidazole-2-ylméthyl)pipéridine-1-yl]-1-[5-(4-fluorophényl)-2-méthylthiazole-4-yl]méthanone |
| 72 | (RS)-1-[2-(4,5-difluoro-1-méthyl-1*H*-benzoimidazole-2-ylméthyl)pipéridine-1-yl]-1-[5-(4-fluorophényl)-2-méthylthiazole-4-yl]méthanone |
| 73 | Amide d'acide (RS)-2-(1-{1-[5-(4-fluorophényl)-2-méthylthiazole-4-yl]méthanoyl}-pipéridine-2-ylméthyl) benzofuranne-3-carboxylique |
| 74 | Amide d'acide (RS)-2-(1-{1-[4-(4-fluorophényl)-1-méthyl-1*H*-pyrazole-3-yl]-méthanoyl}pipéridine-2-ylméthyl) benzofuranne-3-carboxylique |
| 75 | (RS)-1-[5-(4-fluorophényl)-2-méthylthiazole-4-yl]-1-(2-furo[3,2-b]pyridine-2-yl-méthylpipéridine-1-yl) méthanone |
| 76 | (RS)-1-[4-(4-fluorophényl)-1-méthyl-1H-pyrazole-3-yl)-(2-furo[3,2-b]pyridine-2-ylméthylpipéridine-1-yl) méthanone |
| 77 | (RS)-1-[2-(3-bromobenzofuranne-2-ylméthyl)pipéridine-1-yl]-1-[5-(4-fluorophényl)-2-méthylthiazole-4-yl] méthanone |
| 78 | (RS)-2-(1 1-{1-[5-(4-fluorophényl)-2-méthylthiazole-4-yl]méthanoyl}pipéridine-2-ylméthyl)benzofuranne-3-carbonitrile |
| 79 | (RS)-1-[2-(1-{1-[5-(4-fluorophényl)-2-méthylthiazole-4-yl]méthanoyl}pipéridine-2-yl-méthyl)benzofuranne-3-yl]éthanone |
| 80 | (R)-1-[2-(5,6-difluoro-1*H*-benzoimidazole-2-ylméthyl)pipéridine-1-yl]-1-[5-(4-fluorophényt)-2-méthylthiazole-4-yl]méthanone et (S)-1-[2-(5,6-difluoro-1*H*-benzoimidazole-2(ylméthyl)pipéridine-1-yl]-1-[5-(4-fluorophényl)-2-méthylthiazole-4-yl]méthanone |
| 81 | (RS)-1-(2-benzofuranne-2-ylméthylpipéridine-1-yl)-1-[5-(4-méthoxyphényl)-2-méthylthiazole-4-yl] méthanone |
| 82 | (RS)-1-(2-benzofuranne-2-ylméthylpipéridine-1-yl)-1-{5-[3-(2-diméthylamino-éthoxy)phényl]-2-méthylthiazole-4-yl}méthanone |

Suite de tableau

| | |
|---|---|
| 83 | (RS)-1-[2-(4,5-difluoro-1H-benzoimidazole-2-ylméthyl)pipéridine-1-yl]-1-(5-méthyl-3-phénylisoxazole-4-yl) méthanone |
| 84 | 1-[3-(2,6-dichlorophényl)-5-méthylisoxazole-4-yl]-1-[2-(4,5-difluoro-1H-benzoimidazole-2-ylméthyl) pipéridine-1-yl]méthanone |
| 85 | (RS)-1-[2-(4,5-difluoro-1H-benzoimidazole-2-ylméthyl)pipéridine-1-yl]-1-[4-(4-fluorophényl)-2-méthyl-2H-pyrazole-3-yl]méthanone |
| 86 | (RS)-1-[3-(2-chloro-6-fluorophényl)-5-méthylisoxazole-4-yl]-1-[2-(4,5-difluoro-1H-benzoimidazole-2-ylméthyl)pipéridine-1-yl]méthanone |
| 87 | (RS)-1-[3-(2-chlorophényl)-5-méthylisoxazole-4-yl]-1-[2-(4,5-difluoro-1 H-benzoimidazole-2-ylméthyl) pipéridine-1-yl]méthanone |
| 88 | (RS)-1 -[2-(4,5-difluoro-1 H-benzoimidazole-2-ylméthyl)pipéridine-1-yl]-1-(2-propoxyphényl)méthanone |
| 89 | (RS)-1-[2-(4,5-difluoro-1H-benzoimidazole-2-ylméthyl)pipéridine-1-yl]-1-(2-isopropoxyphényl)méthanone |
| 90 | (RS)-1-(2-benzyloxyphényl)-1-[2-(4,5-difluoro-1 H-benzoimidazole-2-ylméthyl)-pipéridine-1-yl]méthanone |
| 91 | (RS)-1-[2-(4,5-difluoro-1 H-benzoimidazole-2-ylméthyl)pipéridine-1-yl]-1-(2-éthoxy-6-méthoxyphényl) méthanone |
| 92 | (RS)-1-[2-(4,5-difluoro-1 H-benzoimidazole-2-ylméthyl)pipéridine-1-yl]-1-(2-éthoxy-6-méthylphényl) méthanone |
| 93 | (RS)-1-(2,6-diéthoxyphényl)-1-[2-(4,5-difluoro-1H-benzoimidazole-2-ylméthyl)-pipéridine-1-yl]méthanone |
| 94 | 1-(3-{1-[2-(4,5-difluoro-1H-benzoimidazole-2-ylméthyl)pipéridine-1-yl]méthanoyl}-4-éthoxyphényl)éthanone |
| 95 | (RS)-1-[2-(4,5-difluoro-1H-benzoimidazole-2-ylméthyl)pipéridine-1-yl]-1-(2-éthoxynaphtalène-1-yl) méthanone |
| 96 | (RS)-1-[2-(4,5-difluoro-1H-benzoimidazole-2-ylméthyl)pipéridine-1-yl]-1-(2-pyridine-2-ylphényl)méthanone |
| 97 | 1-[2-(6,7-difluoro-1H-benzoimidazole-2-ylméthyl)pipéridine-1-yl]-1-(2-pyrrole-1-ylphényl)méthanone |
| 98 | (RS)-1-[2-(4,5-difluoro-1 H-benzoimidazole-2-ylméthyl)pipéridine-1-yl]-1-{5-[3-(3-diméthylaminopropoxy) phényl)-2-méthylthiazole-4-yl}méthanone |
| 99 | (RS)-1-[2-(4,5-difluoro-1H-benzoimidazole-2-ylméthyl)pipéridine-1-yl]-1-{5-[3-(4-diméthylaminobutoxy) phényl]-2-méthylthiazole-4-yl}méthanone |
| 100 | (RS)-1-[2-(5,6-difluoro-1H-benzoimidazole-2-ylméthyl)pipéridine-1-yl]-1-[5-(4-fluorophényl)-2-méthoxythiazole-4-yl]méthanone |
| 101 | (RS)-1-[2-(5,6-difluoro-1H-benzoimidazole-2-ylméthyl)pipéridine-1-yl]-1-[2-éthyl-5-(4-fluorophényl)thiazole-4-yl]méthanone |
| 102 | (RS)-1-[2-(5,6-difluoro-1H-benzoimidazole-2-ylméthyl)pipéridine-1-yl]-1-[5-(2-fluorophényl)-2-méthylthiazole-4-yl]méthanone |
| 103 | (RS)-1-[2-(5,6-difluoro-1H-benzoimidazole-2-ylméthyl)pipéridine-1-yl]-1-[5-(3-fluorophényl)-2-méthylthiazole-4-yl]méthanone |
| 104 | (RS)-1-[2-(4-fluoro-1H-benzoimidazole-2-ylméthyl)pipéridine-1-yl]-1-[5-(4-fluorophényl)-2-méthylthiazole-4-yl]méthanone |
| 105 | (RS)-1-[2-(4-fluoro-1 H-benzoimidazole-2-ylméthyl)pipéridine-1-yl]-1-[4-(4-fluorophényl)-1-méthyl-1 H-pyrazole-3-yl]méthanone |
| 106 | (RS)-1-[2-(4-fluoro-1H-benzoimidazole-2-ylméthyl)pipéridine-1-yl]-1-[4-(4-fluorophényl)-1 H-pyrazole-3-yl] méthanone |
| 107 | (RS)-1-[2-(4-fluoro-1H-benzoimidazole-2-ylméthyl)pipéridine-1-yl]-1-[4-(4-fluorophényl)-2-méthyl-2H-pyrazole-3-yl]méthanone |
| 108 | (RS)-1-(2-éthoxyphényl)-1-[2-(4-fluoro-1H-benzoimidazole-2-ylméthyl)pipéridine-1-yl]-méthanone |

Suite de tableau

| | |
|---|---|
| 109 | (RS)-1-[2-(4-fluoro-1H-benzoimidazole-2-ylméthyl)pipéridine-1-yl]-1-[5-(4-fluorophényl)-2-hydroxyméthylthiazole-4-yl]méthanone |
| 110 | (RS)-1-[5-(4-fluorophényl)-2-méthylthiazole-4-yl]-1-{2-[4-(1-hydroxyéthyl) 1*H*-benzoimidazole-2-ylméthyl] pipéridine-1-yl}méthanone |
| 111 | (RS)-1-(2-benzofuranne-2-ylméthylpipéridine-1-yl)-1-[5-(4-chlorophényl)-2-méthylthiazole-4-yl]méthanone |
| 112 | (RS)-1-[2-(3-chlorofuro[3,2-b]pyridine-2-ylméthyl)pipéridine-1-yl]-1-[5-(4-fluorophényl)-2-méthylthiazole-4-yl] méthanone |
| 113 | (RS)1-[2-(5,6-difluoro-1-méthyl-1H-benzoimidazote-2-ylméthyl)pipéridine-1-yl]-1-[5-(4-fluorophényl)-2-hydroxyméthylthiazole-4-yl]méthanone |
| 114 | (RS)-1-[5-(4-fluorophényl)-2-méthylthiazole-4-yl]-1-[2-(1H-indole-2-ylméthyl)pipéridine-1-yl]méthanone |
| 115 | (RS)-5-[1-(2-benzofuranne-2-ylméthylpipéridine-1-yl)méthanoyl]-4*H*-benzo-[1,4]oxazine-3-one |
| 116 | (RS)-1-[2-(5-bromobenzofuranne-2-ylméthyl)pipéridine-1-yl]-1-[5-(4-fluorophényl)-2-méthylthiazole-4-yl] méthanone |
| 117 | (RS)-1-[2-(5-cyanobenzofuranne-2-ylméthyl)pipéridine-1-yl]-1-[5-(4-fluorophényl)-2-méthylthiazole-4-yl] méthanone |
| 118 | (RS)-1-[2-(4-bromobenzofuranne-2-ylméthyl)pipéridine-1-yl]-1-[5-(4-fluorophényl)-2-méthylthiazole-4-yl] méthanone |
| 119 | (RS)-1-[2-(4-cyanobenzofuranne-2-ylméthyl)pipéridine-1-yl]-1-[5-(4-fluorophényl)-2-méthylthiazole-4-yl] méthanone |
| 120 | (RS)-1-[5-(4-fluorophényl)-2-méthylthiazole-4-yl]-1-[2-(3-méthylbenzofuranne-2-ylméthyl)pipéridine-1-yl] méthanone |
| 121 | (RS)-1-[2-(4-fluorobenzofuranne-2-ylméthyl)pipéridine-1-yl]-1-[5-(4-fluorophényl)-2-méthylthiazole-4-yl] méthanone |
| 122 | (RS)-1-[2-(4-fluorobenzofuranne-2-ylméthyl)pipéridine-1-yl]-1-[4-(4-fluorophényl)-1-méthyl-1 H-pyrazole-3-yl]méthanone |
| 123 | (RS)-1-(2-benzo[b]thiophène-2-ylméthylpipéridine-1-yl)-1-[5-(4-fluorophényl)-2-méthylthiazole-4-yl] méthanone |
| 124 | (RS)-1-(2-benzo[b]thiophène-2-ylméthylpipéridine-1-yl)-1-[4-(4-fluorophényl)-1-méthyl-1 H-pyrazole-3-yl] méthanone |
| 125 | (RS)-1-(2-benzo[b]thiophène-2-ylméthylpipéridine-1-yl)-1-quinoléine-8-yl-méthanone |
| 126 | (RS)-1-(2-benzofuranne-2-ylméthylpipéridine-1-yl)-1-(2-méthyl-5-phényl-thiazole-4-yl)méthanone |
| 127 | (RS)-1-(2-benzofuranne-2-ylméthylpipéridine-1-yl)-1-[2-(3-méthyl[1,2,4]oxadiazole-5-yl)phényl]méthanone |
| 128 | (R)-1-[2-(4.6-difluoro-1*H*-benzoimidazole-2-ylméthyl)pipéridine-1 -yl]-1-[5-(4-fluorophényl)-2-méthylthiazole-4-yl]méthanone et (S)-1-[2-(4,6-difluoro-1*H*-benzoimidazole-2-ylméthyl)pipéridine-1-yl]-1-[5-(4-fluorophényl)-2-méthylthiazole-4-yl]méthanone |

ou un sel pharmaceutiquement acceptable de l'un quelconque de ceux-ci.

**8.** Composition pharmaceutique comprenant un composé de formule (I) tel que défini dans l'une quelconque des revendications 1 à 7, ou un sel pharmaceutiquement acceptable de celui-ci, et un support pharmaceutiquement acceptable.

**9.** Utilisation d'un composé selon l'une quelconque des revendications 1 à 7 pour la fabrication d'un médicament destiné au traitement ou à la prévention de l'obésité, du diabète, de troubles du sommeil, de l'insomnie, de la parasomnie ou d'une attaque.